(19) Europäisches Patentamt
European Patent Office
Office européen des brevets

(11) EP 0 902 893 B1

(12) **FASCICULE DE BREVET EUROPEEN**

(45) Date de publication et mention de la délivrance du brevet:
**10.06.2009 Bulletin 2009/24**

(21) Numéro de dépôt: **98903063.0**

(22) Date de dépôt: **14.01.1998**

(51) Int Cl.:
***G01N 33/543*** (2006.01) ***A61M 1/16*** (2006.01)
***B01D 61/28*** (2006.01)

(86) Numéro de dépôt international:
**PCT/FR1998/000066**

(87) Numéro de publication internationale:
**WO 1998/030905 (16.07.1998 Gazette 1998/28)**

(54) **MOYENS POUR LA BIO-EPURATION D'UN FLUIDE BIOLOGIQUE**

VORRICHTUNG ZUR BIOLOGISCHEN REINIGUNG VON BIOLOGISCHEN FLÜSSIGKEITEN

MEANS FOR THE BIOLOGICAL PURIFICATION OF A BIOLOGICAL FLUID

(84) Etats contractants désignés:
**AT BE CH DE ES FR GB IE IT LI NL SE**

(30) Priorité: **14.01.1997 FR 9700298**

(43) Date de publication de la demande:
**24.03.1999 Bulletin 1999/12**

(73) Titulaire: **Gambro Industries SAS**
**69330 Meyzieu (FR)**

(72) Inventeurs:
- **SIMARD, Laurent**
  **F-69360 Saint Genis Laval (FR)**
- **THOMAS, Michel**
  **F-69360 Serezin du Rhône (FR)**
- **QUASH, Gérard**
  **F-69340 Francheville (FR)**
- **MOACHON, Nicolas**
  **F-69290 Craponne (FR)**

(74) Mandataire: **Fleurance, Raphaël et al**
**Cabinet Plasseraud**
**52 rue de la Victoire**
**75440 Paris Cedex 09 (FR)**

(56) Documents cités:
**EP-A- 0 294 905** **EP-A- 0 312 135**
**EP-A- 0 351 314** **DE-A- 4 209 988**
**GB-A- 2 197 720** **US-A- 5 240 994**
**US-A- 5 417 969** **US-B- 5 403 917**

- **S. MITZNER ET AL: "Extracorporeal endotoxin removal by immobilized polyethylenimine" ARTIFICIAL ORGANS, vol. 17, no. 9, septembre 1993, pages 775-781, XP002044283**
- **KOPACIEWICZ W. ET AL: 'Synthesis of Cation-Exchange Stationary Phases Using an Adsorbed Polymeric Coating' JOURNAL OF CHROMATOGRAPHY vol. 358, no. 1, 16 Mai 1986, NETHERLANDS, pages 107 - 117, XP009018956**
- **MALMSTEN M. ET AL: 'Effects of Hydrophilization and Immobilization on the Interfacial Behaviour of Immunoglobulins' JOURNAL OF COLLOID AND INTERFACE SCIENCE vol. 177, no. 1, 15 Janvier 1996, pages 70 - 78, XP009095797**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention porte sur des membranes pour la bioépuration d'un fluide biologique.

Par épuration ou bioépuration, on entend dans l'ensemble de ce texte le processus d'élimination partielle ou totale d'une molécule ou macromolécule endogène ou hétérologue présente dans le fluide biologique, et dont l'élimination totale ou partielle est recherchée ; cette molécule ou macromolécule peut être à l'état soluble isolée, ou complexée à d'autres substances.

La présente invention est également relative à un dispositif polyvalent pour l'épuration d'un fluide biologique. Le dispositif est constitué d'un support fonctionnalisé, hémocompatible dans le cas d'une utilisation dans un circuit extracorporel pour le traitement du sang. La particularité du dispositif est de pouvoir fixer de manière covalente tout type de ligand utilisable pour modifier l'activité ou la composition d'un fluide biologique.

L'invention est également relative au procédé d'obtention et à la mise en oeuvre du support soit seul, soit intégré dans un kit de préparation extemporanée du support susceptible de porter un ligand spécifique.

Des membranes semi-perméables biocompatibles ont été développées pour la dialyse de patients insuffisants rénaux. Ces supports peuvent prendre la forme soit de membranes planes, soit de fibres creuses. Plusieurs membranes à base de polymères synthétiques ou à base de cellulose modifiée on été mises à la disposition des utilisateurs par les fabricants. Les polymères utilisés sont rarement des homopolymères, et aucune membrane n'est constituée uniquement de polyacrylonitrile.

A titre d'exemple, la membrane PAN d'Asahi est constituée d'un copolymère d'acrylonitrile, de méthacrylate de méthyle et d'acide acrylique ; cette membrane présente une structure microporeuse asymétrique formée d'une couche dense en contact avec le sang et d'une paroi spongieuse côté dialysat. La membrane SPAN d'ENKA est également une membrane microporeuse asymétrique à base d'acrylonitrile, de méthallyl sulfonate de sodium et de méthacrylate de méthyle.

La membrane AN69 fabriquée et commercialisée par HOSPAL (Meyzieu, France) est à base d'un copolymère d'acrylonitrile et de méthallyle sulfonate de sodium. Cette membrane est connue pour ses propriétés de biocompatibilité et de transfert. La teneur en sites anioniques (provenant du comonomère méthallyle sulfonate de sodium) du poylmère AN69 est de 600 m Eq/kg. Celle de la membrane est d'environ 180 Eq/kg (teneur en polymère voisine de 30 %).

D'autres polymères, notamment des polymères de polysulfone, présentent également une structure microporeuse asymétrique ; ils sont fondamentalement hydrophobes et doivent être mélangés à des composants hydrophiles tels la polyvinylpyrrolidone pour pouvoir fournir des membranes de dialyse dotées de propriétés diffusives suffisantes.

Les supports hydrophiles symétriques, qu'ils soient sous forme de fibres creuses ou de membranes planes selon l'usage que l'on souhaite en faire, présentent en outre des avantages fonctionnels quant à leur biocompatibilité en ce qu'ils sont faiblement activateurs du complément, non thrombogènes et d'une grande capacité de diffusion.

De manière générale, tout polymère anionique biocompatible, comme les copolymères d'acrylonitrile, peut fournir des supports fonctionnalisables par les procédés de l'invention.

Deux types de bioépuration sont concernées dans la présente invention ; il s'agit, d'une part, d'une bioépuration non spécifique de type dialyse et/ou hémofiltration communément utilisée lors de la dialyse rénale ; d'autre part, la bioépuration dans laquelle on cherche à éliminer d'un fluide biologique une molécule ou macromolécule indésirable, et ce par interaction spécifique de la molécule ou des macromolécules à éliminer avec un ou des ligands couplé(s) par covalence à des supports hydrophiles.

<u>Dialyse et hémofiltration</u> :

Des appareils pour le traitement du sang par circulation extracorporelle sont utilisés dans diverses applications médicales ou paramédicales telles que : traitement de l'insuffisance rénale par dialyse ou hémofiltration, plasmaphérèse et aphérèse à visée thérapeutique et non thérapeutique, oxygénation du sang, immunoépuration, etc.

Tous les matériaux utilisés dans la fabrication de ces appareils sont choisis pour être aussi biocompatibles que possible de façon que les réactions (coagulation, notamment) qui se produisent lorsque le sang entre au contact d'un matériau étranger ne se produisent pas ou à des niveaux relativement bénins.

Il est connu de traiter, dans la masse ou en surface, les matériaux destinés à être en contact avec du sang pour en améliorer la biocompatibilité. Les traitements connus interviennent soit lors de la préparation des solutions de polymères utilisées pour fabriquer telle ou telle partie d'un appareil (traitement massique), soit après que les différentes parties de l'appareil ont été assemblées et avant stérilisation de l'appareil, soit, extemporanément, juste avant l'utilisation de l'appareil.

Un problème particulièrement ardu à résoudre se pose lorsqu'on cherche à améliorer la biocompatibilité de l'élément actif d'un appareil (membrane de dialyse, par exemple) en respectant les conditions suivantes :

1) le choix de la substance utilisée pour le traitement et les modalités du traitement doivent avoir pour résultat la

modification d'un élément actif connu, cette modification ayant pour effet d'améliorer la biocompatibilité de l'élément actif tout en préservant toutes les qualités connues (par exemple, pour une membrane de dialyse/hémofiltration : performances des transferts diffusifs et convectifs, capacité d'adsorption de substances indésirables, etc.) ;
2) la stérilisation de l'appareil ne doit pas avoir d'influence sur le traitement ;
3) le traitement ne doit pas requérir de manipulation particulière de la part de l'utilisateur.

De façon plus spécifique, l'invention a pour but de proposer un procédé de fabrication d'un dispositif satisfaisant aux conditions énoncées ci-dessus et dont l'élément actif, avant traitement, présente des charges négatives en surface. Lorsque le sang entre en contact avec une surface négativement chargée, il est le siège d'un phénomène biologique, appelé activation de la phase contact qui se manifeste par la génération de substances actives, la kallikréine et le facteur XIIa, à partir de substances inactives, prékallicréine et facteur XII.
L'activation de la phase contact est bénigne en soi, mais quand elle se produit simultanément à certains facteurs perturbateurs (prise de médicaments hypotenseurs de type IEC par le patient, dilution du sang pénétrant dans l'appareil rempli de solution saline, abaissement concomitant du pH), elle semble à l'origine de réactions indésirables, dites anaphylactoïdes qui se manifestent quelques minutes après le début du traitement par divers symptômes, parmi lesquels la sensation de chaleur généralisée, l'engourdissement des doigts, des lèvres ou de la langue, le halètement, la nausée, l'oedème laryngé. On rappelle que les réactions anaphylactoïdes ne sont pas exclusivement liées à l'utilisation d'appareils médicaux dont le compartiment sang a une surface interne chargée négativement. Ces réactions ont été observées avec des échangeurs ayant des membranes de différentes compositions chimiques, tantôt lors d'une première utilisation, tantôt après plusieurs utilisations lorsque les échangeurs, au lieu d'être jetés après un usage unique, sont réutilisés de multiples fois et sont recyclés après chaque usage. Comme exemple, d'échangeurs dont une première utilisation s'est accompagnée d'une réaction indésirable, on peut citer les dialyseurs ayant une membrane de polyméthylméthacrylate et de polyacrylonitrile. Des réactions associées à la réutilisation des dialyseurs à membrane d'acétate de cellulose et de polysulfone ont été également bien documentées (voir "Anaphylactoide réactions associated with reuse of hollow-fiber hemodialyzers and ACE inhibitors" in Kidney International, vol. 42 (1992), pp. 1232-1237).

Bioépuration spécifique

La fixation covalente de macromolécules sur des supports solides a fait l'objet de nombreux développements, plus particulièrement destinés à augmenter le taux de fixation spécifique et diminuer le taux de fixation non spécifique, par exemple par adsorption. A titre d'exemple, on peut citer les demandes de brevet WO 92/07023, 92/07006, et WO 92/05201 qui développent une technologie dans laquelle un support est recouvert d'un polymère hydrophile non chargé : le PEG (polyethylène glycol)-époxy, lié de façon covalente à une polyethylène imine (PEI). Le procédé décrit dans ces trois demandes de brevets est mis en oeuvre dans un milieu réactionnel non polaire ; le PEG époxy forme une liaison stable avec la PEI qui, par réaction directe en diminuant le pH du milieu réactionnel, se fixe sur le support insoluble. On obtient alors un support fonctionnalisé de type époxy ; néanmoins, ces supports présentent l'inconvénient d'être parfois trop hydrophiles par la présence de ces groupes hydroxyles et cet excès d'hydrophilie peut empêcher une protéine que l'on souhaite utiliser à titre de ligand de venir en contact avec les groupements époxy réactionnels ayant pour conséquence la diminution du taux de fixation du ligand sur le support.
Pour éliminer ce problème, une microémulsion contenant le ligand a été développée pour favoriser le contact entre le ligand et un support hydrophile (WO 92/07023).
Une des applications particulières envisagées depuis longtemps d'une fixation de macromolécules biologiques sur des supports est l'épuration spécifique des fluides à des fins de purification de molécules ou macromolécules. Celle-ci, dont un exemple particulier est l'immunoépuration de protéines plasmatiques, est habituellement réalisée en passant le plasma et plus rarement le sang obtenu, par prélèvement à partir de donneurs, sur un support solide sur lequel est lié un ligand capable de réagir spécifiquement et si possible avec une bonne affinité avec la molécule que l'on souhaite obtenir.
Parmi les ligands particulièrement intéressants pour la purification, se trouve la classe des anticorps monoclonaux. Par la spécificité de leur interactions avec les antigènes, et par les affinités élevées avec les antigènes correspondants, leur utilisation est éprouvée dans l'immunoépuration spécifique in vitro dans les nombreuses applications de la chromatographie d'affinité ( Current Protocol in Immunology, Section II, Unit 8-2 ). Néanmoins, l'immunoépuration extracorporelle ayant pour objectif l'épuration maximale de la substance dans le plasma diffère fondamentalement de l'immunoépuration in vitro ayant pour objectif l'obtention d'une substance purifiée et/ou concentrée, par de nombreux paramètres et notamment :

- la nécessaire biocompatibilité du support modifié comme évoqué plus haut,
- la fixation covalente du ligand par une méthode qui lui permette de conserver son intégrité fonctionnelle.
- l'affinité des anticorps pour leurs épitopes doit être telle que le relargage des antigènes dans la circulation est

minime, alors que l'immunoépuration in vitro pour l'obtention de substances purifiées doit permettre en revanche le relargage sans dénaturation desdites substances, au risque d'avoir un rendement de fixation plus faible.

Le seul produit commercial que l'on peut citer à titre d'exemple d'épuration extracorporelle est une colonne qui se compose de Protéine A greffée sur des billes de sépharose et commercialisée sous le nom de colonne Immunosorba (Excorim). La Protéine A, extraite de la paroi de S. aureus, a une affinité pour le fragment Fc des IgG, aussi, les colonnes Immunosorba sont d'une application très limitée dans la mesure où seules les IgG, mais toutes les IgG sans distinction de leur spécificité antigénique sont éliminées, ce qui n'est évidemment pas le but recherché dans la plupart des cas.

On connaît également l'article de S. Mitzner et al. : "Extracorporal endotoxin removal by immobilized polyethyleneimine" ARTIFICIAL ORGANS vol. 11, N° 9, septembre 1993 pages 775-781 qui divulgue une épuration extracorporelle d'endotoxine à l'aide de billes de cellulose hydrophile macroporeuse liées de façon covalente à de la polyéthylènimine (PEI) (page 776, colonne de droite, paragraphe "Adsorbents"). Ceci constitue l'adsorbant. Comme indiqué au dernier paragraphe de cette colonne, 3 ml de cet adsorbant sont placés dans une capsule, au travers de laquelle circule le fluide contenant les endotoxines bactériennes à éliminer.
Il est clair que :

la PEI n'est pas liée de manière ionique au support cellulosique,
la PEI n'est pas liée de manière covalente à des molécules d'un autre type exhibant des groupements carboxyliques,
la capsule selon cet article n'est pas un module de dialyse comprenant un compartiment, pour la circulation du fluide, délimité au moins partiellement par un support fonctionnalisé.

Cet article ne divulgue pas de procédé de préparation d'un support biocompatible fonctionnalisé polyvalent consistant à sélectionner le ligand et à réaliser un couplage du support avec le ligand pour obtenir un produit de couplage permettant l'épuration extracorporelle spécifique d'un fluide biologique et, par suite, le traitement thérapeutique de pathologies liées à la présence de ces molécules indésirables que l'on cherche à éliminer.

DE-A-4 209 988 correspond sensiblement au contenu de l'article de S. Mitzner et al susvisé. Dans cet adsorbant cellulosique pour endotoxines contenues dans des fluides biologiques, la PEI est utilisée seule comme revêtement du support et comme ligand (cf. colonne 2, ligne 48).

GB-A-2 197 720 décrit une plaque de microtitration sans rapport avec des dispositifs d'épuration de fluides biologiques par circulation extracorporelle. La plaque de microtitration selon GB-A-2 197 720 est un support plastique en PVC ou polystyrène comportant des puits sur les parois desquels est adsorbée de la PEI modifiée à l'aide de glutaraldéhyde, pour introduire des fonctions aldéhydes sur lesquelles se fixent des séquences connues d'ADN, aptes à se lier par hybridation à des séquences d'ADN cibles à des fins diagnostic. GB-A-2 197 720 n'enseigne pas l'existence de liaisons ioniques entre le support et la PEI. GB-A-2 197 720 ne divulgue pas de molécules d'un autre type exhibant des groupements carboxyliques permettant l'ancrage de ligands.
Les puits de la plaque de microtitration selon GB-A-2 197 720 ne sont pas des compartiments pour la circulation d'un fluide biologique. GB-A-2 197 720 ne divulgue pas non plus un procédé pour l'épuration spécifique et le traitement d'une maladie donnée en partant d'un support ou d'un module polyvalent préparé stérilement, aisément et extemporanément, par greffage du ligand spécifique approprié.

US-B-5 403 917 décrit un support poreux en verre, en silice et de préférence en cellulose, lié de manière covalente à un polysaccharide sulfaté, par exemple du sulfate de dextran.
Comme indiqué colonne 4 - lignes 28 à 31 de US-B-5 403 917, la cellulose greffée par du sulfate de dextran est logée dans un compartiment cylindrique en verre ou en plastique de 5 à 20 cm de diamètre. Le fluide duquel on cherche à éliminer le TNF ou les LPS circule au travers de ce compartiment contenant l'adsorbant. Le sulfate de dextran n'est pas lié par liaisons ioniques à la cellulose. Le sulfate de dextran ne présente de groupements carboxyliques ayant permis la fixation directe ou indirecte de manière covalente de ligands spécifiques.
US-B-5 403 917 ne décrit pas de module comprenant un compartiment prévu pour la circulation du fluide à épurer et délimité par le support fonctionnalisé. En outre, US-B-5 403 917 ne divulgue pas de moyens ni de méthode d'épuration spécifique.

L'article de Kopaciewicz, W et al. : "Synthesis of Cation-Exchange Stationary Phases Using an Adsorbed Polymeric Coating", JOURNAL OF CHROMATOGRAPHY, NETHERLANDS, 16 mai 1986, Vol. 358, No. 1, pages 107 à 117, divulgue une face stationnaire de chromatographie par échange de cations. Le support est formé par des billes de silice sur lesquelles est absorbé de la PEI, cette PEI étant ensuite réticulée à l'aide d'une solution méthanolique de diglycidol glycérol (cf. schéma 1, étape 2, figure 1, page 110 de cet article). La PEI réticulée est mise à réagir avec un anhydride d'acide carboxylique (cf. étape 3, schéma 1, figure 1) en présence de diméthylformamide et de diisopropyléthylamine DIEA.
Le support selon cet article ne porte pas de ligand spécifique pour l'épuration extracorporelle spécifique d'un fluide

biologique.
Une phase stationnaire de chromatographie par échange de cations n'est pas un support fonctionnalisé ni un module délimité par ce support pour l'épuration spécifique de fluide biologique.
De surcroît, il est à noter que la liaison entre la PEI et le support en silice selon cet article n'est pas une liaison essentiellement ionique, mais une simple adsorption. Dans cette résine échangeuse de cations, la PEI forme le ligand non spécifique.

Les inventeurs ont voulu utiliser les propriétés physiques et fonctionnelles avantageuses des systèmes existants en matière de circulation extracorporelle pour épurer les fluides biologiques, et notamment le plasma ; une telle épuration peut représenter une thérapeutique pour l'élimination de molécules ou macromolécules dont la présence conduit à certaines pathologies ou certains dysfonctionnements biologiques et plus particulièrement les molécules et macromolécules toxiques de masse moléculaire supérieure à l'albumine (66400 daltons) et non épurées par les techniques d'hémofiltration ou d'hémodialyse.

La présente invention a pour objet de fournir un support fonctionnalisé pour l'épuration extracorporelle spécifique d'un fluide biologique qui peut être conformément à l'invention:

1. Soit lié de manière covalente à un ligand lui-même apte à interagir spécifiquement avec des molécules ou des éléments du fluide biologique que l'on souhaite épurer;
2. Soit non lié mais apte à se lier à un tel ligand.

Ainsi, dans une première forme de réalisation (ligandée), le support selon l'invention est **caractérisé en ce que:**

- sa surface comprend des groupements fonctionnalisés et satisfaisant aux conditions suivantes:

  ⇒ être stables au moins à une température comprise entre 15°C et 45 °C;
  ⇒ être stables à au moins un type de procédé de stérilisation applicable aux dispositifs médicaux; et en particulier aux rayonnements gamma;

- et sa surface présente des charges, par l'intermédiaire desquelles sont liées par liaisons ioniques des molécules ou macromolécules d'un premier type exhibant, une fois liées, des groupements amines libres sur lesquels sont fixées, par liaison covalente, des molécules d'un deuxième type qui, une fois liées, exhibent des groupements carboxyliques ayant permis le couplage direct ou indirect, de manière covalente, de ligands spécifiques.

De préférence, les groupements fonctionnalisés satisfont également à la condition suivante: avoir une hydrophilie suffisante pour éviter les fixations non spécifiques de macromolécules, mais inférieure à un seuil critique au-delà duquel le taux de fixation covalente des ligands serait faible sinon nul. Le caractère hydrophile peut être intrinsèque ou conféré par modification chimique.

De préférence, les groupements carboxyliques de ce support fonctionnalisé ont formé directement ou indirectement des liaisons covalentes avec des ligands porteurs de groupements: -CHO, $-NH_2$, -COOH, -SH, -OH,

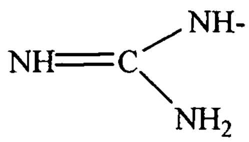

Par exemple, ces groupements carboxyliques :

1) ont formé des liaisons amides, esters et thioesters avec des groupements $NH_2$, OH et SH respectivement; ou
2) ont été modifiés en hydrazines pour former des liaisons hydrazones avec des aldéhydes; ou
3) ont été modifiés en amines pour former des imines avec des aldéhydes, ces imines pouvant être stabilisées en amines par réduction; ou
4) ont été modifiés en 1, 2 dicétone pour pouvoir former des liaisons

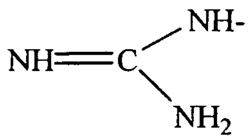

covalentes avec des guanidines.

Selon une 1ère variante, sont fixées par liaison covalente sur les groupements carboxyliques, des molécules de troisième type ayant une fonction d'espaceur pour diminuer l'encombrement stérique et favoriser la fixation ultérieure d'un ligand et/ou de la molécule ou éléments figurés à épurer.
Selon une 2ème variante, sont fixées par liaison covalente sur les groupements carboxyliques, des molécules d'un quatrième type avant pour fonction d'augmenter l'hydrophilie du support en vue de limiter les interactions non spécifiques.
Selon une 3ème variante, sont fixées par liaison covalente sur les groupements carboxyliques, des molécules d'un cinquième type exhibant, une fois liées, des groupements d'une autre nature, permettant un autre type de couplage covalent avec un ligand porteur notamment de groupements - CHO ou -COOH.
Dans une seconde forme de réalisation (non ligandée), le support selon l'invention est **caractérisé en ce que :**

- sa surface comprend des groupements fonctionnalisés et satisfaisant aux conditions suivantes:

  ⇒ être stables au moins à une température comprise entre 15°C et 45 °C;
  ⇒ être stables à au moins un type de procédé de stérilisation applicable aux dispositifs médicaux; et en particulier aux rayonnements gamma;

- et sa surface présente des charges, par l'intermédiaire desquelles sont liées par liaisons ioniques des molécules ou macromolécules d'un premier type exhibant, une fois liées, des groupements amines libres sur lesquels sont fixées, par liaison covalente, des molécules d'un deuxième type qui, une fois liées, exhibent des groupements carboxyliques sur lesquels sont fixés par liaison covalente:

  o des molécules de troisième type ayant une fonction d'espaceur pour diminuer l'encombrement stérique et favoriser la fixation ultérieure d'un ligand et/ou de la molécule et/ou éléments figurés à épurer;
  o et/ou des molécules d'un quatrième type ayant pour fonction d'augmenter l'hydrophilie du support en vue de limiter les interactions non spécifiques;
  o et/ou des molécules d'un cinquième type exhibant, une fois liées, des groupements d'une autre nature, permettant un autre type de couplage covalent avec un ligand porteur notamment de groupements -CHO ou -COOH.

Qu'il soit ligandé ou non, le support fonctionnalisé selon l'invention, peut avantageusement être une membrane semi-perméable fabriquée à partir d'un copolymère d'acrylonitrile et de méthallyle sulfonate de sodium.
S'agissant du ligand, il est avantageusement sélectionné dans un groupe constitué d'anticorps, d'antigènes, de peptides, de protéines, ou glycoprotéines, des hormones, des enzymes, de cofacteurs de ceux-ci, des substrats ou des inhibiteurs de ceux-ci, des polysaccharides, des lectines, des toxines ou des antitoxines, des acides nucléiques ou polynucléotides, des haptènes ou des ligands d'haptènes, des pigments ou colorants.
Avantageusement, la molécule du premier type est une polyéthylèneimine (PEI) de masse moléculaire comprise entre 10 000 et 2 000 000 daltons, la PEI étant substituée ou non.
Avantageusement, la molécule de deuxième type est un anhydride d'un acide dicarboxylique de formule:

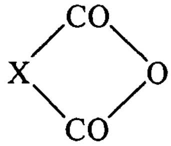

dans laquelle X = $[CH_2]_n$, n étant égal à 2 ou 3, ou X= -CH=CH-.
La molécule de deuxième type est par exemple un anhydride d'un acide monocarboxylique de formule:

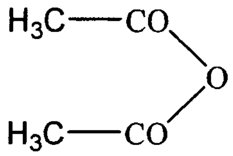

Selon une possibilité, une seule molécule remplit les fonctions des troisième, quatrième, et cinquième types de molécule, ladite molécule étant une dihydrazine de formule:

$NH_2NHCO\text{-}Y\text{-}CO\text{-}NH\text{-}NH_2$

où Y est de préférence un groupement $(CH_2)_m$ dans lequel m est compris entre 2 et 6.
Avantageusement, le support selon l'invention entre dans la constitution d'une membrane notamment une membrane de dialyse sous forme de films plans ou de fibres, poreux ou non, pleines ou creuses, des microbilles poreuses ou non ou d'une combinaison de ceux-ci.
Il peut s'agir d'une membrane de dialyse destinée à la dialyse rénale ou à l'hémofiltration.
Selon un autre de ses aspects, l'invention a pour objet un module pour l'épuration extracorporelle spécifique d'un fluide biologique **caractérisé en ce qu**'il comprend au moins un compartiment pour la circulation dudit fluide, ce compartiment étant délimité au moins partiellement par un support fonctionnalisé tel que défini ci-avant.
L'application de ce module vise l'épuration extracorporelle spécifique d'un fluide biologique, et peut être élargie à l'épuration extracorporelle non spécifique d'un fluide biologique. Dans ce cas, le support fonctionnalisé de ce module polyvalent est un support fonctionnalisé:

- dont la surface comprend des groupements fonctionnalisés et satisfaisant aux conditions suivantes:

  ⇒ être stables au moins à une température comprise entre environ 15°C et environ 45 °C;
  ⇒ être stables à au moins un type de procédé de stérilisation applicable aux dispositifs médicaux; et en particulier aux rayonnements gamma;

- et dont la surface présente des charges, par l'intermédiaire desquelles sont liées par liaisons ioniques des molécules ou macromolécules d'un premier type exhibant, une fois liées, des groupements amines libres sur lesquels sont fixées, par liaison covalente, des molécules d'un deuxième type qui, une fois liées, exhibent des groupements carboxyliques aptes à former, directement ou indirectement, des liaisons covalentes avec des ligands porteurs de groupements:
  $-CHO$, $-NH_2$, $-COOH$, $-SH$, $-OH$,

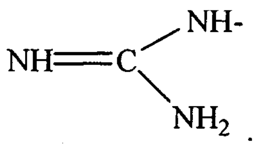

L'invention vise aussi un module pour l'épuration extracorporelle non spécifique d'un fluide biologique, le support dudit module étant "ligandé" et présentant des molécules de 3ème type et/ou de 4ème type et/ou de 5ème type, c'est-à-dire un support fonctionnalisé :

- dont la surface comprend des groupements fonctionnalisés et satisfaisant aux conditions suivantes:

  ⇒ être stables au moins à une température comprise entre 15°C et 45 °C;
  ⇒ être stables à au moins un type de procédé de stérilisation applicable aux dispositifs médicaux; et en particulier aux rayonnements gamma;

  - et dont la surface présente des charges, par l'intermédiaire desquelles sont liées par liaisons ioniques des molécules ou macromolécules d'un premier type exhibant, une fois liées, des groupements amines libres sur

lesquels sont fixées, par liaison covalente, des molécules d'un deuxième type qui, une fois liées, exhibent des groupements carboxyliques sur lesquels sont fixés par liaison covalente:

o des molécules de troisième type ayant une fonction d'espaceur pour diminuer l'encombrement stérique et favoriser la fixation ultérieure d'un ligand et/ou de la molécule et/ou éléments figurés à épurer;
o et/ou des molécules d'un quatrième type ayant Pour fonction d'augmenter l'hydrophilie du support en vue de limiter les interactions non spécifiques;
o et/ou des molécules d'un cinquième type exhibant, une fois liées, des groupements d'une autre nature, permettant un autre type de couplage covalent avec un ligand porteur notamment de groupements -CHO ou -COOH.

Dans l'ensemble du texte, on appellera support fonctionnalisé toute paroi ou surface comportant des groupements stables à température ambiante et pendant une durée compatible avec une utilisation commerciale et aptes, en présence d'une substance activatrice, générer des groupements fonctionnels à eux-mêmes aptes à réagir avec des groupements organiques de type -CHO, -NH2,-COOH, -SH , en vue du couplage direct ou indirect d'un ligand, lui-même apte à interagir spécifiquement avec des molécules ou des éléments figurés du fluide biologique que l'on souhaite épurer.
Le module pour l'épuration extracorporelle d'un fluide biologique peut également comprendre un support fonctionnalisé dont la surface présente des charges, par l'intermédiaire desquelles sont liées par des liaisons ioniques, des molécules ou macromolécules d'un premier type exhibant, une fois liées des groupements amines libres sur lesquels sont fixés, par liaison covalente, des molécules d'un deuxième type qui, une fois liées, exhibent des groupements carboxyliques libres 1) aptes à former des liaisons amides, esters et thioesters avec des groupements $NH_2$, OH et SH respectivement ; ou 2) aptes à être modifiés en hydrazines pour former des liaisons hydrazones avec des aldéhydes ; ou 3) aptes à être modifiés en amines pour former des imines avec des aldéhydes et qui peuvent être stabilisées en amines par réduction ; ou 4) aptes à être modifiés en 1, 2 dicétone (par exemple cyclohexandione, glyoxal, etc...) pour pouvoir former des liaisons covalentes avec des guanidines

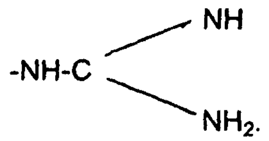

Pour l'application à la bioépuration spécifique, les groupements carboxyliques libres peuvent former des liaisons amides soit avec le ligand permettant la bioépuration spécifique, soit avec une molécule d'un troisième type qui peut avoir une ou plusieurs des fonctions suivantes:

- ajouter un espaceur pour diminuer l'encombrement stérique et favoriser la fixation ultérieure d'un ligand et/ou de la molécule ou éléments figurés à épurer,
- augmenter l'hydrophilie de la membrane ou du support en vue de limiter les interactions non spécifiques avec des protéines ou avec toute substance susceptible de telles interactions,
- exhiber un groupement d'une autre nature, par exemple un groupement aminé, permettant un autre type de couplage covalent avec un ligand porteur notamment de groupements - CHO, -COOH, $-NH_2$.

Ces différentes fonctionnalités peuvent être cumulées et se retrouver dans une seule molécule. Un exemple avantageux de molécule du troisième type qui associe les trois fonctionnalités ci-dessus est une di-hydrazide de formule:

$$NH_2 - NH - CO - Y - CO - NH - NH_2,$$

ou Y est de préférence un groupement $(CH_2)_m$ dans lequel m est compris entre 2 et 6.
En outre, Y doit être choisi de telle façon qu'il doit être porteur de groupements hydrophiles, il ne doit en aucun cas être activateur du complément, et doit être stables à au moins une méthode de stérilisation de dispositifs médicaux, tels les rayons gamma.
L'utilisation d'un module selon l'invention, dans un circuit extra-corporel, implique l'existence de propriétés intrinsèques desdits supports, qui sont :

- une adsorption non spécifique minimale,
- une absence d'activation de la phase contact, c'est-à-dire une génération de kallicréine inférieure à 10 Unités par litre ;

- une non-toxicité.

En outre, pour l'utilisation en bioépuration spécifique, ils doivent présenter une forte capacité de couplage avec les ligands spécifiques.
Les modules confectionnés à partir desdits supports doivent permettre la circulation d'un fluide biologique dans des conditions compatibles avec l'approche thérapeutique envisagée.
Les modules de l'invention comprendront de préférence comme membrane, une membrane semi-perméable fabriquée à partir d'un copolymère d'acrylonitrile et de méthallyle sulfonate de sodium, de type AN69.
La présente invention porte également sur un procédé de préparation d'un support biocompatible fonctionnalisé porteur de groupements aptes à former des liaisons covalentes avec des groupements organiques, notamment :
-CHO, $-NH_2$, -COOH, -SH, -OH,

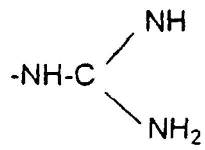

et comprenant les étapes suivantes :

a) mise en contact de supports portant des groupements anioniques fixes avec une polyéthylénimine (PEI) de masse moléculaire comprise entre 10 000 et 2 000 000,daltons, la PEI étant substituée ou non ;
b) mise en contact du support traité en a) avec une solution d'un anhydride d'acide dicarboxylique de formule :

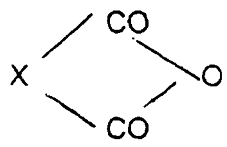

dans laquelle X = $[CH_2]_n$, n étant égal à 2 ou 3, ou X = -CH = CH-,
ou d'un anhydre d'acide monocarboxylique comme celui de l'acide acétique de formule :

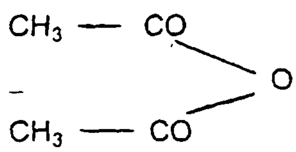

Le groupement carboxylique terminal peut être utilisé directement pour le couplage d'un ligand, soit en ajoutant un carbodiimide, soit en convertissant le groupement carboxyle en ester activé sous forme de N. hydroxy succinimide.
c) le cas échéant, mise en contact du support modifié par les étapes a) et b) avec une di-hydrazide de formule :

$NH_2$ - NH -CO- Y - CO - NH - $NH_2$, en présence d'un agent de couplage activateur des groupements carboxyles tels que les carbodiimides.
Y est choisi parmi les groupements qui n'activent pas le complément et d'être stables à au moins un type de procédé de stérilisation de dispositifs médicaux comme les rayonnements $\gamma$. Y est de préférence un groupement $[CH_{2]m,}$ dans lequel m est compris entre 1 et 4.

Cette première variante du procédé de l'invention est appelée dans la suite du texte "post-acylation".
Le procédé de l'invention peut comporter une variante conduisant à un résultat équivalent dans laquelle l'anhydride carboxylique de l'étape b) a réagi avec la PEI de l'étape a) avant le traitement du support tel proposé dans l'étape a). ; autrement dit, dans cette variante un support biocompatible fonctionnalisé porteur de groupements aptes à former des liaisons covalentes avec des groupements organiques, notamment :

-CHO, $-NH_2$, -COOH, -SH, -OH,

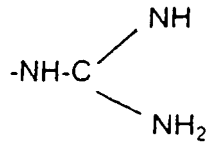

peut être obtenu par la mise en contact de supports à caractère ionique avec le produit de la réaction entre : une polyethylenimine (PEI) de masse moléculaire comprise entre 10000 et 2.000000 daltons ladite PEI étant substituée ou non, avec une solution d'un anhydride d'acide dicarboxylique de formule :

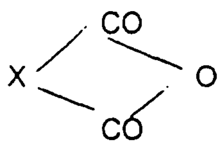

dans laquelle X à la même signification que ci-dessus,
ou d'un anhydre d'acide monocarboxylique de formule:

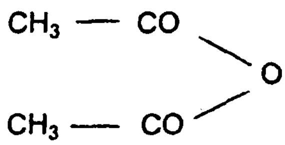

Le support ainsi modifié est alors mis en contact avec une di-hydrazide de formule:
$NH_2$ - NH -CO-Y- CO - NH - $NH_2$,
Y ayant la même signification que ci-dessus.

En d'autres termes, dans ce procédé de préparation, le support est obtenu :

- par réaction de supports à caractère ionique avec:

  → soit

    a) de la polyéthylèneimine (PEI) de masse moléculaire comprise entre 10.000 et 2.000 000, substituée ou non; puis avec
    b) une solution d'un anhydride d'acide carboxylique de formule:

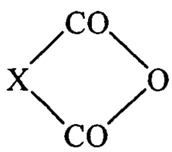

dans laquelle X = $[CH_2]n$, n étant compris entre 1 et 4,
ou X = -CH=CH-,
ou de formule:

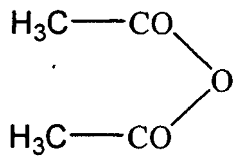

→ soit avec le produit de la réaction entre a) et b):

- et éventuellement enfin :

  → soit c) par fixation par liaison covalente sur les groupements carboxyliques:

  - des molécules de troisième type ayant une fonction d'espaceur pour diminuer l'encombrement stérique et favoriser la fixation ultérieure d'un ligand et/ou de la molécule et/ou éléments figurés à épurer;
  - et/ou des molécules d'un quatrième type avant pour fonction d'augmenter l'hydrophilie du support en vue de limiter les interactions non spécifiques:
  - et/ou des molécules d'un cinquième type exhibant, une fois liées, des groupements d'une autre nature, permettant un autre type de couplage covalent avec un ligand porteur notamment de groupements - CHO ou -COOH;

  → soit par mise en contact du support ainsi modifié avec une di-hydrazide de formule:

  $NH_2$-NH-CO-Y-CO-NH-$NH_2$,

  Y étant choisi parmi les groupements qui ont la double propriété d'être porteurs de groupements hydrophiles et d'être stables aux rayonnements $\gamma$, et qui sont de préférence un groupement $[CH_2]_m$, dans lequel m est compris entre 2 et 6.

Dans l'un quelconque des procédés ci-dessus, le support à caractère anionique est de préférence une membrane constituée d'un copolymère acrylonitrile et de méthallyle sulfonate de sodium, de type AN69 tel que décrit plus haut. La PEI a de préférence une masse moléculaire comprise entre 10 000 et 2 000 000 daltons.

L'anhydride d'acide dicarboxylique est de préférence celui de l'acide succinique de formule :

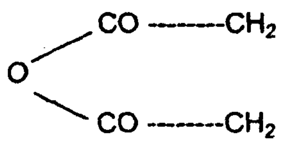

L'anhydride est utilisée en solution de concentration de 0,5 M à 2 M dans l'acétonitrile. Cette deuxième variante est appelée « pré-acylation », pour bien la différencier de la première.

Après le couplage soit de l'anhydride acétique, soit de l'anhydride succinique à la PEI par une liaison amide, l'ensemble est mis en contact avec le support anionique. Dans le cas du traitement avec la PEI présuccinylée, les fonctionnalités -COOH peuvent réagir avec la dihydrazide qui est de préférence l'ADH (adipic-dihydrazide) dans laquelle m est égale à 4.

L'invention porte plus généralement sur les supports susceptibles d'être obtenus par le procédé ou sa variante décrits ci-dessus, ainsi que sur les modules de bioépuration qui en comprennent. Ces supports doivent satisfaire aux mêmes conditions que celles énumérées ci-dessus.

Les procédés de l'invention décrits ci-dessus peuvent être mis en oeuvre pour la préparation de membranes de dialyse et/ou d'hémofiltration. L'invention concerne les membranes susceptibles d'être obtenues par ces procédés, qu'ils consistent en une post-acylation de la PEI ou une pré-acylation de cette dernière.

Les modules de dialyse comprenant les membranes ainsi réalisées font partie de l'invention.

Selon un autre de ses aspects, l'invention a pour objet un procédé de préparation d'un support biocompatible fonctionnalisé polyvalent pour l'épuration extracorporelle spécifique d'un fluide biologique, ou d'un module polyvalent pour l'épuration extracorporelle spécifique d'un fluide biologique, ce module comprenant au moins un compartiment

pour la circulation dudit fluide, ce compartiment étant délimité au moins partiellement par le susdit support fonctionnalisé,

→ ce support étant tel que:

- sa surface comprend des groupements fonctionnalisés et satisfaisant aux conditions suivantes:

  ⇒ être stables au moins à une température comprise entre 15°C et 45°C:
  ⇒ être stables à au moins un type de procédé de stérilisation applicable aux dispositifs médicaux: et en particulier aux rayonnements gamma:

- et que sa surface présente des charges, par l'intermédiaire desquelles sont liées par liaisons ioniques des molécules ou macromolécules d'un premier type exhibant, une fois liées, des groupements amines libres sur lesquels sont fixées, par liaison covalente, des molécules d'un deuxième type qui, une fois liées, exhibent des groupements carboxyliques permettant le couplage direct ou indirect, de manière covalente, de ligands spécifiques;

→ et cette épuration extracorporelle spécifique d'un fluide biologique représentant une thérapeutique pour l'élimination de molécules ou macromolécules dont la présence conduit à certaines pathologies ou certains dysfonctionnements biologiques;

**caractérisé en ce qu**'il consiste

a. à sélectionner le ligand ou le mélange de ligands appropriés en fonction de la pathologie donnée;
b. à réaliser un couplage du support avec le ligand ou les mélanges de ligands, pour obtenir un produit de couplage.

Les ligands susceptibles d'être couplés aux supports fonctionnalisés peuvent être de nature homogène ou hétérogène, c'est-à-dire qu'ils peuvent être constitués d'une seule espèce moléculaire ou d'un mélange compatible de plusieurs espèces moléculaires si l'on cherche à épurer le fluide biologique de plusieurs molécules simultanément, ou d'une seule molécule avec des ligands qui ont une affinité différente pour différents sites de celle-ci ; par exemple il peut s'agir, quand le ligand est un anticorps ou un fragment d'anticorps, d'un mélange d'anticorps de spécificités différentes. La mise en contact d'un fluide biologique avec le support ainsi modifié par un couplage covalent desdits ligands permet d'éliminer sélectivement une ou plusieurs espèces moléculaires ou macromoléculaires, ou élément figuré du fluide biologique, présentant une affinité avec le ou les ligands.
Les ligands seront fixés de façon covalente par tout moyen connu, par action d'une substance activatrice compatible avec une utilisation extracorporelle du support modifié. De tels moyens sont décrits dans les documents suivants : brevet US 4 217 338 ; Thomas et al., Colloids and surfaces A. (1993) vol. 77;125-139 ; Malmsten et al. J. Colloid and Interface Science. (1996) vol. 177;70-78.
Les chiffres entre parenthèses correspondent à des références bibliographiques dont la liste, pour l'ensemble du texte, est donnée en fin de description.
Quand les ligands sont des anticorps, deux approches sont possibles pour ce faire ; la première consiste à coupler les anticorps par liaison amide entre les groupements aminés des anticorps et les groupements carboxyle des supports.
La deuxième consiste à oxyder les anticorps par le periodate de sodium, puis à les coupler par formation d'une liaison hydrazone entre des groupements aldéhyde engendrés sur les groupements glucidiques de la molécule et les acides hydrazides du support fonctionnalisé par les di-hydrazides ; l'efficacité de couplage dans ces conditions est maximale quand le pH est compris entre 4 et 6.
Un exemple de chacune de ces approches est représenté dans les figures 1 et 2.
Il serait fastidieux et vain d'énumérer toutes les situations dans lesquelles une bioépuration des fluides biologiques présenterait un intérêt thérapeutique, d'autant que l'évolution constante des connaissances sur les processus métaboliques et pathologiques fournira constamment de nouveaux candidats à l'épuration ; l'homme du métier saura choisir et sélectionner le ligand ou le mélange de ligands appropriés en fonction de la décision thérapeutique qu'il aura prise ; par exemple, il pourra, le cas échéant, associer des mélanges d'anticorps de spécificités épitopiques ou antigéniques différentes dans le cas d'épuration de molécules ou de mélanges complexes ; il pourra enfin choisir d'épurer le fluide biologique par un ligand qui présente une affinité non pour la substance à épurer mais pour une autre substance complexée spécifiquement à celle que l'on souhaite épurer.
Les anticorps peuvent être des anticorps polyclonaux ou de préférence des anticorps monoclonaux, soit monospécifiques, soit en mélange de plusieurs spécificités épitopiques ou moléculaires. A titre d'exemple, on pourra citer :

- les anticorps anti-cytokines pro-inflammatoires (IL-1β ; TNFa ; IL-6) dans le traitement du choc septique ou dans le traitement de phénomènes inflammatoires tels la polyarthrite rhumatoïde ;
- les anticorps anti RhD dans le traitement ou la prévention de la maladie hémolytique du nouveau-né ;
- les anticorps anti-HLA, en prophylaxie de greffes d'organes

On pourra également choisir d'épurer spécifiquement des éléments figurés du sang tels les cellules ou les plaquettes à l'aide de ligands appropriés comme les anticorps, les récepteurs, les lectines etc...

Les produits de couplage entre les supports fonctionnalisés de l'invention avec les ligands et notamment les anticorps monoclonaux ou polyclonaux sont nouveaux et font également partie de l'invention, ainsi que leur utilisation dans l'épuration de fluides biologiques.

Afin d'être efficace, le produit de couplage doit être caractérisé par une quantité optimale d'anticorps fixée variable pour chaque type d'antigènes. A titre d'exemple, on peut fixer environ 2 mg/m2.

Les antigènes représentent également une classe de candidats intéressants pour l'épuration des fluides biologiques, notamment dans le cas de maladies autoimmunes pour l'élimination des autoanticorps.

Les maladies autoimmunes représentent aujourd'hui le troisième grand processus pathologique après les maladies cardio-vasculaires et les cancers, et pour lesquelles seuls des traitements symptomatiques existent.

Les maladies autoimmunes sont avant tout marquées par une hyperactivité des lymphocytes B et se caractérisent parmi d'autres symptômes biologiques, par la présence de multiples autoanticorps dirigés contre des autoantigènes. A titre d'exemple, on peut citer des anticorps anti-antigènes nucléaires variés, des anticorps anti ADN, des anticorps contre des enzymes et des nucléoprotéines intervenant dans les processus de transcription et de traduction de l'ARN. Des exemples typiques de maladies autoimmunes sont ceux du Lupus érythemateux disséminé (LED) ou de la polyarthrite rhumatoïde. La grande diversité des autoanticorps qui peuvent être présents dans plusieurs maladies systémiques rend le diagnostic très difficile d'autant que cliniquement les symptômes sont parfois mêlés.

Si la production d'autoanticorps semble être une caractéristique chez les patients atteints de maladies autoimmunes, il est intéressant de constater que cette production augmente aussi avec l'âge chez des individus sains (8, 14, 18) sans pour autant que ces individus ne développent de pathologies autoimmunes. Par ailleurs, les autoantigènes cibles (ADN, histones, enzymes et nucléoprotéines) des autoanticorps produits chez ces mêmes personnes sont les mêmes que ceux rencontrés dans les maladies autoimmunes, et notamment dans le LED (11).

En revanche, de nombreux travaux font état du caractère pathogène des immuncomplexes. En effet, il a été démontré que l'insuffisance rénale qui est l'une des causes ultimes de mortalité chez les malades atteints de LED est induite par les dépôts d'immun-complexes dans les reins et notamment d'immun-complexes ADN/anti ADN (9, 10). Récemment Sasaki et al. ont apporté un certain nombre d'éléments conduisant à associer la présence d'immuns-complexes ADN/antiADN à l'incidence de la glomérulonéphrite proliférative diffuse d'où l'intérêt d'éliminer, avant toute complication rénale, les antigènes et les anticorps impliqués dans la formation d'immun-complexes circulant afin de réduire leur formation chez les malades atteints.

Les différentes voies thérapeutiques actuellement utilisées sont principalement :

a) le traitement par des glucocorticoïdes qui entraînent à forte dose (de 0,7 à 1 ml/kg) une amélioration clinique franche avec diminution du taux des divers autoanticorps, disparition des immun-complexes et remontée du taux de complément sérique ;

b) les immunosuppresseurs, dont l'utilisation est plus controversée ;

c) les échanges plasmatiques ou plasmaphérèses ;

Si cette dernière technique s'est avérée efficace pour quelques patients lupiques, elle présente de nombreux inconvénients : elle est onéreuse, de mise en oeuvre délicate, l'élimination est non sélective car elle épure non seulement les immun-complexes pathogènes mais, aussi, des constituants essentiels tels que les immunoglobulines, les facteurs de la coagulation, les molécules du complément, et beaucoup d'autres composés ayant une activité régulatrice. Elle est donc beaucoup plus traumatisante pour l'organisme qu'une technique spécifique.

Enfin, le traitement est souvent suivi de phénomènes de « rebonds » où les taux de complexe immuns et d'autoanticorps augmentent dramatiquement quelques jours après l'arrêt du traitement.

d) l'aphérèse qui est habituellement réalisée en passant le plasma ou plus rarement le sang sur un support solide sur lequel est lié un ligand capable de réagir spécifiquement et si possible avec une grande affinité avec les sites présents sur les cellules cibles ou sur les constituants de faible ou haut masse moléculaire.

Dans le cas du LED, une voie thérapeutique possible consiste à éliminer spécifiquement les anticorps pathogènes en l'occurrence les anticorps anti-ADN par ce procédé. Les premières données avaient été fournies par Terman et al. qui avaient utilisé une colonne d'ADN sous forme colloïdale pour épurer le sang de patients lupiques. Cependant, l'usage de l'ADN comme ligand reste très limité en raison, d'une part, de son instabilité au contact d'enzymes telles les nucléases et éstérases plasmatiques et, d'autre part, en raison du danger potentiel lié à son caractère potentiellement

transformant que pourrait présenter son relargage dans la circulation, complexé à des protéines sériques. Néanmoins, les anticorps anti-ADN peuvent être spécifiquement piégés non pas par l'ADN mais par d'autres composés avec lesquels ces anticorps donnent des réactions croisées. De nombreux auteurs parmi lesquels Lafer et al. puis Shoenfeld ont montré que les anticorps anti-ADN murin et humain sont capables de réagir avec des composés très différents de l'ADN telle que la cardiolipine ou d'autres molécules phospholipidiques négativement chargées ; Susuki et al. ont conçu un système d'aphérèse en continu sur gel de sulfate de dextran couplé à un système de regénération du support et mis en évidence une diminution du taux d'anticorps anti-ADN chez des patients lupiques. Cependant, l'intérêt de cette stratégie thérapeutique paraît limité par le fait que seuls les anticorps anti-ADN sont partiellement éliminés et qu'ils ne sont pas les seuls auto-anticorps à l'origine de lésions rencontrées dans le lupus.

L'acide lipoïque (AL) ou acide lipoïque 6,8 dithiooctanoïque (AT) est une molécule à huit atomes de carbone liée à deux atomes de soufre sur les carbones 6 et 8 pour former un hétérocycle dithiolane.

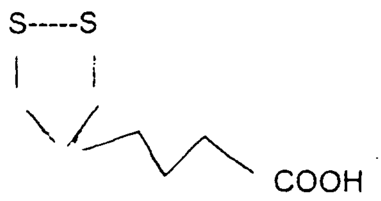

Sur le plan métabolique, il constitue un cofacteur essentiel pour des oxo acides deshydrogénases que sont les complexes multi-enzymatiques essentiel pour des enzymes comme la pyruvate deshydrogénase (PDH) ou oxoglutarate deshydrogénase auxquelles il se lie de façon covalente aux groupements NH2 de la lysine (1, 2, 21).

Sur le plan immunologique, il a été clairement montré que la partie de la PDH correspondant au site de fixation de l'acide lipoïque constitue un autoantigène susceptible d'être reconnu par des autoanticorps chez des malades atteints de cirrhose biliaire primitive (7). Récemment, des auteurs ont montré que la présence de l'acide lipoïque sur cet autoantigène est déterminante pour cette reconnaissance chez ces malades (6, 7).

Toujours dans le domaine immunologique, il a été démontré que les composés porteurs des groupements sulfhydryles, parmi lesquels figurent le 2-mercaptoéthanol et le dithiothréitol, sont capables de stimuler la production d'anticorps chez des lymphocytes en culture (3, 4, 15, 17).

Ohmori a étudié in vitro l'effet de l'AL dans le but de définir son éventuel caractère immunostimulant (16). Il apparaît évident que l'augmentation de la réponse anticorps consécutive à une stimulation antigénique en présence d'AL est dépendante de la population T. En effet, une déplétion en lymphocytes T auxiliaires annule l'action de l'AL. Par ailleurs, la stimulation obtenue est spécifique de l'antigène utilisé.

Le choix de l'acide lipoïque comme ligand dans le traitement du LED par immunoépuration a été fondé sur les observations suivantes :

1) les sérums de malades atteints de LED contiennent un taux élevé d'anticorps anti-AL de classe IgG mais surtout de classe IgM, à la différence des sujets normaux comme cela apparaît dans la figure 3.
2) Le taux d'anticorps anti-AL augmente dans le sérum de souris MRL/lpr avec l'âge : en outre ces souris développent spontanément le LED en vieillissant. Une étude comparative des taux d'anticorps anti-AL chez les souris MRL/Ipr et des souris contrôles appelées MRL/mp en fonction de l'âge des animaux fait apparaître clairement que le titre en anticorps anti-AL augmente à partir de la douzième semaine chez la souris MRL/lpr, tandis que ce taux reste faible chez la souris contrôle MRL/mp. Les résultats sont représentés dans la figure 4.
3) Des anticorps anti-AL réagissent avec de l'ADN double brin, mais pas avec l'ADN simple brin (13) ; or, le LED est caractérisé par la présence à un moment donné de la maladie d'anticorps anti-ADN double brin.
4) L'épuration des anticorps anti-AL à partir d'un sérum de patient malade du LED diminue simultanément le taux d'anticorps anti-ADN double brin (figure 5).

En fonction des observations et des résultats exposés ci-dessus, il apparaissait que l'acide lipoïque était un excellent candidat pour épurer le plasma en anticorps anti-AL et anti-ADN dans la pathologie lupique.

L'acide lipoïque est susceptible d'être couplé au support fonctionnalisé par liaison amide avec des groupements aminés conférés, par une monohydrazide, par une dihydrazide, ou par une diamine de formule $NH_2$ - $(CH_2)_n$-$NH_2$ avec n compris entre 2 et 6, ou une polyamine de formule $NH_2$ - $(CH_2)$x - NH- $(CH_2)$y - $NH_2$ avec x et y compris 3 et 6, par exemple. Les exemples de réalisation de couplage selon l'invention avec l'acide lipoïque pour le traitement du LED seront donnés dans les exemples de réalisation ci-dessous.

L'invention concerne également le produit de couplage entre un support fonctionnalisé selon l'invention avec l'acide lipoïque, ainsi que son utilisation pour épurer le sérum de patients atteints de LED. Des dispositifs comprenant

ce produit de couplage sont particulièrement performants pour traiter les maladies autoimmunes, et plus particulièrement le LED.

L'homme du métier saura, selon les cas, sélectionner le ligand approprié à la thérapeutique d'une pathologie donnée en le sélectionnant dans un groupe constitué, outre les anticorps et les antigènes décrits ci-dessus, de peptides, de glycoprotéines, d'hormones, des enzymes, des cofacteurs de ceux-ci, des substrats ou des inhibiteurs de ceux-ci, des polysaccharides, des lectines, des toxines ou des antitoxines, des acides nucléiques ou polynucléotidiques, des haptènes, des ligands d'haptène, des pigments ou des colorants.

Le traitement d'une pathologie donnée pourra être réalisé par un mélange de ligands, soit simultanément, soit séquentiellement, par utilisation de plusieurs produits de couplage.

la présente invention porte sur l'utilisation d'un support fonctionnalisé tel que décrit ci-dessus et résultant de la mise en oeuvre des procédés de l'invention à la fabrication d'un module polyvalent destiné à l'épuration extracorporelle de fluides biologiques et dans lequel sont épurées des espèces biochimiques ou des cellules, présentes dans des fluides biologiques, et dont l'élimination est recherchée, en particulier des antigènes, des anticorps ou des haptènes.

L'invention vise également un dispositif de circulation extracorporelle de sang incorporant un support ou un module selon l'invention.
De préférence, ce dispositif est un dispositif de traitement thérapeutique pour l'élimination de molécules ou macromolécules dont la présence conduit à certaines pathologies ou certains dysfonctionnements biologiques.

La présente invention porte également sur un kit polyvalent de préparation d'un produit de couplage selon l'invention, entre le support fonctionnalisé et un ou plusieurs ligands spécifiques pour l'épuration des fluides biologiques, ledit kit comportant au moins :

1 - un dispositif porteur d'un support susceptible d'être couplé de façon covalente à un ligand ,
2 - un récipient ou poche stérile contenant une solution activatrice de la réaction entre le support fonctionnalisé et le ligand ;

- le ligand dans une solution et une concentration appropriées ;
- le cas échéant une ou des solutions de rinçage.

Plus précisément, ce kit peut comprendre :

- un module polyvalent selon l'invention,
- un récipient ou poche stérile contenant une solution activatrice de la réaction entre le support fonctionnalisé et un ligand,

le cas échéant un récipient ou poche stérile contenant une solution du ligand choisi;
le cas échéant les solutions tampons ou de rinçages nécessaires à l'activation et/ou au couplage covalent du ligand.

Le kit de l'invention dont un mode de réalisation est représenté sur la figure 10 consiste à proposer une solution thérapeutique associant un support modifié susceptible d'être obtenu par un procédé décrit ci-dessus, la porosité et la modification du support étant adaptées au ligand et à la nature et à la concentration de la ou des molécule(s), ou des éléments figurés que l'on cherche à éliminer dans le fluide biologique.

Dans le kit, le dispositif pourra avantageusement être une cartouche de dialyse, et plus particulièrement une cartouche d'AN 69 fonctionnalisée telle que décrite plus haut.

Quand le ligand du kit de l'invention est l'acide lipoïque, et que les groupements présents sur le support sont des groupements aminés, la solution activatrice sera de préférence la carbodiimide sous forme hydrosoluble.

Quand le ligand du kit de l'invention est un anticorps, la solution activatrice est par exemple le periodate de Na. Les réactions mises en jeu dans les couplages sont des réactions classiques bien connues de l'homme du métier qui pourra mettre en oeuvre les conditions opératoires les plus performantes, tant par la nature de l'activateur que par les conditions physicochimiques de la réaction, en fonction du ligand choisi.

Le kit de l'invention pourra être avantageusement utilisé par un personnel soignant ou un personnel qualifié associé à ceux-ci pour préparer de façon stérile et extemporanée une cartouche porteuse d'un ligand spécifique et utilisable en circulation extracorporelle pour l'épuration d'un fluide biologique qui sera de préférence le sang ou le plasma.

L'invention porte sur l'utilisation d'un kit tel que décrit ci-dessus dans tout traitement prophylactique ou thérapeutique d'un mammifère et plus particulièrement des humains.

L'invention porte enfin sur un procédé de bioépuration spécifique de sang ou de plasma humain ou animal par circulation extracorporelle d'un dispositif comportant au moins un module de l'invention. Dans la représentation schématique de la figure 11, du sang total est traité par circulation extracorporelle, par passage dans un module de l'invention

et réadministration au patient. Dans le cadre d'un tel traitement, il y a possibilité d'ultrafiltrer une partie du sang si nécessaire.

D'autres caractéristiques et avantages de l'invention apparaîtront dans les exemples qui suivent et en se reportant aux figures 1 à 23.

La figure 1 représente à titre d'illustration le couplage d'une molécule de faible masse moléculaire, la glycine, ou d'un anticorps à un support fonctionnalisé à la PEI avec les groupements aminés succinylés.

La figure 2 représente un couplage similaire par liaison hydrazone à une molécule de faible poids moléculaire (pyruvate) ou de haut poids moléculaire (anticorps oxydé).

La figure 3 présente un titre d'anticorps antiacide lipoïque dans le sérum de souris MLR . En gris clair est représenté le taux dans les souris MRL/lpr et en gris foncé hachuré, le taux obtenu chez les souris MRUmp.

La figure 4 représente la comparaison des taux d'IgM anti-acide lipoïque dans différentes pathologies. C représente le contrôle, PBC, la cirrhose biliaire primitive, M, la Myasténie, RA, l'arthrite rhumatoïde, SLE, représente le lupus érythémateux disséminé, S, la syphilis et TH la thyroïdite.

La figure 5 représente la diminution des anticorps anti-ADN épurés après passage d'un sérum lupique sur un support laquelle est couplée de façon covalente l'acide lipoïque.

La figure 6 représente le nombre de sites disponibles après fonctionnalisation de l'AN69-PEI par l'anhydride succinique. La courbe représente la quantité des anhydrides obtenues par rapport à la quantité d'éthanolamine introduit sur l'AN69 seul succinylé (représenté sur la courbe par un rond) et l'AN69-PEI succinylé. Le dosage a été fait tant à l'entrée (représenté sur la courbe par un triangle), au centre (représenté sur la courbe par un losange) et à la sortie du module (représenté sur la courbe par un carré).

Les figures 7, 8 et 9 représentent la fixation de l'acide pyruvique $^{14}C$ sur l'AN69 non fonctionnalisée ou fonctionnalisée avec respectivement l'EDC et l'ADH, l'anhydride succinique, l'EDC et l'ADH, et la PEI, l'anhydride succinique, l'EDC et l'ADH.

La figure 10 représente les modalités de préparation d'un module de traitement et la figure 11 les modalités de traitement par circulation extracorporelle.

La figure 12 est une représentation schématique du procédé d'obtention d'un support constitué d'une surface époxy couplée à de la putrescine, apte à former une liaison covalente avec l'acide lipoïque, en présence d'un activateur qui est l'EDC.

La figure 13 représente , à titre de comparaison, la formation du support par liaison ester directement entre l'AL et les groupements OH de l'epoxy.

La figure 14 compare les taux d'IgM anti-AL obtenus, autrement dit les taux d'épuration, sur un support AN 69-AL obtenu par liaison ester (points noirs) ou par liaison amide (losanges noirs).

La figure 15 compare les taux d'épuration obtenus après passages successifs sur des colonnes dont le support est constitué d'AN 69- AL.

La figure 16 représente le profil obtenu en électrophorèse en gel de polyacrylamide 10% des protéines éluées après plusieurs passages sur le même support.

La figure 17 indique le taux d'amines substituées de la PEI-P en fonction de quantités croissantes d'anhydride succinique réagissant avec la PEI.

La figure 18 représente l'influence de la post-succinylation, de la PEI-P sur l'activation de contact.

La figure 19 représente la quantité de PEI-P pré-succinylée par minimodule d'AN69 en fonction du degré de succinylation.

La figure 20 représente la quantité de PEI-P pré-succinylée par minimodule d'AN69 en fonction du degré de succinylation et pour des concentrations variables de PEI-P pré-succinylée.

La figure 21 est une représentation schématique de la présentation des groupes chargés de l'acide succinique expliquant les résultats obtenus selon le taux de présuccinylation de la PEI-P.

La figure 22 représente l'activation de la phase contact mesurée par la génération de kallicréine, en fonction du taux de succinylation.

La figure 23 indique la quantité d'héparine qui se fixe sur les minimodules recouverts de PEI-P pré-succinylée en fonction du taux de pré-succinylation.

Procédé de préparation des supports fonctionnalisés pour la bioépuration spécifique et pour la dialyse et l'hémonofiltration :

L'étude qui suit porte sur la préparation de membranes de type copolymère d'acrylonitrile, traitées par post-acylation ou par pré-acylation. Elle permet de mettre en évidence les avantages de la deuxième méthode pour les applications en bioépuration non spécifiques (membranes de dialyse et/ou hémofiltration) ou spécifique (couplage d'un ou de ligands spécifiques de molécules à épurer).

Les modifications de la PEI ont été réalisées dans le but de :

- optimiser le taux de fonctionnalisation, c'est-à-dire le taux de groupements réactifs aptes à être couplés le cas échéant avec un ligand ;
- limiter la thrombogénicité observée avec les membranes polyanioniques non traitées tout en évitant les fixations non spécifiques, par exemple la fixation de l'héparine.

La PEI, qui a été utilisée dans ces expériences, est le LUPASOL-P, commercialisé par la société BASF, et de masse moléculaire moyenne 750 000 Dalton, mesurée par diffusion de la lumière.
D'autres types de PEI peuvent être utilisés, notamment le lupasol-WF commercialisé par la même société et de masse moléculaire moyenne 25 000.
L'anhydride d'acide carboxylique utilisé est celui de l'acide succinique. L'acylation est dans ce cas une succinylation.

1° <u>Méthode</u> :

1.1 Modification de surface :

Toutes les expériences sont réalisées en utilisant des mini-dialyseurs comprenant 170 fibres creuses AN69 (diamètre interne : 210 μm ; épaisseur de la paroi : 42 μm ; longueur : 0,18 m) et la PEI-P marquée au tritium modifiée ou non en solution dans du sérum physiologique (NaCl 0,14N). Deux approches ont été utilisées :

a) les modules ont été d'abord traités avec la PEI-P, marquée au tritium puis succinylée ;
b) les modules ont été recouverts avec la PEI-P marquée au tritium préalablement succinylée, totalement ou partiellement.

1.2 Préparation de la PEI-P à différents taux de succinylation :

A 3 mg de PEIP dans 50 ml de Borate 0,05 M pH 8,6 est ajouté de l'anhydride succinique (AS) dissous dans l'acétonitrile.
La quantité d'A.S. ajoutée selon le degré de succinylation est la suivante :

100 % de Succinylation : 162 μmol d'AS
80 % de Succinylation : 54 μmol d'AS
60 % de Succinylation 31 μmol d'AS
40 % de Succinylation : 18 μmol d'AS
20 % de Succinylation : 8 μmol d'AS

Après un temps de contact d'une heure sous agitation en ajustant le pH à 8,6 si nécessaire avec de la soude 0.1 M, la réaction est arrêtée par diminution du pH à 5 par ajout d'acide acétique 12 M.
Le taux de substitution des amines est vérifié à l'aide de la fluorescamine selon la méthode décrite dans "Pratique de l'analyse organique calorimétrique et fluorométrique", de J. Bartos et M. Tesez, deuxième édition, 1984, Masson, pages 84 à 85 (figure 17).
Pour revêtir les minimodules, le pH est ajusté à 8.

1.3 Evaluation de la stabilité de la membrane d'AN 69 recouverte par la PEI :

La stabilité des modifications de surface a été évaluée en établissant une circulation d'une solution de NaCl à différentes concentrations (0,14 M, 1, 2 et 4 M).

1.4 Evaluation biologique :

Pour évaluer si les minimodules traités conformément à l'invention activent la phase contact, on les soumet au test suivant : un liquide biologique a été préparé, propre à stimuler la production de kallicréines au contact d'une membrane chargée négativement en surface. Le liquide biologique utilisé pour l'essai était constitué de plasma humain pauvre en plaquettes, dilué à 5 % dans du sérum physiologique additionné de citrate (on note que les conditions du test utilisé sont éloignées des conditions d'utilisation d'un appareil pour circulation extracorporelle de sang : le taux de dilution est très élevé, le liquide choisi est du plasma et non du sang, le plasma est additionné de citrate, donc acidifié, alors

qu'en dialyse, l'anticoagulant utilisé est de l'héparine. Ces conditions de test sont choisies à dessein car elles stimulent et amplifient l'activation de la phase contact). 150 ml de ce liquide ont été mis en circulation en circuit ouvert dans le compartiment sang du minimodule à un débit de 05 ml/min pendant 30 minutes. Les kallicréines plasmatiques ont été dosées dans des échantillons de liquide prélevés à intervalles de temps au moyen d'un test chromogénique classique, à partir du substrat S 2302 de la société BIOGENIC.

On précise que, compte tenu de la sensibilité du test chromogénique utilisé, on considère qu'il n'y a pas d'élévation significative du taux de kallicréines si la concentration en kallicréines reste en deçà d'environ 10 unités par litre.

2° Résultats obtenus :

2.1 Modification de surface :

2.1.1 Post-succinylation :

Les modules sont d'abord recouverts de la PEI-P marquée au tritium puis succinylée. Les résultats sont indiqués dans le tableau I ci-dessous :

TABLEAU I : Influence de la post-succinylation sur le relargage du PEI-P pré-adsorbé sur AN69

| Quantité de PEI-P introduite sur l'AN69 (µg) | Quantité de PEI-P retenue sur l'AN69 (µg) | Quantité de PEI-P restant sur l'AN69 après succinylation (µg) | Quantité de PEI-P relarguée après succinylation (µg) |
|---|---|---|---|
| 320 ± 12 | 57 ± 7 | 32 ± 2 | 25 ± 2 |

Ces résultats montrent que la post-succinylation entraîne 43 % de relargage de la PEI-P.

Par ailleurs, le niveau d'activation de la phase contact mesurée par le biais de la génération de kallicréines, est représentée sur la figure 18. Cette figure compare la kallicréine produite, mesurée en unité par litre, dans le cas de la membrane d'AN69 nue, de la membrane d'AN69 recouverte de PEI-P et de la membrane d'AN69 recouverte de PEI-P puis succinylée.

Cette figure montre que l'AN69 traitée avec de la PEI-P n'active pas la phase contact. En revanche, la post-succinylation désorbant partiellement la PEI-P, l'AN69 traitée avec de la PEI-P post-succinylée active la phase contact.

2.1.2 *Effet de la pré-succinylation* ;

Un minimodule recouvert avec la PEI-P marqué au tritium a eté pré-succinylée à des degrés divers (figure 17) et la quantité de PEI-P marquée au tritium pré-succinylée adsorbée sur AN69 a été déterminée. Les résultats apparaissent sur la figure 19.

La figure 19 indique que la quantité de PEI-P adsorbée sur l'AN69 augmente avec le degré de succinylation, pour atteindre un maximum de 60 %. Au-delà de 60 %, la quantité de PEI-P adsorbée diminue rapidement.

Par ailleurs, la quantité de la PEI-P adsorbée augmente tant avec le degré de succinylation (comme l'indique la figure 19) que avec la concentration de PEI-P pré-succinylée introduite dans le minimodule. Le maximum d'adsorption est observé avec une pré-succinylation de la PEI-P de 60 %, et ce dans tous les cas.

La figure 20 indique que la quantité de PEI-P adsorbée sur le mini module atteint un plateau de 216 µg pour des concentrations de PEI-P pré-succinylée introduite dans le minimodule supérieures ou égales à 60 mg/l.

2.1.3 *Conclusion :*

L'augmentation de l'adsorption de la PEI-P pré-succinylée sur les modules d'AN69 peut résulter d'un changement conformationnel résultant d'une modification des interactions électrostatiques entre les groupes chargés amines et carboxyles.

La figure 21 est une représentation schématique de l'adsorption de PEI non pré-succinylée (schéma du haut) et pré-succinylée à 30 %, 60 % et 100 %, sur une membrane d'AN69.

Lorsque la PEI-P n'est pas pré-succinylée, le nombre de groupements amine par mole de PEI-P interagissant avec les groupements ioniques ( $SO_3^-$ ) de la membrane est importante. La pré-succinylation diminue le nombre de groupements amine par mole de PEI-P induisant un changement de conformation de la PEI-P présuccinylée, ce qui augmente la quantité de PEI-P adsorbée et par conséquent le nombre de sites réactifs disponibles pour la bioépuration

spécifique. En outre, le contrôle du taux de pré-succinylation permet de maîtriser la quantité de sites réactifs susceptibles d'être obtenus sur les membranes d'AN 69 ainsi traitées.

En revanche, dans le cas de la post-succinylation, il est visible sur le premier schéma de la figure 21 que le nombre de sites N+ réactifs est relativement faible, conduisant ainsi à un nombre de sites carboxyl réactifs après succinylation plus faible que dans le cas de la pré-succinylation.

2.2 *Stabilité de la PEI marquée au tritium non succinylée et pré-succinylée après lavage des minimodules avec des concentrations salines croissantes*.

Dans le cas de la post-succinylation, le couplage de l'anhydre succinique sur la PEI passe par une étape de déprotonation conduisant à un relargage de 30 à 40 % de la PEI du fait de l'affaiblissement de la liaison entre les groupes NH3+ et les groupes SO3- de la membrane (comme cela été montré plus haut dans le tableau I).

Le tableau II ci-dessous montre le pourcentage de désorption de PEI-P après lavage avec accroissement de la concentration saline.

TABLEAU II :

| CONCENTRATION SALINE<br>DEGRE DE SUCCINYLATION | 0.14M | 1M | 2M | 4M |
|---|---|---|---|---|
| 0% | 0 | 0 | 13 | 13 |
| 20% | 0 | 0 | 16 | 16 |
| 60% | 0 | 0 | 42 | 54 |
| 90% | 0 | 0 | 49 | 71 |

Le résultat montre que quand les minimodules sont traités avec 40, 60 et 110 mg/l de PEI-P pré-succinylée, puis lavés avec 20 ml de sérum physiologique. (0,14 M NaCl), il n'y a pas de désorption de la PEI-P, et ce jusqu'à une concentration saline de 1 M). En revanche, quand les minimodules sont lavés avec une solution saline ayant une molarité supérieure ou égale à 2M, une désorption partielle de la PEI-P pré-succinylée est observée dans tous les cas.

Le niveau de désorption de la PEI-P pré-succinylée augmente avec le degré de pré-succinylation.

Un traitement des minimodules par de la PEI-P pré-succinylée à 60 % est optimal pour obtenir le maximum de sites fonctionnels.

2.3 *Hémocompatibilité*

La figure 22 indique que l'AN69 traitée avec de la PEI-P pré-succinylée entre 20 et 70 % ne provoque pas l'activation de la phase contact. En revanche, quand le traitement de l'AN69 est réalisé avec une PEI-P présuccinylée à 100 % (figure 22), l'activation de la phase contact observée est équivalente à celle de l'AN69 non traité par la PEI-P.

Un avantage supplémentaire du traitement de surface de l'AN69 avec la PEI-P pré-succinylée est la diminution de l'adsorption de l'héparine sur le support par rapport à l'adsorption observée avec l'AN69 traité avec la PEI-P non succinylée (figure 23). Ceci représente un avantage considérable lorsque ces membranes sont utilisées en hémodialyse/hémofiltration car l'héparine est souvent utilisée comme anticoagulant.

3° *Conclusions*

Ces expériences comparatives ont permis de définir les conditions optimales de préparation des membranes d'AN69 fonctionnalisées en particulier lorsqu'elles sont traitées avec de la PEI-P pré-acylée. Si l'anhydre d'acide carboxylique est celui de l'acide succinique, ces conditions sont les suivantes :

- un degré de succinylation de 60 % ;
- un traitement en surface à une concentration de 60 mg/l de PEI-présuccinylée.

Ceci conduit à :

- une fonctionnalité maximum de 2 μM de COOH/m$^2$ de membrane:

- une très bonne stabilité dans un milieu à haute force ionique (jusqu'à 1 molaire) ;
- pas d'activation de la phase contact du sang ;
- une adsorption limitée d'héparine sur le support ce qui présente un avantage considérable lorsque ce type de membranes est utilisé en dialyse/hémofiltration.

De façon générale, cette technique permet de maîtriser de façon reproductible le nombre de sites réactifs présents sur la membrane traitée, à savoir :

- une augmentation du nombre de sites réactifs pour l'application à la bioépuration spécifique ;
- la reproductibilité du nombre de sites réactifs obtenus dans l'ensemble des applications des membranes de l'invention.

Les expériences ci-dessus mettent en outre en évidence l'avantage de la pré-succinylation par rapport à la post-succinylation ; en effet, dans cette dernière un relargage de la PEI préfixée entraîne la formation probable de « trous » dans le revêtement de la membrane AN69. L'existence de tels « trous » conduit à des effets indésirables notamment des effets d'activation de la phase contact, observés sur la membrane nue, non recouverte.

<u>Exemple 1</u> : <u>Immobilisation des Ac anti IL6 sur AN69 PEI-PEG epoxyde</u> :

Nous avons entrepris des expériences pour déterminer la meilleure méthode pour immobiliser les Ac anti IL6 sur les supports insolubles, par exemple le sépharose, et une fois la méthode établie de l'appliquer à l'AN69.

Deux techniques de couplage des AC ont été mises en oeuvre en utilisant les IgG marqués au tritium [$^3$H].

a) un couplage par liaison amide sur des $\varepsilon NH_2$ de la lysine dans la chaîne protéique des IgG ;

b) un couplage par liaison hydrazone sur des groupements glucidiques préalablement oxydés et qui se trouvent sur la partie Fc de l'IgG selon la technique décrite dans les documents suivants : brevet US n° 4217338 ; Quash et al. J. Immunol. Methods, 1978, 22, 165-174.

Les billes de sépharose et les Ac anti IL6 sont mis en contact pendant 2 heures à raison d'un volume de billes pour un volume d'AcIL6 à des concentrations croissantes d'AcIL6 déposées.

La quantité de radioactivité obtenue sur chaque échantillon de billes est alors mesurée sur un compteur à scintillation liquide PACKARD MINAXI β.

Les résultats sont indiqués dans les tableaux I et II ci-dessous :

TABLEAU I : COUPLAGE COVALENT DE l'Ac anti IL6 SUR BILLES de SEPHAROSE

| I - a : PAR LIAISON AMIDE | | | | |
|---|---|---|---|---|
| | Supports de billes utilisée ($cm^2$) : 40 | | | |
| Quantité d'Ac anti IL6 non oxydé introduite (μg) | 2,6 | 10 | 20 | 52 |
| Quantité d'Ac anti IL6 non oxydé couplé (μg) | 0,54 | 2,64 | 5,20 | 13,60 |
| % Ac anti IL6 non oxydé couplé | 21 | 26 | 26 | 26 |
| | | | | |
| I - b : PAR LIAISON HYDRAZONE | | | | |
| | Supports de billes utilisée ($cm^2$) : 40 | | | |
| Quantité d'Ac anti IL6 oxydé introduite (μg) | 2 | 8,4 | 16,8 | 42 |
| Quantité d'Ac anti IL6 oxydé couplé (μg) | 0.38 | 2,72 | 6,26 | 18 |
| % Ac anti IL6 oxydé couplé | 20 | 32 | 37 | 42 |

Les résultats du tableau I montrent que :

1) pour des faibles concentrations d'Ac anti IL6 mises en jeu, la quantité d'Ac couplé représente 20 % de la quantité d'Ac déposé et ce pour les deux techniques (tableau I).

2) le fait d'augmenter la concentration d'Ac anti IL6 déposé entraîne une augmentation de la quantité d'Ac anti IL6

couplé par les deux techniques puisqu'on passe de 0,54 µg à 13,6 µg d'Ac anti IL6 couplé pour 40 $cm^2$ de billes lorsque l'Ac anti IL6 est couplé par liaison amide. Cette quantité est de 0,38 µg à 18 µg d'Ac anti IL6 couplé pour 40 $cm^2$ de billes lorsque l'Ac anti IL6 est couplé par liaison hydrazone.

3) lorsque les Ac anti IL6 sont couplés par liaison hydrazone, le rendement de couplage augmente en fonction de la quantité d'Ac anti IL6 déposée, passant de 20 % à 42 % alors que pour les Ac anti IL6 couplés par liaison amide, le rendement d'Ac anti IL6 couplé reste constant (26 %) en fonction de la quantité d'Ac déposé.

Des expériences entreprises pour déterminer le degré d'adsorption des anticorps anti IL-6, oxydés ou non oxydés, sur des billes de Sépharose ont montré que cette adsorption ne dépassait pas 7 %.

Il apparaît donc que si la quantité d'Ac anti IL6 couplé augmente pour les deux techniques en fonction de la concentration d'Ac anti IL6 déposé, l'efficacité de couplage de ces Ac anti IL6 est supérieure lorsque ces Ac anti IL6 sont couplés par liaison hydrazone.

Ceci est plus marqué à des fortes concentrations d'Ac ajouté:

- 42 % pour liaison hydrazone,
- 26 % pour liaison amide.

<u>Exemple 2</u> : <u>Immunoépuration de l'antigène IL6 sur AN69 PEI-PEG epoxyde</u> :

2.1 <u>Sur billes de sépharose avec des Ac anti IL-6 couplés par liaison amide ou liaison hydrazone</u> :

Des essais d'immunoépuration de l'IL6 ont été effectués en utilisant :

- d'une part des billes sur lesquelles l'Ac anti IL6 a été couplé à des quantités croissantes soit par liaison amide soit par liaison hydrazone selon les techniques et les mêmes rapports énoncés dans le paragraphe I, 1) et le tableau I,
- d'autre part en faisant varier le taux d'IL6 présent dans les sérums à tester. Pour cela, nous avons dilué un sérum contenant un taux très élevé d'IL6 (1,7 µg d'IL6 pour 1 ml de sérum) dans un mélange de sérums négatifs, testés préalablement. Finalement les sérums testés ont un taux d'IL6 final de 33 050, 22 470, 11 235 et 3 157 pg/ml.

Ces billes de sépharose/Ac anti IL6 sont alors mises en contact avec les différents mélanges de sérums modifiés à raison d'un volume de billes pour un volume de sérums.

Le taux d'IL6 restant dans les sérums avant et après passage est mesuré par une technique immunoenzymatique de type sandwich.

Les résultats figurent dans le tableau II ci-dessous.

TABLEAU II a : % d'IL6 immunoépuré après passage sur billes :

| PAR LIAISON AMIDE | | |
|---|---|---|
| Quantité d'Ac anti IL6 non oxydé couplé (µg/40 $cm^2$) | Quantité d'IL6 introduite (pg/ml) | % d'IL6 retenu |
| 0,54 | 3157<br>11235<br>22470<br>35050 | 86,9<br>75,7<br>67,6<br>18,5 |
| 2,64 | 3157<br>11235<br>22470<br>35050 | 97,9<br>89,6<br>93,9<br>81,3 |
| 13,6 | 3157<br>11235<br>22470<br>35050 | 98,1<br>98,9<br>100<br>97,8 |

TABLEAU II b : % d'IL6 immunoépuré après passage sur billes :

| PAR LIAISON HYDRAZONE | | |
|---|---|---|
| Quantité d'Ac anti IL6 oxydé couplé (μg/40 $cm^2$) | Quantité d'IL6 introduite (pg/ml) | % d'IL6 retenu |
| 0,38 | 3157<br>11235<br>22470<br>35050 | 57<br>-<br>41,7<br>28,5 |
| 2,72 | 3157<br>11235<br>22470<br>35050 | 89,1<br>72,5<br>70,9<br>54,2 |
| 18,00 | 3157<br>11235<br>22470<br>35050 | 99,3<br>97,4<br>98,7<br>90,0 |

Ces résultats montrent que :

1) le support seul (billes sans Ac anti IL6) traité ou non avec l'hydrazine ne fixe pas l'IL6.
2) plus la quantité d'Ac anti IL6 couplée est importante plus l'immunoépuration de l'IL6 est efficace et ceci pour les deux techniques de couplage utilisées.
3) lorsque les Ac anti IL6 sont couplés à raison de 13,6 μg/40 $cm^2$ (liaison amide) et 18 μg/40 cm2 (liaison hydrazone), l'immunoépuration de l'IL6 est efficace à 90-100 % pour les deux techniques de couplage utilisées et pour des sérums ayant un faible ou un fort taux d'IL6.
4) pour des quantités d'Ac anti IL6 couplés inférieures à 13.6 μg/40 $cm^2$ (liaison amide) et 18 μg/40$cm^2$ (liaison hydrazone) l'immunoépuration de l'IL6 diminue en fonction de la quantité d'Ac anti IL6 couplé et en fonction du taux d'IL6 présent dans le sérum.

En effet, lorsque l'AC ANTI IL6 est couplé par liaison amide à 2,64 μg/40 $cm^2$, l'efficacité d'immunoépuration diminue de 98 % pour un sérum avec 3.157 pg/ml d'IL6 à 81,3 % pour un sérum avec 35 030 pg/ml d'IL6. De même, lorsque l'Ac anti IL6 est couplé à O,54 μg/40 $cm^2$, l'efficacité d'immunoépuration diminue de 86,9 % pour un sérum avec 3 157 pg/ml d'IL6 à 18,5 % pour un sérum avec 35 030 pg/ml d'IL6. (tableau II).

Lorsque l'Ac anti IL6 est couplé par liaison hydrazone à 2,72 μg/40 $cm^2$, l'efficacité d'immunoépuration diminue de 9° % pour un sérum avec 3 157 pg/ml d'IL6 à 54,2 % pour un sérum avec 35 030 pg/ml d'IL6. De même lorsque l'Ac anti IL6 est couplé à 0,38 μg/40 $cm^2$, l'efficacité d'immunoépuration diminue de 57,1 % pour un sérum avec 3 157 pg/ml d'IL6 à 28,5 % pour un sérum avec 35 030 pg/ml d'IL6 (tableau III).

Conclusion :

Le couplage des Ac anti IL6 par liaison amide ou par liaison hydrazone préserve leur activité vis-à-vis de l'IL6.

Pour que l'immunoépuration de l'IL6 présent dans un sérum contenant jusqu'à 35 000 pg/ml soit efficace à 90-100 %, la quantité d'Ac anti IL6 couplé doit être de l'ordre de 0,4 μg/$cm^2$. Cette efficacité d'immunoépuration est la même pour des Ac anti IL6 couplés soit par liaison amide soit par liaison hydrazone.

Pour des quantités d'Ac anti IL6 couplé inférieures à 0,4 μg/$cm^2$, l'immunoépuration de l'IL6 est d'autant moins efficace que le taux d'IL6 présents dans un sérum est fort. Cette constatation est vraie pour les deux types de liaison, néanmoins lorsque les Ac anti IL6 sont couplés par liaison amide l'immunoépuration de l'IL6 est d'une manière générale plus efficace que lorsque les Ac anti IL6 sont couplés par liaison hydrazone.

La quantité optimale d'Ac anti IL6 couplé par $cm^2$ pour une immunoépuration de l'IL6 totale ayant été définie sur billes, nous pouvions passer à l'immunoépuration de l'IL6 sur minimodules AN69.

2.2. Essais d'immunoépuration de l'IL6 sur minimodules AN69 :

Les minimodules ont été hydrophilisés et fonctionnalisés (H&F) selon la méthode décrite dans WO 92/07023.

1) Méthodes :

Des minimodules comprenant 170 fibres creuses en AN69 de 18 cm de long ont été assemblés (surface interne : 256 $cm^2$). Ces minimodules ont subi un traitement par le PEG tétrapoxyde afin de générer des groupements époxides.

Nous avons couplé par liaison hydrazone des Ac anti IL6 marqués au propionate $^3H$, sur un des deux minimodules AN69 dans les mêmes conditions que pour les billes à raison de 0,4 $\mu g/cm^2$ sachant que ces 0,4 $\mu g/cm^2$ d'Ac couplés représentent théoriquement 42 % des Ac déposés si on considère que l'efficacité de couplage sur les fibres AN69 est similaire à l'efficacité de couplage sur les billes de sépharose.

Nous avons donc mis en contact 0.95 $\mu g$ d'Ac pour 1$cm^2$ de fibre soit 243 $\mu g$ d'Ac pour 256 $cm^2$ fibres.

D'autre part, nous avons couplé des Ac de chèvres témoins toujours par liaison hydrazone sur le deuxième minimodule A69 dans les mêmes conditions que pour le minimodule AN69 + Ac anti IL6.

Trois ml de sérum à 11 203 pg/ml d'IL6 ont été recyclés pendant deux heures sur ces minimodules (O.5 ml/mn).

Puis le taux d'IL6 présent est alors mesuré par une technique immunoenzymatique de type sandwich avant et après passage sur ces minimodules.

D'après le tableau III, il apparaît que l'immunoépuration a été efficace et spécifique puisque après passage sur l'AN69 greffé avec les Ac témoins il n'y a que 14 % d'IL6 enlevés non spécifiquement tandis que après passage sur AN69 avec les Ac anti IL6 le taux d'immunoépuration augmente jusqu'à 77,5 %.

TABLEAU III : IMMUNOEPURATION D'IL6 APRES PASSAGE SUR AN69 :

| | IL6 avant passage sur AN69 | IL6 après passage sur AN69 + AC témoins oxydés | IL6 après passage sur AN69 + Ac anti IL6 oxydés |
|---|---|---|---|
| pg/ml | 11 203 | 9 623 | 2 512 |
| IL6 immunoépuré % | 0 | 14,0 | 77,5 |

Dans les conditions expérimentales utilisées, le taux de fixation d'anticorps anti-IL6 sur le module d'AN69 est de 0,11 $\mu g/cm^2$.

L'hypothèse la plus vraisemblable qui peut expliquer ce rendement moyen de couplage des Ac anti IL6 sur les fibres AN69 est la rareté des sites époxides à la surface des fibres et donc la rareté après traitement à l'adipic dihydrazide de groupements hydrazines disponibles pour générer une liaison hydrazone avec les aldéhydes de la partie glucidique des Ac oxydés.

Nous savons que l'immunoépuration de l'IL6 est efficace à 100 % pour des sérums où leurs taux d'IL6 est ≤ à 35 000 pg/ml, à condition que la quantité d'Ac anti IL6 couplé soit d'au moins 0,4 $\mu g/cm^2$. Or, nous avons à notre disposition un système (fibre AN69) qui vraisemblablement ne permet pas d'obtenir une telle densité d'Ac couplé de façon covalente au $cm^2$ et donc limite l'immunoépuration de l'IL6.

2.3 Contrôle de la capacité à fixer le complément :

L'efficacité d'épuration d'IL6 sérique par le minimodule AN69 hydrophilisé et fonctionnalisé H& F greffé avec des Ac anti IL6 ayant été prouvée, nous avons déterminé si ce même minimodule chimiquement modifié préservait sa propriété initiale concernant sa non réactivité envers le système du complément. Nous avons donc vérifié sa capacité à fixer ou non le complément.

Les membranes AN69 H&F n'ont pas fixé le complément ajouté à raison de 0.625 UH50 ou à 2,5 UH.

Les cartouches AN69 H&F greffées avec l'Ac permettent d'éliminer spécifiquement l'Ag correspondant et n'engendrent pas la fixation du complément.

Exemple 3 : Préparation de l'AN69 PEI post-succinylée pour la bioépuration spécifique :

Cette préparation a été faite par traitement des minimodules avec de la PEI WF, suivi par une post-succinylation.

3.1 Détermination du nombre de sites disponibles sur AN 69-PEI pour la fixation de molécules de faible masse moléculaire (FPM) :

1) Après fonctionnalisation de l'AN 69-PEI par l'anhydride succinique :

Pour cette étude, nous avons utilisé un module constitué d'une membrane plane représentant une surface de

1 $m^2$. Ce module a été traité avec de la PEI puis démonté afin de pouvoir prélever aux extrémités et au centre du module des échantillons de membrane de 4,5 $cm^2$ à l'aide d'un emporte pièce.

Nous avons comparé le nombre de sites disponibles sur ces pastilles AN 69-PEI par rapport à des pastilles AN 69 témoins en utilisant une molécule de FPM radiomarquée : la [$^{14}C$] éthanolamine.

L'anhydride succinique est ensuite fixé par le protocole suivant :

- Préparation d'une solution d'anhydride succinique à 1 M en acétonitrile ;
- Dilution de celle-ci au 1/10 dans tampon borate 50 mM pH 8,5 et laisser en contact avec les pastilles AN 69 témoin et AN 69 PEI pendant 15 minutes ;
- Recommencer l'opération une deuxième fois ;
- Lavages intenses en $H_2O$.

Sur les parties ainsi obtenues, l'éthanolamine marquée est fixée de la façon suivante :

a) activation des groupements COOH générés :

a - 1) : préparer une solution d'1 ethyl 3(3 diméthylaminopropyl carbodiimide) (EDC) (50nM) et de N. hydroxysuccinimide (NHS) (50nM) dans $NaH_2PO_4$ 10nM et laisser en contact 15 minutes avec les pastilles.
a - 2) : éliminer la solution d'activation.

b) fixation d'éthanolamine :

b - 1) : préparer des dilutions isotopiques d'éthanolamine froid/éthanolamine marquée

- 4nmol froid / O,4 nmol marqué
- 40 nmol froid / O,4 nmol marqué
- 400 nmol froid / 0,4 nmol marqué
- 4000 nmol froid / 0,4 nmol marqué.

Chaque solution étant préparée dans 50 mM Borate pH 8 final.

b - 2) : laisser en contact avec les pastilles AN69 témoin et AN69-PEI pendant deux heures sous agitation, puis laver en borate/NaCl 0,5 M et compter.

La figure 6 montre que :

- la quantité optimale d'éthanolamine retenue sur AN69 + PEI + anhydride succinique est de 5,6 nmol/pastille ;
- la quantité d'éthanolamine retenue sur AN 69 + anhydride succinique est de 1.2nmol/pastille.

Donc la quantité d'éthanolamine retenue réellement sur AN69 + PEI succinylé est de 5,6 - 1,2 = 4,4 nmol / 4,5 $cm^2$ , soit : 1 nmol / $cm^2$ ou 10 $\mu mol/m^2$.

Enfin la figure 6 montre que la quantité d'éthanolamine retenue sur AN 69 + PEI succinylé est homogène sur la totalité du module puisque nous trouvons le même nombre de sites à l'entrée, au centre ou à la sortie du module.

2) après fonctionnalisation de l'AN 69-PEI succinylé par l'adipique dihydrazide (ADH)

L'ADH est fixé de la façon suivante :

Après activation des groupements COOH (voir a)), préparer une solution d'ADH à 50 mM en borate 50 mM pH 8,5 et laisser en contact avec les pastilles d'AN69 pendant 1 heure.

Laver abondamment en borate puis en $H_2O$ puis en acétate.

La fixation d'acide pyruvique $^{14}C$ sur les pastilles est effectuée de la manière suivante :

Préparer des dilutions isotopiques d'acide pyruvique froid / acide pyruvique marqué dans 1 ml d'acétate 50 nM pH 5,4 :

- 0 nmol froid / 5 nmol marqué
- 20 nmol froid / 5 nmol marqué
- 200 nmol froid / 5 nmol marqué

- 2000 nmol froid / 5 nmol marqué ;

Laisser en contact avec les pastilles pendant 2 heures puis laver en acétate, NaCl 0,5 M et compter.

La figure 7 montre que la quantité d'acide pyruvique optimale retenue sur AN 69 + ADH est de 0,1 nmol / pastille avec ou sans activation (EDC) du support.

La figure 8 montre que la quantité d'acide pyruvique optimale retenue sur AN 69 succinylé + ADH est de 0,5 nmol / pastille avec ou sans activation (EDC) du support pour des quantités d'acide pyruvique introduites supérieures à 150 nMole par pastille.

La figure 9 montre que :

- la quantité d'acide pyruvique couplée sur AN 69 + PEI + anhydride succinique + ADH est d'au moins 1,1 nmole par pastille après activation des carboxyles générés sur le support (via l'anhydride succinique) pour la fixation par liaison amide de l'ADH ;
- lorsqu'on n'active pas les groupements carboxyles générés sur le support, la quantité d'acide pyruvique adsorbée sur AN 69 + PEI + succinylation + ADH est de 0,45 nmole par pastille.

En conclusion, la fonctionnalisation de l'AN 69 par le PEI tout d'abord et ensuite par l'anhydride succinique augmente le nombre de sites disponibles pour les molécules de faible masse moléculaire d'au moins 5 fois. Cette augmentation est du même ordre de grandeur après traitement par l'ADH.

3.2 <u>Détermination du nombre de site disponible sur AN 69-PEI pour la fixation de molécules de haut masse moléculaire (HPM)</u> :

Pour cette étude, nous avons mis en contact deux concentrations d'Immunoglobuline (Ig) oxydées ou non oxydées (5 μg et 60 μg) avec des pastilles d'AN 69 fonctionnalisées ou non fonctionnalisées.

| μg d'Ig | μg d'Ig non oxydé retenu | | μg d'Ig oxydé retenu | |
|---|---|---|---|---|
| introduit | sur AN 69 | sur AN 69 + PEI + ADH | sur AN 69 | sur AN 69 + PEI + ADH |
| 5 | 0,122 | 0.290 | 0,452 | 0,870 |
| 60 | 0,950 | 2,835 | 3,990 | 7,760 |

La fonctionnalisation de l'AN 69 tout d'abord par le PEI et ensuite par l'anhydride succinique et finalement par l'ADH permet d'augmenter d'au moins deux fois le nombre de sites disponibles pour les molécules de haut masse moléculaire.

<u>Exemple 4</u> : <u>Immunoépuration des Anticorps anti acide lipoïque (AL) sur AN69 PEI post-succinylé</u> :

En ce qui concerne le LED, les résultats présentés sur les figures 1, 2 et 3 nous ont incités à mettre au point des cartouches capables d'éliminer spécifiquement les Ac anti AL dans le plasma des malades atteints de LED. L'AL étant un haptène de PM de 100 kDa, la technique développée pourrait être étendue à d'autres haptènes contenant des groupements COOH fonctionnels.

La fixation de l'AL de façon covalente sur un support insoluble se fait de la manière suivante :

- le groupement fonctionnel terminal époxy a été substitué avec de la putrescine directement pour former un bras se terminant par une αNH2 sur laquelle a été greffé par liaison amide l'acide lipoïque (voir figure 1).

Cette mise au point était nécessaire pour deux raisons : d'une part, elle devrait permettre la réutilisation de la cartouche après élution des Ac en milieu alcalin et, d'autre part, la liaison amide est intrinsèquement beaucoup plus stable que la liaison ester (voir figure 2) qui aurait été formée si l'AL avait été couplé directement avec des groupements OH de l'époxide hydrolysée (voir figure 5).

4.1 <u>Etude comparative d'immunoépuration en fonction du type de liaison de l'AL</u> :

La figure 5 montre que l'immunoépuration des IgM anti-AL est nettement plus efficace lorsque l'AL est couplé par une liaison amide puisque la totalité de ces anti-AL est éliminée alors que l'AL couplé par une liaison ester ne

provoque qu'une diminution très faible des IgM anti-AL de l'ordre de 8 %.

L'efficacité d'immunoépuration des Ac anti-AL par AL couplé par liaison amide étant prouvé, l'étape suivante consistait à tester la réutilisation et donc la stabilité du support.

Pour cela, nous avons, sur le même support, immunoépuré six échantillons provenant du même sérum lupique.

4.2 Essais de réutilisation du minimodule AN69 H&F -AL :

La figure 8 montre que six passages successifs, avec une étape d'élution entre chaque passage, sur le même support de six échantillons différents mais provenant du même sérum lupique donne une efficacité d'épuration des Ac anti-AL identique après le premier passage comme après le sixième passage.

L'élution successive des protéines retenues après ces six passages donne le même profil sur gel de polyacrylamide (figure 9).

Conclusion :

Nous avons donc mis au point sur l'AN 69 un système d'immunoépuration des Ac anti-AL efficace, spécifique et réutilisable en circulation extracorporelle.

Exemple 5 : Couplage par covalence d'IgG purifiées sur AN69 PEI-P pré-succinylée :

5.1 Matériels :

Les expériences sont réalisées sur des minidialyseurs comportant 170 fibres creuses en AN69 (diamètre interne : 210 μm ; épaisseur de la paroi : 42 μm ; longueur : 0.18 m).

Les IgG de chèvre proviennent de chez Biosys (Compiègne France).

Des IgG de souris et IgG de lapin ont été purifiées au laboratoire respectivement sur colonne de protéine A sépharose à partir d'un pool de liquide d'ascite et sur DEAE cellulose à partir d'un sérum de lapin.

5.2 Méthode :

5.2.1 Préparation des IgG radiomarquées :

Les IgG de souris, de lapin et de chèvre ont été radiomarqués N-succinidyl(2 - 3 $^3$H) propionate et l'excès de radioactivité éliminé par dialyse.

- activité spécifique des IgG après dialyse :

  IgG desouris :1,25 $10^6$ cpm/mg protéine
  IgG de lapin : 1,86 $10^6$ cpm/mg protéine
  IgG de chèvre : 0,77 $10^6$ cpm/mg protéine

5.2.2 Modification des minimodules

5.2.2.1) 6 minimodules ont été déglycérinés puis traités avec une solution de PEI-P présuccinylée à 60 % ;
5.2.2.2) 3 minimodules, notés A, B, C sont ensuite activés par un carbodiimide tandis que les 3 autres appelés D, E, F sont laissés tels quels ;
5.2.2.3) 50 μg d'IgG de lapin radiomarqués sont recyclés pendant 2 heures en PBS (volume final : 3ml) sur les minimodules A et D.
50 μg d'IgG de souris radiomarqués sont recyclés pendant 2 heures en PBS (volume final: 3 ml) sur les minimodules B et E.
50 μg d'IgG de chèvre radiomarqués sont recyclés pendant 2 heures en PBS (volume final : 3 ml) sur les minimodules C et F.
5.2.2.4) Les 6 minimodules sont ensuite lavés jusqu'à ce qu'il n'y ait plus de traces de radioactivité.

La quantité d'IgG couplée correspond à la quantité de radioactivité introduit - la quantité de radioactivité restituée.

5.3 Résultats : Quantité d'IgG couplée sur AN69 présuccinylé

| Support | Quantité d'IgG introduite (μg) | Quantité d'IgG retenue (μg) |
|---|---|---|
| A | 50 | 3,74 |
| B | 50 | 7,82 |
| C | 50 | 3,80 |
| D | 50 | 7,05 |
| E | 50 | 3,60 |
| F | 50 | 6,30 |

REFERENCES BIBLIOGRAPHIQUES

1- Bradford, A.P., Aitken, A., Beg, F., Cook, K.G. et Yeaman, S.J. (1987a).Amino acid sequence surrounding the lipoic acid cofactor of bovine kidney 2-oxoglutarate dehydrogenase complex. FEBS Lett. 22, 1, 211

2- Bradford, A.P., Howell, S., Aitken, A., James, L.A. et Yeaman, S.J. (1987b).Primary structure around the lipoate attachment site on the E2 component of bovine heart PDH complex. Biochem. J. 245 (3), 919

3- Click, R.E.. Benck, L. et Alter, B.J. (1972a).Enhancement of antibody response by mercaptoethanol. Cell. Immunol. 3, 156

4- Click, R.E., Benck, L. et Alter, B.J. (1972b).Immune response in vitro. I. Culture conditions for antibody synthesis. Cell. Immunol. 3, 264

5- Flannery, G.R., Burroughs, A.K., Butler, P., Chelliah, J., Hamilton-Miller, J., Brumfitt, W. et Baum, H. (1989).Antimitochondrial antibodies in primary biliary cirrhosis recognize both specific peptides and shared epitopes of the M2 family of antigens. Hepatology 10, 370

6- Fussey, S.P.M., Ali, S.T., Guest. J.R., James, O.F.W., Bassendine, M.F. et Yeaman, S.J. (1990).Reactivity of primary biliary cirrhosis sera with Escherichia coli dihydrolipoamide acetyltransferase (E2p) : Characterization the main immunogenic region. Proc. Natl. Acad. Sci. 87, 3987

7- Fussey, S.P.M., Guest, J.R., James, O.F.W., Bassendine, M.F. et Yeaman, S.J. (1988).Identification and analysis of the major M2 autoantigens in primary biliary cirrhosis. Proc. Natl. Acad. Sci. USA 85, 8654-8658

8- Hallgren, H.M., Buclkey, C.E., Gillusten, V.A. et Yunis, E.A. (1973).Lymphocyte phytohemagglutinin responsiveness. Immunoglobulins and autoantibodies in aging man. J. Immunol. 111, 1101

9- Isenberg, D.A. et Collin, C. (1985).Detection of cross-reactive anti-DNA antibody idiotype on renal tissue-bound immunogobulins from lupus patients. J. Clin. Invest. 76, 287

10- Kalunian, K.C., Sahakian, N.P. et Ebling, F.M. (1989).Idiotypic characteristics of immunoglobulins associated with systemic lupus erythematosus. Arth. Rheum. 32, 513

11- Klinman, D.M., Shirai, A., Ishigatsubo, Y., Conover, J. et Strinberg, A.D. (1991).Quantitation of IgG and IgM secreting B cells in the peripheral blood of patients with systemic lupus erythematosus. Arthritis Rheum. 134, 1404

12- Lafer. E.M., Rauch, J., Andrzejewski, C.J.R., Mudd, D., Furie, B., Schwartz, R.S. et Stollar, B.D. (1981).Polyspecific monoclonal lupus autoantibodies reactive with bath polynucleotides and phospholipids. J. Exp. Med. 153, 897

13- Legastelois, S., Thomas, V., Quash, G., Métais, M.P., Tebib, J., Moreira, A. et Monier, J.C. (1994).Naturally occurring antibodies reacting with lipoic acid : screening method, characterization and biochemical interest. J. Immunol. Meth. 171, 111

14- Naor, D., Bonavida, B., Robinson, R.A., Shibata. I.N., Percy, D.E., Chia, D. et Barnett, E.V. (1976).Immune response of New Zealand mice with trinitrophenylated syngeneic mouse red cells. Eur. J. Immunol. 6, 783

15- Ohmori, H. et Yamamoto. I. (1981).Activation of murine lympocytes by 2-mercaptoethanol and related thiol compounds and its mechanism. I.. Relationship between mitogenic activities and augmenting effects on antibody synthesis in vitro. Immunopharmacol. 3, 333

16- Ohmori, H. et Yamamoto, I. (1982).Mechanism of augmentation of the antibody response in vitro by 2-mercaptoethanol-induced stimulation of the uptake of cystine, an essential animo acid. J. Exp. Med. 155, 1277

17- Opitz, H.G., Opitz, U., Lemcke, H., Hewlett, G., Schreml, W. et Flad, H.D. (1977). The role of fetal calf serum in the primary immune response in vitro. J. Exp. Med. 145, 1029

18- Romball, C.G. et Weigle, W.O. (1987). The effect of aging on the induction of experimental autoimmune thyroidite. J. Immunol. 193, 1490

19- Sasaki, T., Muryoi, T., Hatakeyama, A., Suzuki, M., Sato, H., Seino, J., Saito, T. et Yoshinaga, K. (1991).Circulating anti-DNA immune complexes in active lupus nephritis. Am. J. Med. 91, 355

20- Shoenfeld, Y., Isenberg, D.A., Rauch, J., Madaio, M., Stollar, B.D. et Schwartz, R.S. (1983).Idiotypic cross-reactions of monoclonal human lupus autoantibodies. J. Exp. Med. 158, 718

21- Stephens, P.E., Darlison, M.G., Lewis, H.M. et R., G.J. (1983). The pyruvate dehydrogenase complex of E.coli K12. Nucleotide sequence encoding the dihydrolipoamide acetyltransferase component. Eur. J. Biochem. 133(3), 481

22- Suzuki, K., Hara, M., Harigai, M., Ishizuka, T., Hirose, T., Matsuki, Y., Kawaguchi, Y., Kitani, A., Kawagoe, M. et Nakamura, H. (1991).Continuous removal of anti-DNA antibody, using a new extracorporeal immunoadsorption system, in patients with systemic lupus erythematosus. Arth. Rheum. 34, 1546

23- Terman, D.S., Buffaloe, G., Mattioli, C., Cook, G., Tillquist, R., Sullivan, M. et Ayus, J.C. (1979).Extracorporeal immunoadsorption : initial experience in human systemic lupus erythematosus. Lancet II, 824

## Revendications

1. Support fonctionnalisé pour l'épuration extracorporelle spécifique d'un fluide biologique **caractérisé en ce que :**

   • sa surface comprend des groupements fonctionnalisés et satisfaisant aux conditions suivantes:

   ⇒ être stables au moins à une température comprise entre 15°C et 45 °C;
   ⇒ être stables à au moins un type de procédé de stérilisation applicable aux dispositifs médicaux; et en particulier aux rayonnements gamma;

   • et sa surface présente des charges, par l'intermédiaire desquelles sont liées par liaisons ioniques des molécules ou macromolécules d'un premier type exhibant, une fois liées, des groupements amines libres sur lesquels sont fixées, par liaison covalente, des molécules d'un deuxième type qui, une fois liées, exhibent des groupements carboxyliques ayant permis le couplage direct ou indirect, de manière covalente, de ligands spécifiques.

2. Support fonctionnalisé selon la revendication 1, **caractérisé en ce que** les groupements carboxyliques ont formé directement ou indirectement des liaisons covalentes avec des ligands porteurs de groupements:
   $-CHO$, $-NH_2$, $-COOH$, $-SH$, $-OH$,

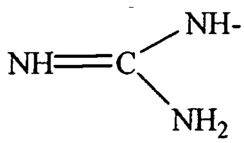

3. Support fonctionnalisé selon la revendication 1, **caractérisé en ce que** sont fixées par liaison covalente sur les groupements carboxyliques, des molécules de troisième type ayant une fonction d'espaceur pour diminuer l'encombrement stérique et favoriser la fixation ultérieure d'un ligand et/ou de la molécule ou éléments figurés à épurer.

4. Support fonctionnalisé selon la revendication 1, **caractérisé en ce que** sont fixées, par liaison covalente, sur les groupements carboxyliques, des molécules d'un quatrième type ayant pour fonction d'augmenter l'hydrophilie du support en vue de limiter les interactions non spécifiques.

5. Support fonctionnalisé selon la revendication 1, **caractérisé en ce que** sont fixées, par liaison covalente, sur les groupements carboxyliques, des molécules d'un cinquième type exhibant, une fois liées, des groupements d'une autre nature, permettant un autre type de couplage covalent avec un ligand porteur notamment de groupements -CHO ou -COOH.

6. Support fonctionnalisé pour l'épuration extracorporelle d'un fluide biologique **caractérisé en ce que** :

• sa surface comprend des groupements fonctionnalisés et satisfaisant aux conditions suivantes:

⇒ être stables au moins à une température comprise entre 15°C et 45 °C;
⇒ être stables à au moins un type de procédé de stérilisation applicable aux dispositifs médicaux; et en particulier aux rayonnements gamma;

• et sa surface présente des charges, par l'intermédiaire desquelles sont liées par liaisons ioniques des molécules ou macromolécules d'un premier type exhibant, une fois liées, des groupements amines libres sur lesquels sont fixées, par liaison covalente, des molécules d'un deuxième type qui, une fois liées, exhibent des groupements carboxyliques sur lesquels sont fixés par liaison covalente:

o des molécules de troisième type ayant une fonction d'espaceur pour diminuer l'encombrement stérique et favoriser la fixation ultérieure d'un ligand et/ou de la molécule et/ou éléments figurés à épurer;
o et/ou des molécules d'un quatrième type ayant pour fonction d'augmenter l'hydrophilie du support en vue de limiter les interactions non spécifiques;
o et/ou des molécules d'un cinquième type exhibant, une fois liées, des groupements d'une autre nature, permettant un autre type de couplage covalent avec un ligand porteur notamment de groupements -CHO ou -COOH.

7. Support fonctionnalisé selon l'une quelconque des revendications précédentes, **caractérisé en ce qu'**il est une membrane semi-perméable fabriquée à partir d'un copolymère d'acrylonitrile et de méthallyle sulfonate de sodium.

8. Support fonctionnalisé selon l'une quelconque des revendications précédentes, **caractérisé en ce que** le ligand est sélectionné dans un groupe constitué d'anticorps, d'antigènes, de peptides, de protéines, ou glycoprotéines, des hormones, des enzymes, de cofacteurs de ceux-ci, des substrats ou des inhibiteurs de ceux-ci, des polysaccharides, des lectines, des toxines ou des antitoxines, des acides nucléiques ou polynucléotides, des haptènes ou des ligands d'haptènes, des pigments ou colorants.

9. Support selon l'une quelconque des revendications précédentes, **caractérisé en ce qu'**il entre dans la constitution d'une membrane notamment une membrane de dialyse sous forme de films plans ou de fibres, poreux ou non, pleines ou creuses, des microbilles poreuses ou non ou d'une combinaison de ceux-ci.

10. Module pour l'épuration extracorporelle spécifique d'un fluide biologique **caractérisé en ce qu'**il comprend au moins un compartiment pour la circulation dudit fluide, ce compartiment étant délimité au moins partiellement par un support fonctionnalisé selon l'une quelconque des revendications précédentes.

11. Module polyvalent pour l'épuration extracorporelle d'un fluide biologique comprenant au moins un compartiment

pour la circulation dudit fluide, ce compartiment étant délimité au moins partiellement par un support fonctionnalisé:

• dont la surface comprend des groupements fonctionnalisés et satisfaisant aux conditions suivantes:

⇒ être stables au moins à une température comprise entre environ 15°C et environ 45 °C;
⇒ être stables à au moins un type de procédé de stérilisation applicable aux dispositifs médicaux; et en particulier aux rayonnements gamma;

• et dont la surface présente des charges, par l'intermédiaire desquelles sont liées par liaisons ioniques des molécules ou macromolécules d'un premier type exhibant, une fois liées, des groupements amines libres sur lesquels sont fixées, par liaison covalente, des molécules d'un deuxième type qui, une fois liées, exhibent des groupements carboxyliques aptes à former, directement ou indirectement, des liaisons covalentes avec des ligands porteurs de groupements:
-CHO, $-NH_2$, -COOH, -SH, -OH,

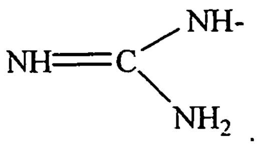

12. Module pour l'épuration extracorporelle non spécifique d'un fluide biologique **caractérisé en ce qu'**il comprend un support fonctionnalisé :

• dont la surface comprend des groupements fonctionnalisés et satisfaisant aux conditions suivantes:

⇒ être stables au moins à une température comprise entre 15°C et 45 °C;
⇒ être stables à au moins un type de procédé de stérilisation applicable aux dispositifs médicaux; et en particulier aux rayonnements gamma;

• et dont la surface présente des charges, par l'intermédiaire desquelles sont liées par liaisons ioniques des molécules ou macromolécules d'un premier type exhibant, une fois liées, des groupements amines libres sur lesquels sont fixées, par liaison covalente, des molécules d'un deuxième type qui, une fois liées, exhibent des groupements carboxyliques sur lesquels sont fixés par liaison covalente:

o des molécules de troisième type ayant une fonction d'espaceur pour diminuer l'encombrement stérique et favoriser la fixation ultérieure d'un ligand et/ou de la molécule et/ou éléments figurés à épurer;
o et/ou des molécules d'un quatrième type ayant pour fonction d'augmenter l'hydrophilie du support en vue de limiter les interactions non spécifiques;
o et/ou des molécules d'un cinquième type exhibant, une fois liées, des groupements d'une autre nature, permettant un autre type de couplage covalent avec un ligand porteur notamment de groupements - CHO ou -COOH.

13. Procédé de préparation d'un support biocompatible fonctionnalisé polyvalent pour l'épuration extracorporelle spécifique d'un fluide biologique, ou d'un module polyvalent pour l'épuration extracorporelle spécifique d'un fluide biologique, ce module comprenant au moins un compartiment pour la circulation dudit fluide, ce compartiment étant délimité au moins partiellement par le susdit support fonctionnalisé,

→ ce support étant tel que:

• sa surface comprend des groupements fonctionnalisés et satisfaisant aux conditions suivantes:

⇒ être stables au moins à une température comprise entre 15°C et 45°C;
⇒ être stables à au moins un type de procédé de stérilisation applicable aux dispositifs médicaux; et en particulier aux rayonnements gamma;

• et que sa surface présente des charges, par l'intermédiaire desquelles sont liées par liaisons ioniques

des molécules ou macromolécules d'un premier type exhibant, une fois liées, des groupements amines libres sur lesquels sont fixées, par liaison covalente, des molécules d'un deuxième type qui, une fois liées, exhibent des groupements carboxyliques permettant le couplage direct ou indirect, de manière covalente, de ligands spécifiques;

→ et cette épuration extracorporelle spécifique d'un fluide biologique représentant une thérapeutique pour l'élimination de molécules ou macromolécules dont la présence conduit à certaines pathologies ou certains dysfonctionnements biologiques;

**caractérisé en ce qu'**il consiste

a. à sélectionner le ligand ou le mélange de ligands appropriés en fonction de la pathologie donnée;
b. à réaliser un couplage du support avec le ligand ou les mélanges de ligands, pour obtenir un produit de couplage.

**14.** Procédé de préparation du support mis en oeuvre dans le procédé selon la revendication 13, ce support étant biocompatible fonctionnalisé et porteur de groupements susceptibles de former des liaisons covalentes avec des groupements organiques, notamment
-CHO, -NH2, -COOH, -SH, -OH,

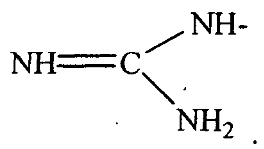

**caractérisé en ce que** ledit support est obtenu :

- par réaction de supports à caractère ionique avec:

→ soit

a) de la polyéthylèneimine (PEI) de masse moléculaire comprise entre 10.000 et 2.000 000, substituée ou non; puis avec
b) une solution d'un anhydride d'acide carboxylique de formule:

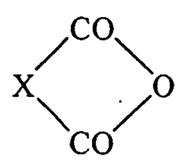

dans laquelle X = $[CH_2]n$, n étant compris entre 1 et 4,
ou X = -CH=CH-,
ou de formule:

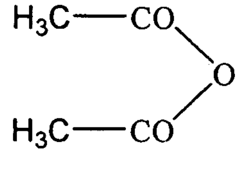

→ soit avec le produit de la réaction entre a) et b);

- et éventuellement enfin :

→ soit c) par fixation par liaison covalente sur les groupements carboxyliques:

• des molécules de troisième type ayant une fonction d'espaceur pour diminuer l'encombrement stérique et favoriser la fixation ultérieure d'un ligand et/ou de la molécule et/ou éléments figurés à épurer;
• et/ou des molécules d'un quatrième types ayant pour fonction d'augmenter l'hydrophilie du support en vue de limiter les interactions non spécifiques:
• et/ou des molécules d'un cinquième type exhibant, une fois liées, des groupements d'une autre nature, permettant un autre type de couplage covalent avec un ligand porteur notamment de groupements -CHO ou -COOH:

→ soit par mise en contact du support ainsi modifié avec une di-hydrazide de formule:

$$NH_2\text{-NH-CO-Y-CO-NH-}NH_2,$$

Y étant choisi parmi les groupements qui ont la double propriété d'être porteurs de groupements hydrophiles et d'être stables aux rayonnements γ, et qui sont de préférence un groupement $[CH_2]_m$, dans lequel m est compris entre 2 et 6.

**15.** Procédé selon la revendication 14, dans lequel le support à caractère ionique est un copolymère d'acrylonitrile et de metallylsulfonate de Na.

**16.** Dispositif de circulation extracorporelle de sang incorporant un support selon l'une quelconque des revendications 1 à 9 ou un module selon l'une quelconque des revendications 10 à 12.

**17.** Dispositif selon la revendication 16, **caractérisé en ce qu'**il s'agit d'un dispositif de traitement thérapeutique pour l'élimination de molécules ou macromolécules dont la présence conduit à certaines pathologies ou certains dysfonctionnements biologiques.

**18.** Kit comprenant:

• un module polyvalent selon l'une quelconque des revendications 10 à 12,
• un récipient ou poche stérile contenant une solution activatrice de la réaction entre le support fonctionnalisé et un ligand,

- le cas échéant un récipient ou poche stérile contenant une solution du ligand choisi;
- le cas échéant les solutions tampons ou de rinçages nécessaires à l'activation et/ou au couplage covalent du ligand.

## Claims

**1.** A functionalised support for the specific extracorporeal purification of a biological fluid, **characterised in that:**

• the surface thereof comprises functionalised groups which satisfy the following conditions:

⇒ being stable at least at a temperature of between 15°C and 45°C;
⇒ being stable to at least one type of sterilisation process applicable to medical devices; and in particular to gamma radiation;

• and the surface thereof comprises charges, by means of which are bound by ionic bonds molecules or macromolecules of a first type exhibiting, once bound, free amine groups to which are attached, by covalent bond, molecules of a second type which, once bound, exhibit carboxylic groups having allowed direct or indirect covalent coupling of specific ligands.

**2.** A functionalised support according to claim 1, **characterised in that** the carboxylic groups have directly or indirectly formed covalent bonds with ligands bearing groups:

-CHO, $-NH_2$, -COOH, -SH, -OH,

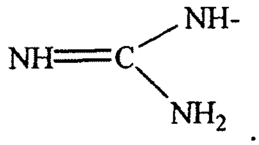

**3.** A functionalised support according to claim 1, **characterised in that** molecules of a third type having a spacer function for reducing steric hindrance and promoting subsequent attachment of a ligand and/or of the molecule or formed elements to be purified are attached by covalent bond onto the carboxylic groups.

**4.** A functionalised support according to claim 1, **characterised in that** molecules of a fourth type aiming to increase the hydrophilicity of the support for limiting non-specific interactions are attached by covalent bond onto the carboxylic groups.

**5.** A functionalised support according to claim 1, **characterised in that** molecules of a fifth type exhibiting, once bound, groups of another nature, allowing another type of covalent coupling with a ligand bearing in particular -CHO or -COOH groups are attached by covalent bond onto the carboxylic groups.

**6.** A functionalised support for the extracorporeal purification of a biological fluid, **characterised in that:**

- the surface thereof comprises functionalised groups which satisfy the following conditions:
  - ⇒ being stable at least at a temperature of between 15°C and 45°C;
  - ⇒ being stable to at least one type of sterilisation process applicable to medical devices; and in particular to gamma radiation;
- and the surface thereof comprises charges, by means of which are bound via ionic bonds molecules or macromolecules of a first type exhibiting, once bound, free amine groups to which are attached, by covalent bond, molecules of a second type which, once bound, exhibit carboxylic groups onto which are attached by covalent bond:
  - o molecules of a third type having a spacer function for reducing steric hindrance and promoting subsequent attachment of a ligand and/or of the molecule and/or formed elements to be purified;
  - o and/or molecules of a fourth type aiming to increase the hydrophilicity of the support for limiting non-specific interactions;
  - o and/or molecules of a fifth type exhibiting, once bound, groups of another nature, allowing another type of covalent coupling with a ligand bearing in particular -CHO or -COOH groups.

**7.** A functionalised support according to any one of the preceding claims, **characterised in that** it is a semipermeable membrane manufactured from an acrylonitrile and sodium methallyl sulfonate copolymer.

**8.** A functionalised support according to any one of the preceding claims, **characterised in that** the ligand is selected from a group made up of antibodies, antigens, peptides, proteins, or glycoproteins, hormones, enzymes, cofactors of the latter, substrates or inhibitors of the latter, polysaccharides, lectins, toxins or antitoxins, nucleic acids or polynucleotides, haptens or hapten ligands, pigments or dyes.

**9.** A support according to any one of the preceding claims, **characterised in that** it is a constituent part of a membrane, in particular a dialysis membrane in the form of planar films or solid or hollow fibres, porous or non-porous, porous or non-porous microbeads or a combination thereof.

**10.** A module for the specific extracorporeal purification of a biological fluid, **characterised in that** it comprises at least one compartment for circulation of said fluid, this compartment being defined at least in part by a functionalised support according to any one of the preceding claims.

**11.** A multi purpose module for the extracorporeal purification of a biological fluid comprising at least one compartment

for circulation of said fluid, this compartment being defined at least in part by a functionalised support:

- the surface of which comprises functionalised groups which satisfy the following conditions:

  ⇒ being stable at least at a temperature of between approximately 15°C and approximately 45°C;
  ⇒ being stable to at least one type of sterilisation process applicable to medical devices; and in particular to gamma radiation;

- and the surface of which comprises charges, by means of which are bound by ionic bonds molecules or macromolecules of a first type exhibiting, once bound, free amine groups to which are attached, by covalent bond, molecules of a second type which, once bound, exhibit carboxylic groups able to form, directly or indirectly, covalent bonds with ligands bearing groups:
  -CHO, $-NH_2$, -COOH, -SH, -OH,

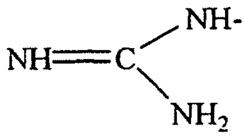
.

**12.** A module for the non-specific extracorporeal purification of a biological fluid, **characterised in that** it comprises a functionalised support:

- the surface of which comprises functionalised groups which satisfy the following conditions:

  ⇒ being stable at least at a temperature of between 15°C and 45°C;
  ⇒ being stable to at least one type of sterilisation process applicable to medical devices; and in particular to gamma radiation;

- and the surface of which comprises charges, by means of which are bound via ionic bonds molecules or macromolecules of a first type exhibiting, once bound, free amine groups to which are attached, by covalent bond, molecules of a second type which, once bound, exhibit carboxylic groups onto which are attached by covalent bond:

  o molecules of a third type having a spacer function for reducing steric hindrance and promoting subsequent attachment of a ligand and/or of the molecule and/or formed elements to be purified;
  o and/or molecules of a fourth type aiming to increase the hydrophilicity of the support for limiting non-specific interactions;
  o and/or molecules of a fifth type exhibiting, once bound, groups of another nature, allowing another type of covalent coupling with a ligand bearing in particular -CHO or -COOH groups.

**13.** A method of preparing a multi purpose functionalised biocompatible support for the specific extracorporeal purification of a biological fluid, or a multi purpose module for specific extracorporeal purification of a biological fluid, this module comprising at least one compartment for circulation of said fluid, this compartment being defined at least in part by said functionalised support,

→ this support being such that:

- the surface thereof comprises functionalised groups which satisfy the following conditions:

  ⇒ being stable at least at a temperature of between 15°C and 45°C;
  ⇒ being stable to at least one type of sterilisation process applicable to medical devices; and in particular to gamma radiation;

- and the surface thereof comprises charges, by means of which are bound by ionic bonds molecules or macromolecules of a first type exhibiting, once bound, free amine groups to which are attached, by covalent bond, molecules of a second type which, once bound, exhibit carboxylic groups allowing direct or indirect

covalent coupling of specific ligands;

→ and this specific extracorporeal purification of a biological fluid constituting a therapeutic treatment for removing molecules or macromolecules, the presence of which gives rise to certain pathological conditions or certain biological dysfunctions;

**characterised in that** it involves

a. selecting the appropriate ligand or mixture of ligands depending on the pathological condition in question;
b. carrying out coupling of the support with the ligand or mixture of ligands, in order to obtain a coupling product.

**14.** A method of preparing the support used in the method according to claim 13, this support being biocompatible, functionalised and bearing groups capable of forming covalent bonds with organic groups, in particular -CHO, $-NH_2$, -COOH, -SH, -OH,

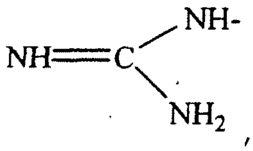

**characterised in that** said support is obtained:

- by reaction of supports of an ionic nature with:

→ either

a) substituted or unsubstituted polyethyleneimine (PEI) of a molecular mass of between 10,000 and 2,000,000; then with
b) a solution of an anhydride of carboxylic acid of the formula:

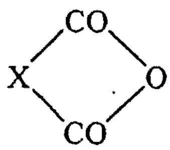

in which X = $[CH_2]_n$, n being between 1 and 4,
or X = -CH=CH-,
or of the formula:

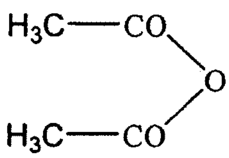

→ or with the product of the reaction between a) and b):

- and optionally finally:

→ either c) by attachment by covalent bond onto the carboxylic groups:

• of molecules of a third type having a spacer function for reducing steric hindrance and promoting subsequent attachment of a ligand and/or of the molecule and/or formed elements to be purified;

• and/or of molecules of a fourth type aiming to increase the hydrophilicity of the support for limiting non-specific interactions;
• and/or of molecules of a fifth type exhibiting, once bound, groups of another nature, allowing another type of covalent coupling with a ligand bearing in particular -CHO or -COOH groups;

→ or by contacting the support modified in this manner with a dihydrazide of the formula:

$$NH_2\text{-}NH\text{-}CO\text{-}Y\text{-}CO\text{-}NH\text{-}NH_2,$$

Y being selected from groups which have the double property of bearing hydrophilic groups and of being stable to y radiation, and which are preferably a $[CH_2]_m$ group, in which m is between 2 and 6.

**15.** A method according to claim 14, in which the support of an ionic nature is an acrylonitrile and Na methallyl sulfonate copolymer.

**16.** A device for the extracorporeal circulation of blood incorporating a support according to any one of claims 1 to 9 or a module according to any one of claims 10 to 12.

**17.** A device according to claim 16, **characterised in that** it is a device for therapeutic treatment for removing molecules or macromolecules, the presence of which gives rise to certain pathological conditions or certain biological dysfunctions.

**18.** A kit comprising:

• a multi purpose module according to any one of claims 10 to 12,
• a sterile receptacle or pouch containing a solution which activates the reaction between the functionalised support and a ligand,

- if need be, a sterile receptacle or pouch containing a solution of the selected ligand;
- if need be, the buffer or rinsing solutions required for activating and/or for covalent coupling of the ligand.

**Patentansprüche**

**1.** Funktionalisierter Träger für die spezifische Aufreinigung einer biologischen Flüssigkeit außerhalb des Körpers, **dadurch gekennzeichnet, dass:**

• seine Oberfläche funktionalisierte Gruppen umfasst und diese die folgenden Bedingungen erfüllen:

⇒ zumindest bei einer Temperatur zwischen 15°C und 45°C stabil zu sein;
⇒ zumindest gegenüber einer Art Sterilisationsverfahren, die für medizinische Vorrichtungen verwendbar ist, und insbesondere gegenüber Gammastrahlen, stabil zu sein;

• und seine Oberfläche Ladungen aufweist, durch die über Ionenbindungen Moleküle oder Makromoleküle eines ersten Typs gebunden sind, die, sobald sie gebunden sind, freie Amingruppen aufweisen, an die durch kovalente Bindung Moleküle eines zweiten Typs gebunden sind, die, sobald sie gebunden sind, Carboxylgruppen aufweisen, welche die direkte oder indirekte kovalente Kopplung von spezifischen Liganden erlauben.

**2.** Funktionalisierter Träger nach Anspruch 1, **dadurch gekennzeichnet, dass** die Carboxylgruppen direkt oder indirekt kovalente Bindungen mit Liganden gebildet haben, die die Gruppen:
$-CHO$, $-NH_2$, $-COOH$, $-SH$, $-OH$,

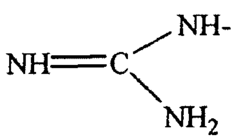

tragen.

**3.** Funktionalisierter Träger nach Anspruch 1, **dadurch gekennzeichnet, dass** an den Carboxylgruppen durch kovalente Bindung Moleküle eines dritten Typs gebunden sind, die eine Spacerfunktion haben, um die sterische Blokkierung zu verringern und die spätere Bindung eines Liganden und/oder des aufzureinigenden Moleküls oder der aufzureinigenden Blutkörperchen zu fördern.

**4.** Funktionalisierter Träger nach Anspruch 1, **dadurch gekennzeichnet, dass** an den Carboxylgruppen durch kovalente Bindung Moleküle eines vierten Typs gebunden sind, deren Funktion es ist, die Hydrophilie des Trägers zu erhöhen, um die unspezifischen Interaktionen einzuschränken.

**5.** Funktionalisierter Träger nach Anspruch 1, **dadurch gekennzeichnet, dass** an den Carboxylgruppen durch kovalente Bindung Moleküle eines fünften Typs gebunden sind, die, sobald sie gebunden sind, Gruppen einer anderen Art aufweisen, die eine andere Art der kovalenten Kopplung mit einem Liganden, der insbesondere die Gruppen -CHO oder -COOH trägt, erlauben.

**6.** Funktionalisierter Träger für die Aufreinigung einer biologischen Flüssigkeit außerhalb des Körpers, **dadurch gekennzeichnet, dass:**

• seine Oberfläche funktionalisierte Gruppen umfasst und diese die folgenden Bedingungen erfüllen:

⇒ zumindest bei einer Temperatur zwischen 15°C und 45°C stabil zu sein;
⇒ zumindest gegenüber einer Art Sterilisationsverfahren, die für medizinische Vorrichtungen verwendbar ist, und insbesondere gegenüber Gammastrahlen, stabil zu sein;

• und seine Oberfläche Ladungen aufweist, durch die über Ionenbindungen Moleküle oder Makromoleküle eines ersten Typs gebunden sind, die, sobald sie gebunden sind, freie Amingruppen aufweisen, an die durch kovalente Bindung Moleküle eines zweiten Typs gebunden sind, die, sobald sie gebunden sind, Carboxylgruppen aufweisen, an die durch kovalente Bindung gebunden sind:

o Moleküle eines dritten Typs, die eine Spacerfunktion haben, um die sterische Blockierung zu verringern und die spätere Bindung eines Liganden und/oder des aufzureinigenden Moleküls und/oder der aufzureinigenden Blutkörperchen zu fördern;
o und/oder Moleküle eines vierten Typs, deren Funktion es ist, die Hydrophilie des Trägers zu erhöhen, um die unspezifischen Interaktionen einzuschränken;
o und/oder Moleküle eines fünften Typs, die, sobald sie gebunden sind, Gruppen einer anderen Art aufweisen, die eine andere Art der kovalenten Kopplung mit einem Liganden, der insbesondere die Gruppen -CHO oder -COOH trägt, erlauben.

**7.** Funktionalisierter Träger nach einem der vorhergehenden Ansprüche, **dadurch gekennzeichnet, dass** er eine semipermeable Membran ist, die aus einem Copolymer ausgehend von Acrylnitril und Natriummethallylsulfonat hergestellt wurde.

**8.** Funktionalisierter Träger nach einem der vorhergehenden Ansprüche, **dadurch gekennzeichnet, dass** der Ligand aus einer Gruppe bestehend aus Antikörpern, Antigenen, Peptiden, Proteinen oder Glykoproteinen, Hormonen, Enzymen, deren Cofaktoren, deren Substraten oder Inhibitoren, Polysacchariden, Lektinen, Toxinen oder Antitoxinen, Nukleinsäuren oder Polynukleotiden, Haptenen oder Liganden von Haptenen, Pigmenten oder Farbstoffen ausgewählt ist.

**9.** Träger nach einem der vorhergehenden Ansprüche, **dadurch gekennzeichnet, dass** er in den Aufbau einer Membran, insbesondere einer Dialysemembran in Form ebener Folien, oder gefüllter oder hohler Fasern, poröser oder nicht-poröser, poröser nicht-poröser Mikrokügelchen oder einer Mischung davon, eingefügt wird.

**10.** Einheit für die spezifische Aufreinigung einer biologischen Flüssigkeit außerhalb des Körpers **dadurch gekennzeichnet, dass** sie mindestens ein Kompartiment für die Zirkulation der Flüssigkeit enthält, wobei das Kompartiment zumindest teilweise durch einen funktionalisierten Träger nach einem der vorhergehenden Ansprüche begrenzt ist.

**11.** Vielseitige Einheit für die Aufreinigung einer biologischen Flüssigkeit außerhalb des Körpers, umfassend mindestens

ein Kompartiment für die Zirkulation der Flüssigkeit, wobei das Kompartiment zumindest teilweise durch einen funktionalisierten Träger begrenzt ist:

• dessen Oberfläche funktionalisierte Gruppen umfasst und diese folgenden Bedingungen erfüllen:

⇒ zumindest bei einer Temperatur zwischen etwa 15°C und etwa 45°C stabil zu sein;
⇒ zumindest gegenüber einer Art Sterilisationsverfahren, die für medizinische Vorrichtungen verwendbar ist, und insbesondere gegenüber Gammastrahlen, stabil zu sein;

• und dessen Oberfläche Ladungen aufweist, durch die über Ionenbindungen Moleküle oder Makromoleküle eines ersten Typs gebunden sind, die, sobald sie gebunden sind, freie Amingruppen aufweisen, an die durch kovalente Bindung Moleküle eines zweiten Typs gebunden sind, die, sobald sie gebunden sind, Carboxylgruppen aufweisen, welche geeignet sind direkt oder indirekt kovalente Bindungen mit Liganden zu bilden, die die Gruppen:
-CHO, $-NH_2$, -COOH, -SH, -OH,

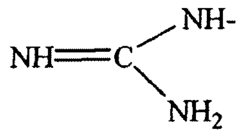

tragen.

**12.** Einheit für die unspezifische Aufreinigung einer biologischen Flüssigkeit außerhalb des Körpers **dadurch gekennzeichnet, dass** sie einen funktionalisierten Träger umfasst:

• dessen Oberfläche funktionalisierte Gruppen umfasst und diese folgenden Bedingungen erfüllen:

⇒ zumindest bei einer Temperatur zwischen 15°C und 45°C stabil zu sein;
⇒ zumindest gegenüber einer Art Sterilisationsverfahren, die für medizinische Vorrichtungen verwendbar ist, und insbesondere gegenüber Gammastrahlen, stabil zu sein;

• und dessen Oberfläche Ladungen aufweist, durch die über Ionenbindungen Moleküle oder Makromoleküle eines ersten Typs gebunden sind, die, sobald sie gebunden sind, freie Amingruppen aufweisen, an die durch kovalente Bindung Moleküle eines zweiten Typs gebunden sind, die, sobald sie gebunden sind, Carboxylgruppen aufweisen, an die durch kovalente Bindung gebunden sind:

o Moleküle eines dritten Typs, die eine Spacerfunktion haben, um die sterische Blockierung zu verringern und die spätere Bindung eines Liganden und/oder des aufzureinigenden Moleküls und/oder der aufzureinigenden Blutkörperchen zu fördern;
o und/oder Moleküle eines vierten Typs, deren Funktion es ist, die Hydrophilie des Trägers zu erhöhen, um die unspezifischen Interaktionen einzuschränken;
o und/oder Moleküle eines fünften Typs, die, sobald sie gebunden sind, Gruppen einer anderen Art aufweisen, die eine andere Art der kovalenten Kopplung mit einem Liganden, der insbesondere die Gruppen -CHO oder -COOH trägt, erlauben.

**13.** Verfahren zur Herstellung eines biokompatiblen, funktionalisierten, vielseitigen Trägers für die spezifische Aufreinigung einer biologischen Flüssigkeit außerhalb des Körpers oder einer vielseitigen Einheit für die spezifische Aufreinigung einer biologischen Flüssigkeit außerhalb des Körpers, wobei die Einheit mindestens ein Kompartiment für die Zirkulation der Flüssigkeit umfasst, wobei das Kompartiment zumindest teilweise durch den oben genannten funktionalisierten Träger begrenzt ist,

→ wobei der Träger so ist, dass:

• seine Oberfläche funktionalisierte Gruppen umfasst und diese die folgenden Bedingungen erfüllen:

⇒ zumindest bei einer Temperatur zwischen 15°C und 45°C stabil zu sein;
⇒ zumindest gegenüber einer Art Sterilisationsverfahren, die für medizinische Vorrichtungen verwendbar ist, und insbesondere gegenüber Gammastrahlen, stabil zu sein;

• und seine Oberfläche Ladungen aufweist, durch die über Ionenbindungen Moleküle oder Makromoleküle eines ersten Typs gebunden sind, die, sobald sie gebunden sind, freie Amingruppen aufweisen, an die durch kovalente Bindung Moleküle eines zweiten Typs gebunden sind, die, sobald sie gebunden sind, Carboxylgruppen aufweisen, welche die direkte oder indirekte kovalente Kopplung von spezifischen Liganden erlauben;

→ und die spezifische Aufreinigung einer biologischen Flüssigkeit außerhalb des Körpers eine Therapeutik zur Entfernung von Molekülen oder Makromolekülen darstellt, deren Vorhandensein zu bestimmten Pathologien oder bestimmten biologischen Funktionsstörungen führt;

**dadurch gekennzeichnet, dass** es aus

a. dem Auswählen des Liganden oder der Mischung von Liganden, die im Zusammenhang mit der angegebenen Pathologie geeignet sind;
b. dem Durchführen einer Kopplung des Trägers mit dem Liganden oder den Mischungen von Liganden, um ein Kopplungsprodukt zu erhalten,

besteht.

**14.** Verfahren zur Herstellung des Trägers, welcher durch das Verfahren nach Anspruch 13 hergestellt wird, wobei der Träger biokompatibel funktionalisiert ist und Gruppen trägt, die geeignet sind, kovalente Bindungen mit organischen Gruppen, insbesondere
-CHO, $-NH_2$, -COOH, -SH, -OH,

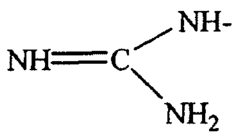

zu bilden, **dadurch gekennzeichnet, dass** der Träger erhalten wird:

- durch Umsetzen des ionischen Trägers mit:

→ entweder

a) substituiertem oder unsubstituiertem Polyethylenimin (PEI) mit einer Molekülmasse zwischen 10.000 und 2.000.000; dann mit
b) einer Lösung eines Carbonsäureanhydrids der Formel:

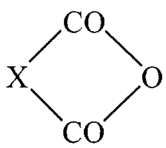

in der X = $[CH_2]n$, wobei n zwischen 1 und 4 ist,
oder X = -CH=CH-,
oder der Formel:

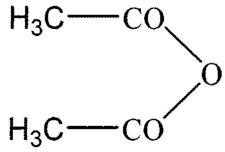

→ oder mit dem Produkt der Reaktion zwischen a) und b);

- und schließlich eventuell:

→ entweder c) durch Bindung von:

• Molekülen eines dritten Typs, die eine Spacerfunktion haben, um die sterische Blockierung zu verringern und die spätere Bindung eines Liganden und/oder des aufzureinigenden Moleküls und/oder der aufzureinigenden Blutkörperchen zu fördern;
• und/oder Molekülen eines vierten Typs, deren Funktion es ist, die Hydrophilie des Trägers zu erhöhen, um die unspezifischen Interaktionen einzuschränken;
• und/oder Molekülen eines fünften Typs, die, sobald sie gebunden sind, Gruppen einer anderen Art aufweisen, die eine andere Art der kovalenten Kopplung mit einem Liganden, der insbesondere die Gruppen -CHO oder -COOH trägt, erlauben,

an Carboxylgruppen durch kovalente Bindung;
→ oder durch Inkontaktbringen des so modifizierten Trägers mit einem Dihydrazid der Formel:

$$NH_2\text{-}NH\text{-}CO\text{-}Y\text{-}CO\text{-}NH\text{-}NH_2,$$

wobei Y ausgewählt ist aus den Gruppen, die die zweifache Eigenschaft haben, hydrophile Gruppen zu tragen und gegenüber gamma-Strahlen stabil zu sein, und die bevorzugt eine Gruppe $[CH_2]_m$ sind, in der m zwischen 2 und 6 ist.

**15.** Verfahren nach Anspruch 14, wobei der ionische Träger ein Copolymer aus Acrylnitril und Natriummethallylsulfonat ist.

**16.** Vorrichtung für die Zirkulation von Blut außerhalb des Körpers, umfassend einen Träger nach einem der Ansprüche 1 bis 9 oder eine Einheit nach einem der Ansprüche 10 bis 12.

**17.** Vorrichtung nach Anspruch 16, **dadurch gekennzeichnet, dass** es sich um eine Vorrichtung zur therapeutischen Behandlung handelt, um Moleküle oder Makromoleküle, deren Vorhandensein zu bestimmten Pathologien oder bestimmten biologischen Funktionsstörungen führt, zu entfernen.

**18.** Kit umfassend:

• eine vielseitige Einheit nach einem der Ansprüche 10 bis 12,
• einen sterilen Behälter oder eine sterile Tasche, der/die eine Aktivierungslösung für die Reaktion zwischen dem funktionalisierten Träger und einem Liganden enthält,

- gegebenenfalls einen sterilen Behälter oder eine sterile Tasche, der/die eine Lösung mit dem ausgewählten Liganden enthält,
- gegebenenfalls Puffer- oder Spüllösungen, die für die Aktivierung und/oder kovalente Kopplung des Liganden notwendig sind.

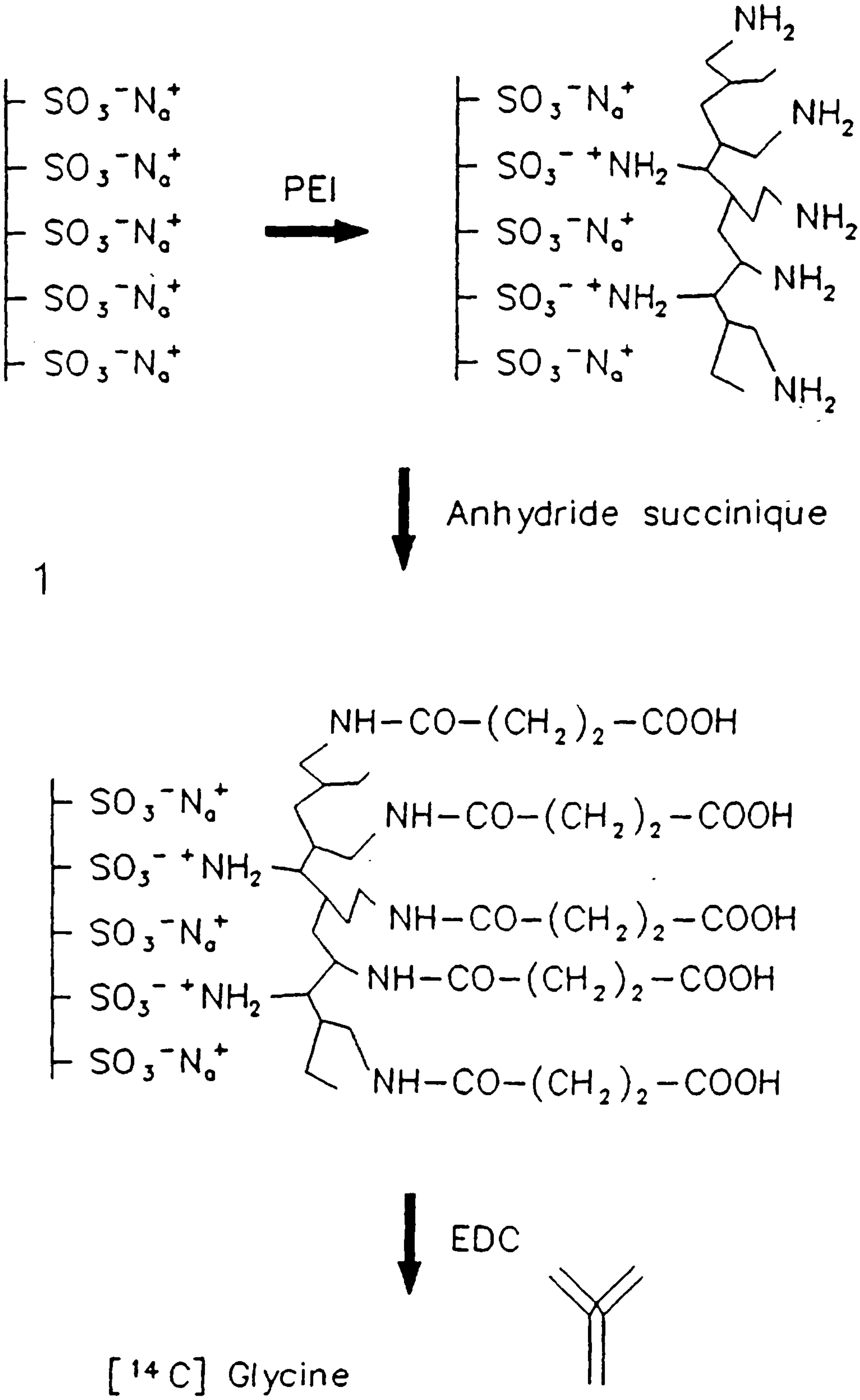


Fig. 1

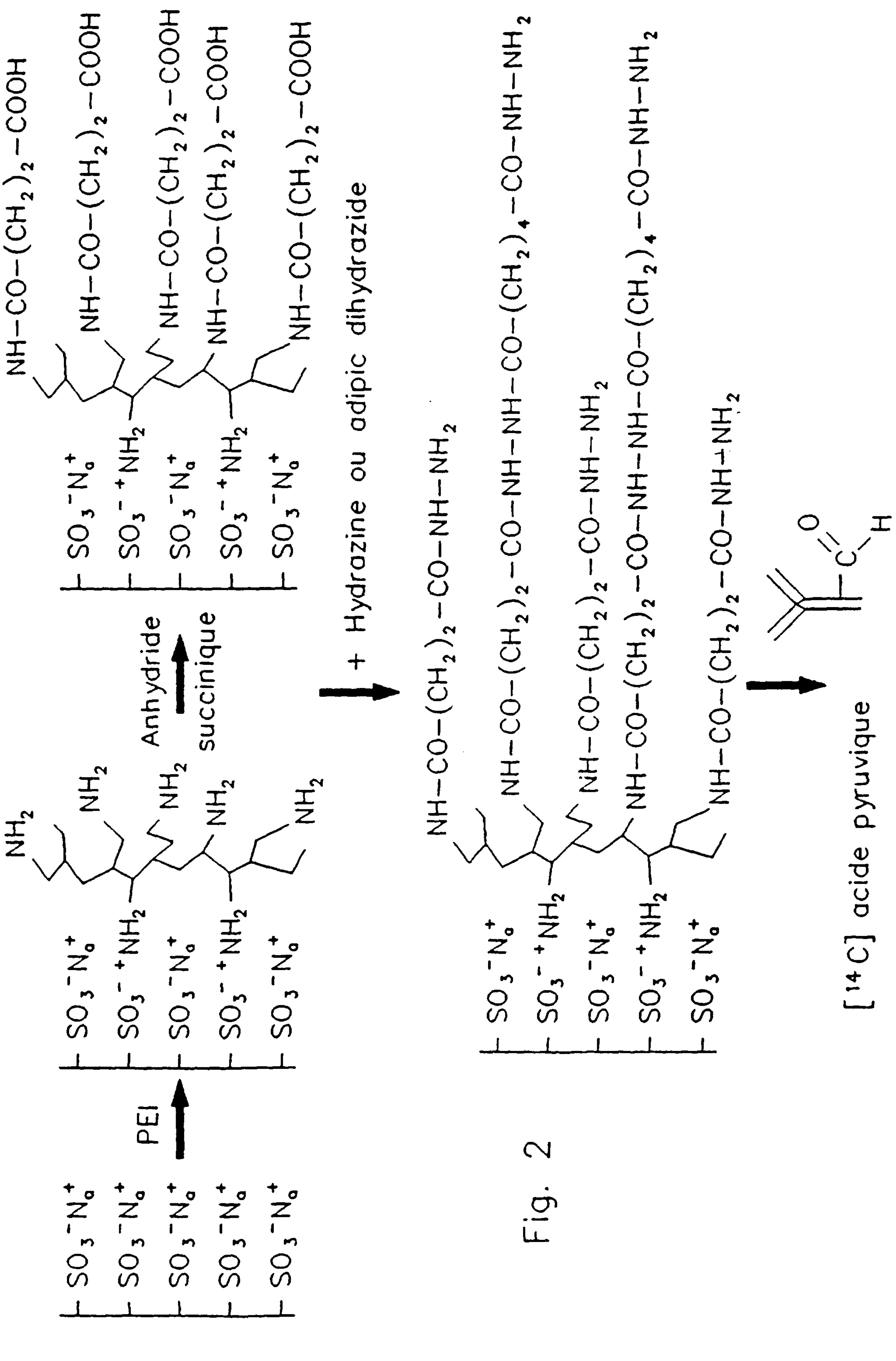
PEI
Anhydride succinique
+ Hydrazine ou adipic dihydrazide
[14C] acide pyruvique

Fig. 2

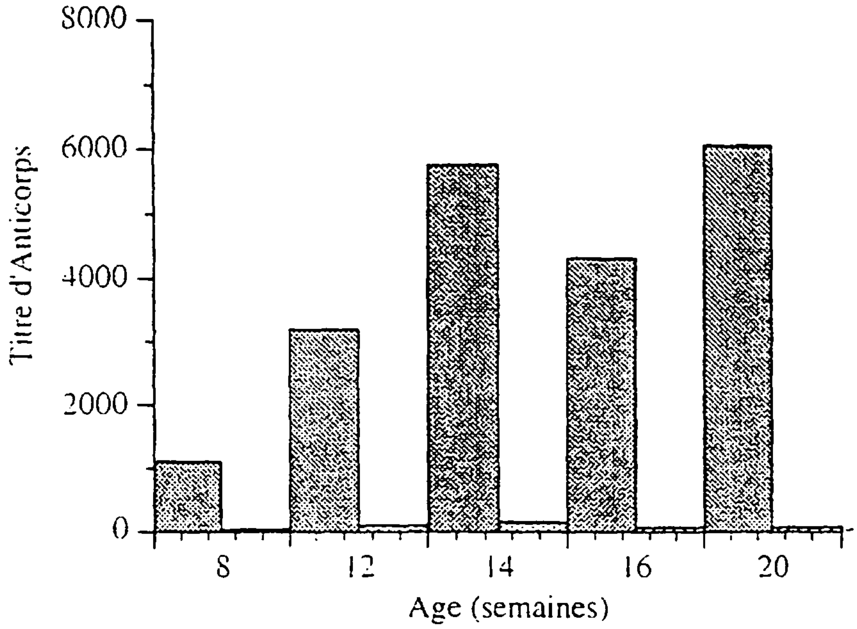
8000
6000
4000
2000
0
Titre d'Anticorps
8
12
14
16
20
Age (semaines)

Fig. 3

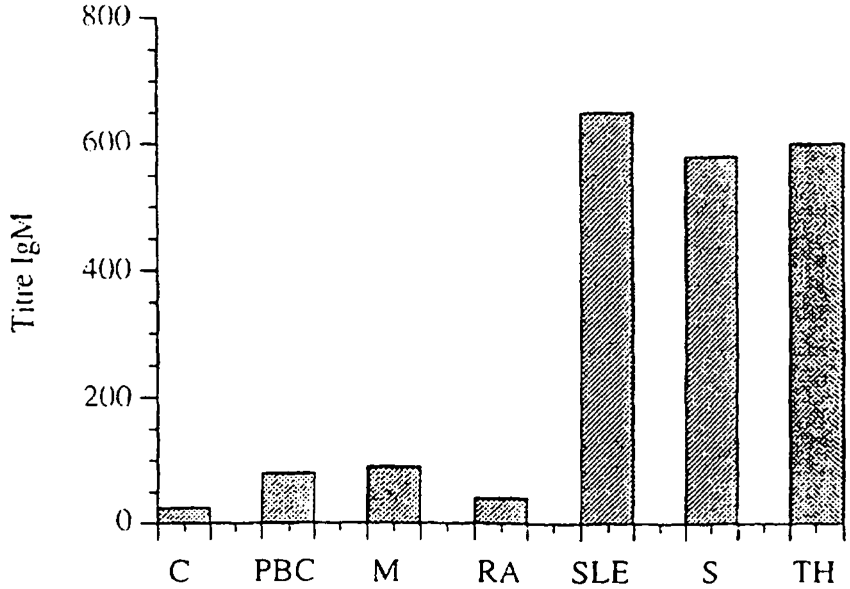
800
600
400
200
0
Titre IgM
C
PBC
M
RA
SLE
S
TH

Fig. 4

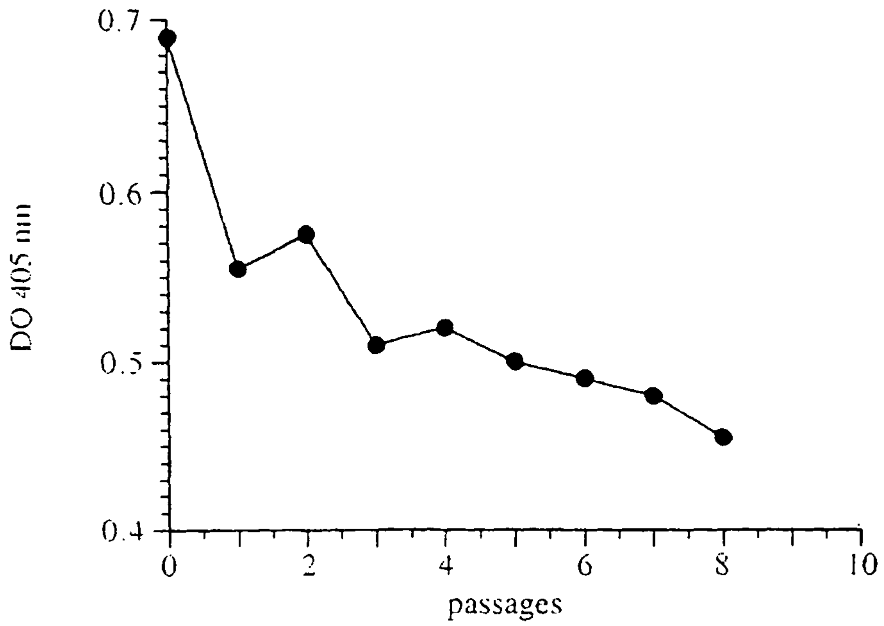
0.7
0.6
0.5
0.4
DO 405 nm
0
2
4
6
8
10
passages

Fig. 5

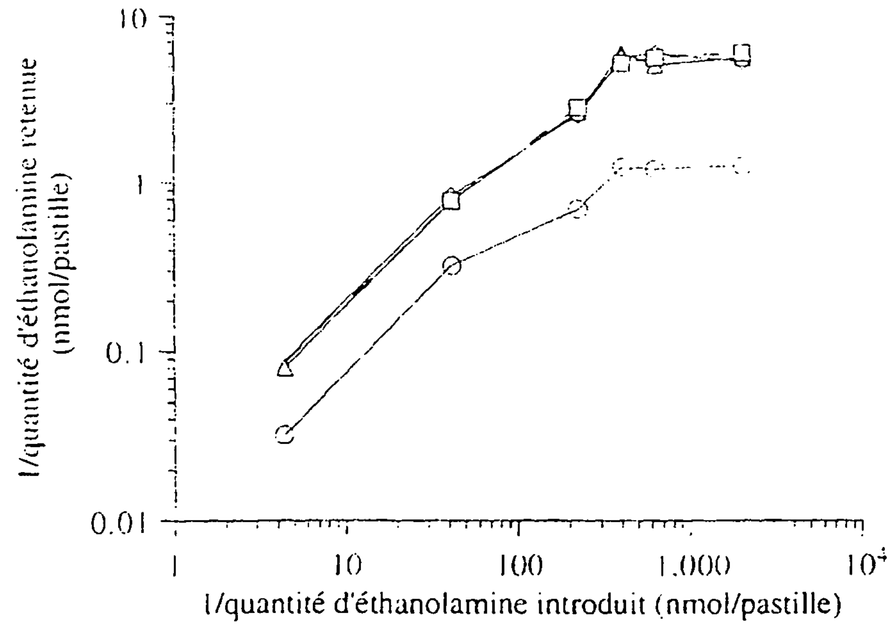
10
1
0.1
0.01
1/quantité d'éthanolamine retenue (nmol/pastille)
1
10
100
1.000
$10^4$
1/quantité d'éthanolamine introduit (nmol/pastille)

Fig. 6

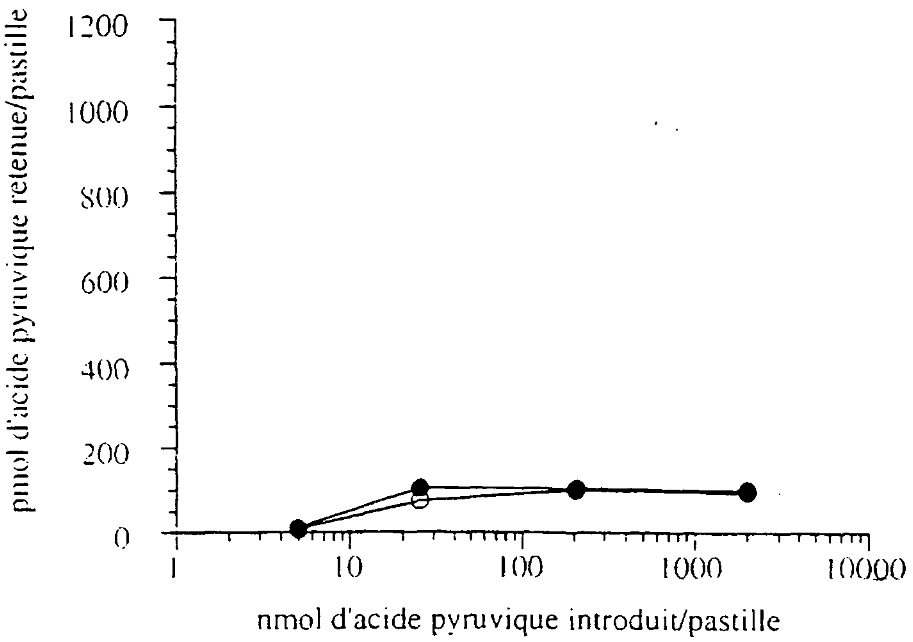
pmol d'acide pyruvique retenue/pastille
1200
1000
800
600
400
200
0
1
10
100
1000
10000
nmol d'acide pyruvique introduit/pastille

Fig. 7

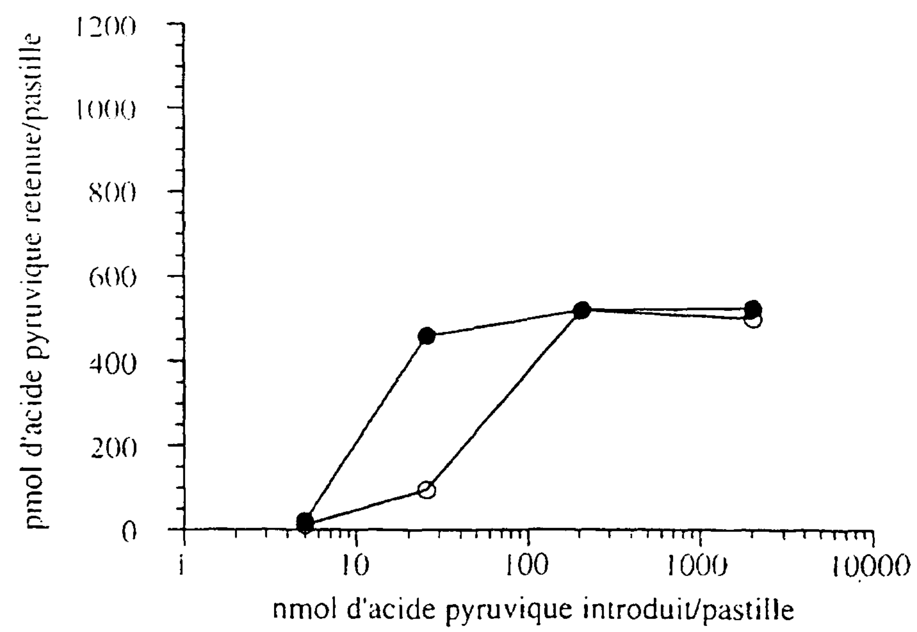
pmol d'acide pyruvique retenue/pastille
1200
1000
800
600
400
200
0
1
10
100
1000
10000
nmol d'acide pyruvique introduit/pastille

Fig. 8

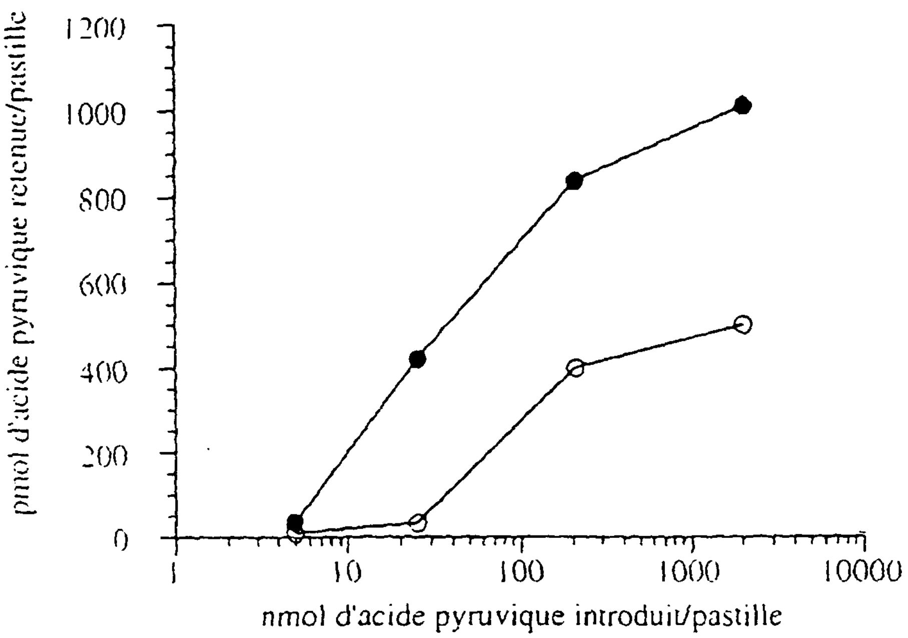
1200
1000
800
600
400
200
0
pmol d'acide pyruvique retenue/pastille
1
10
100
1000
10000
nmol d'acide pyruvique introduit/pastille

Fig. 9

Phase de préparation du module

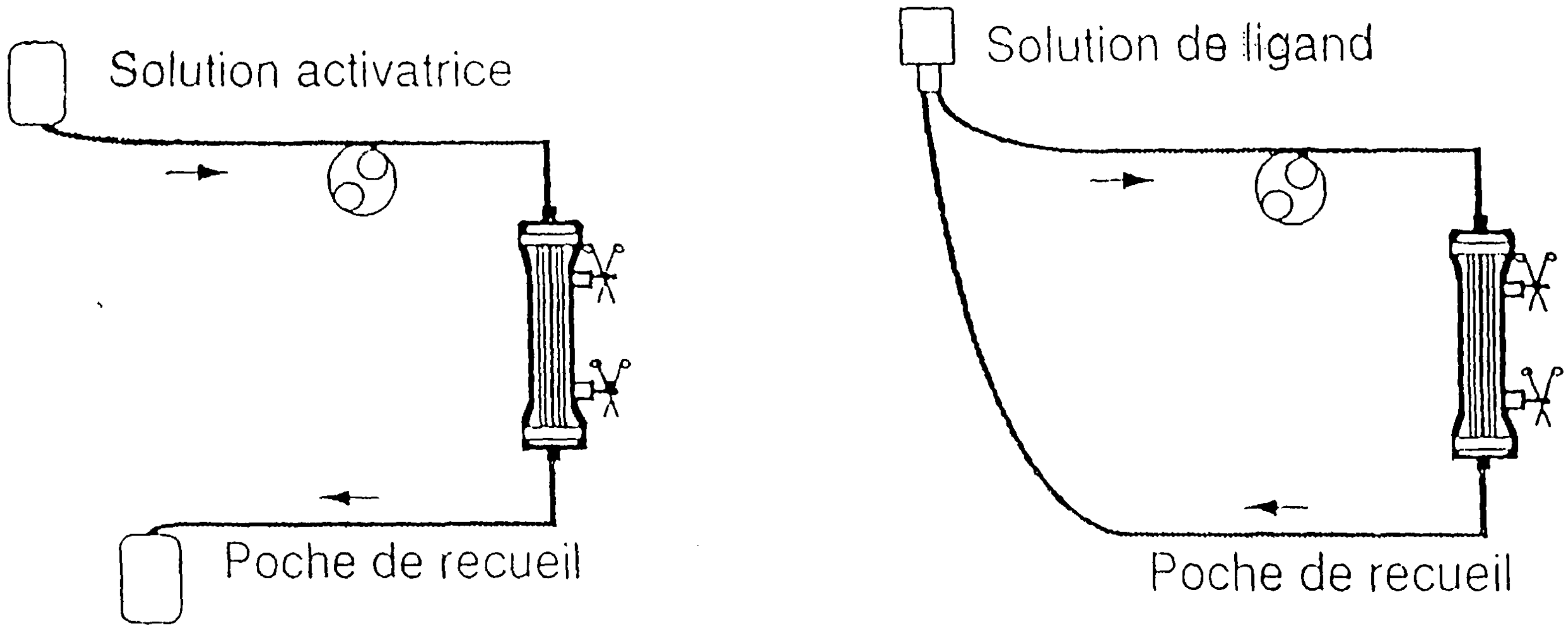


Fig. 10

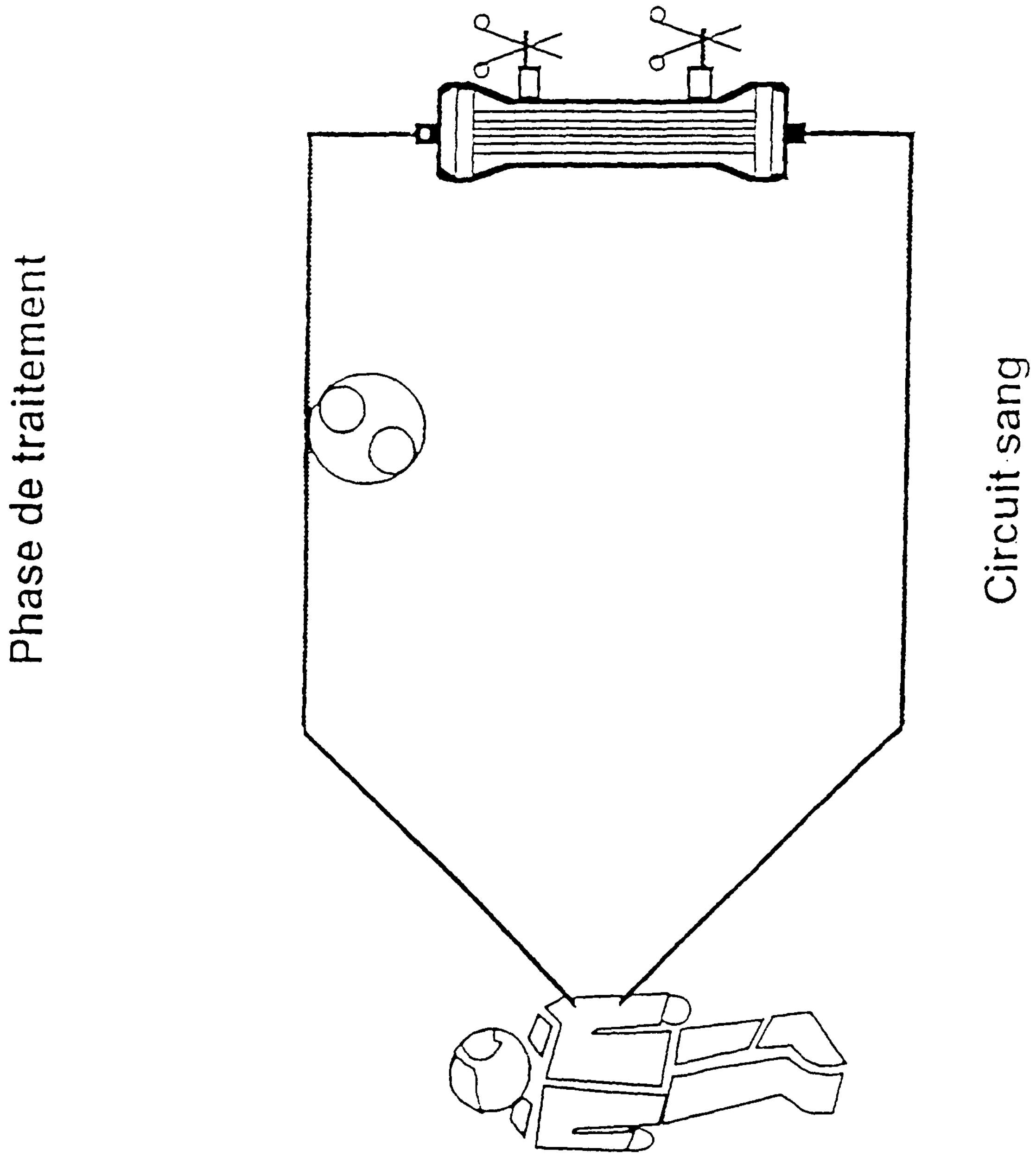


Fig. 11

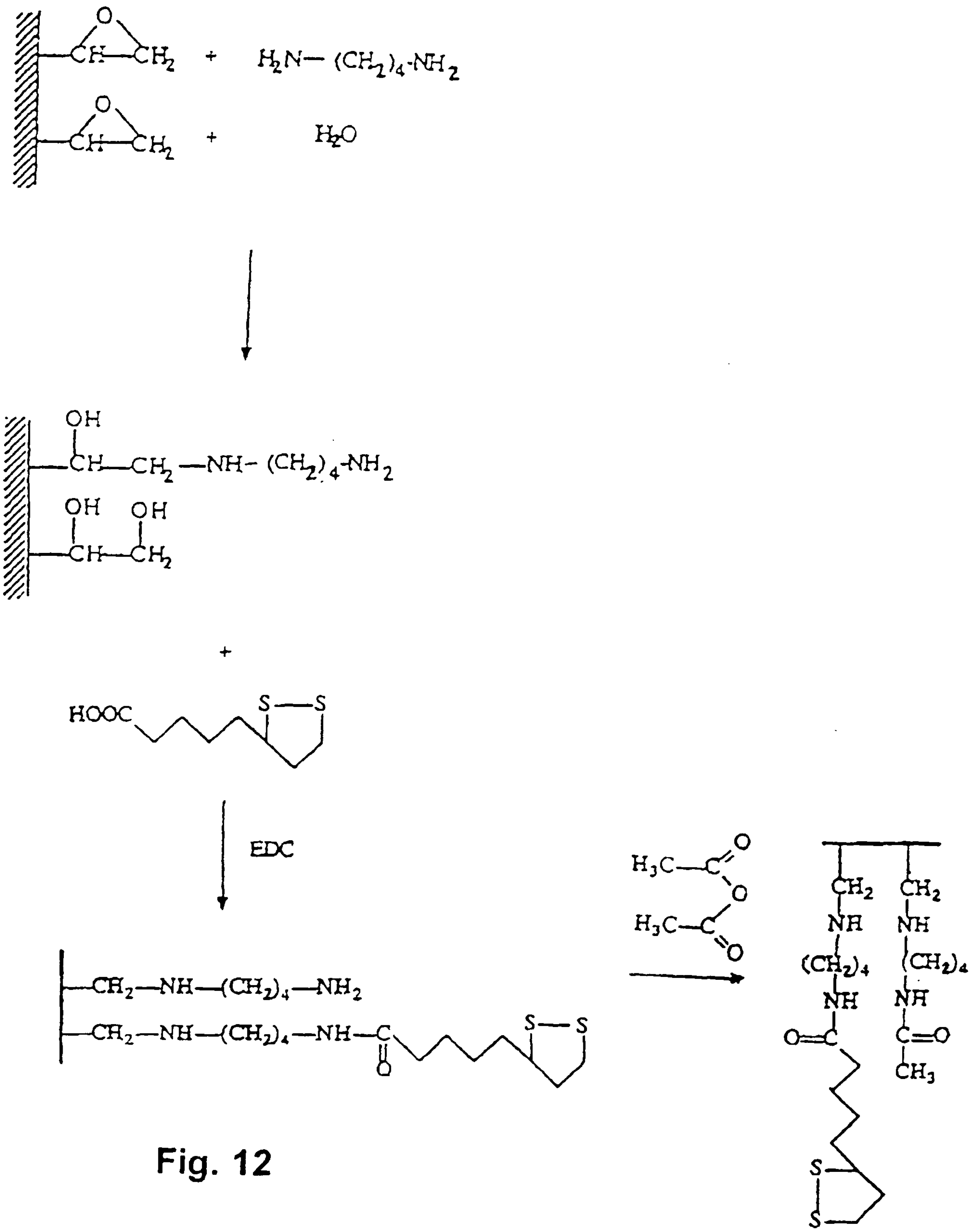


Fig. 12

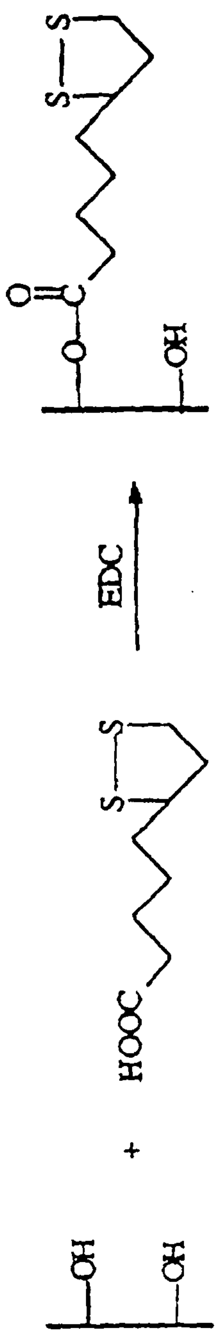


Fig. 13

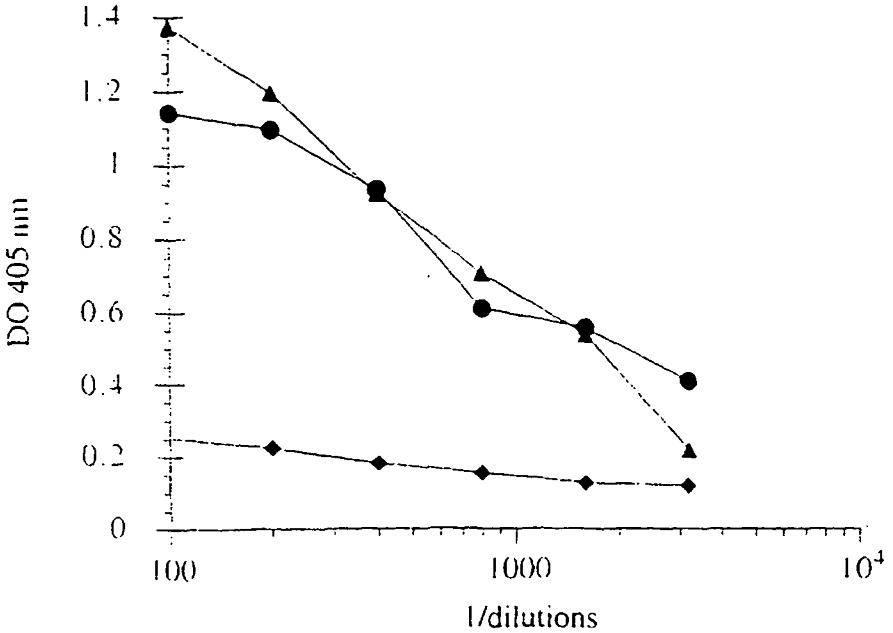
1.4
1.2
1
0.8
0.6
0.4
0.2
0
DO 405 nm
100
1000
$10^4$
1/dilutions

Fig. 14

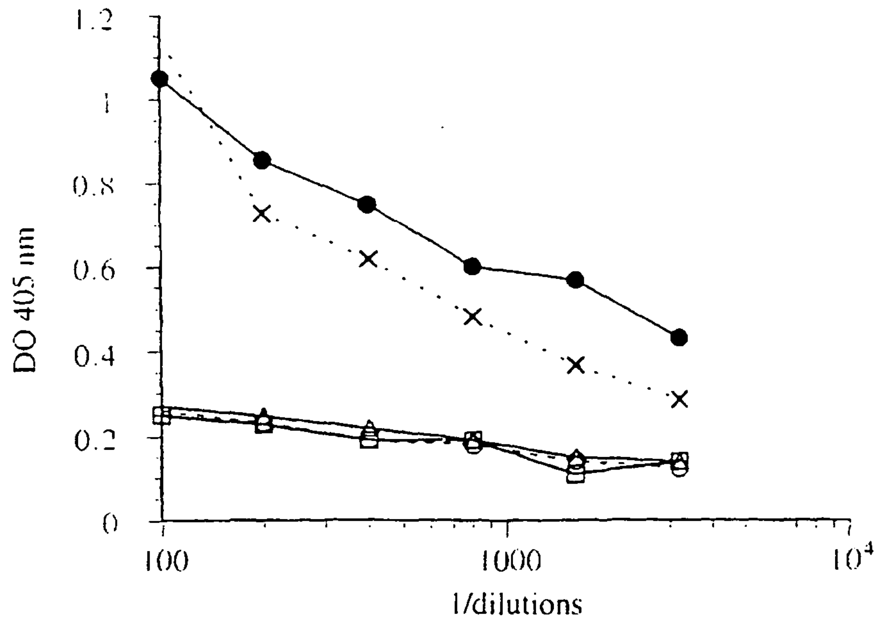
1.2
1
0.8
0.6
0.4
0.2
0
DO 405 nm
100
1000
$10^4$
1/dilutions

Fig. 15

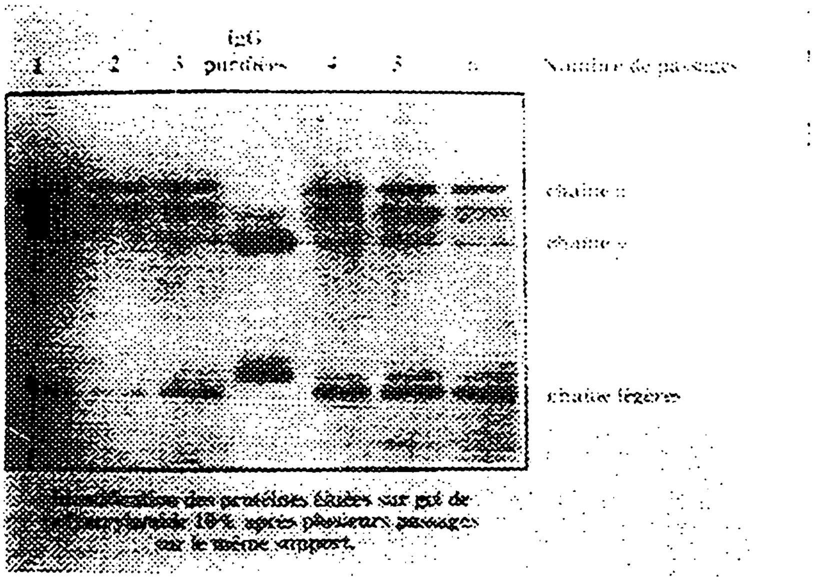


Fig. 16

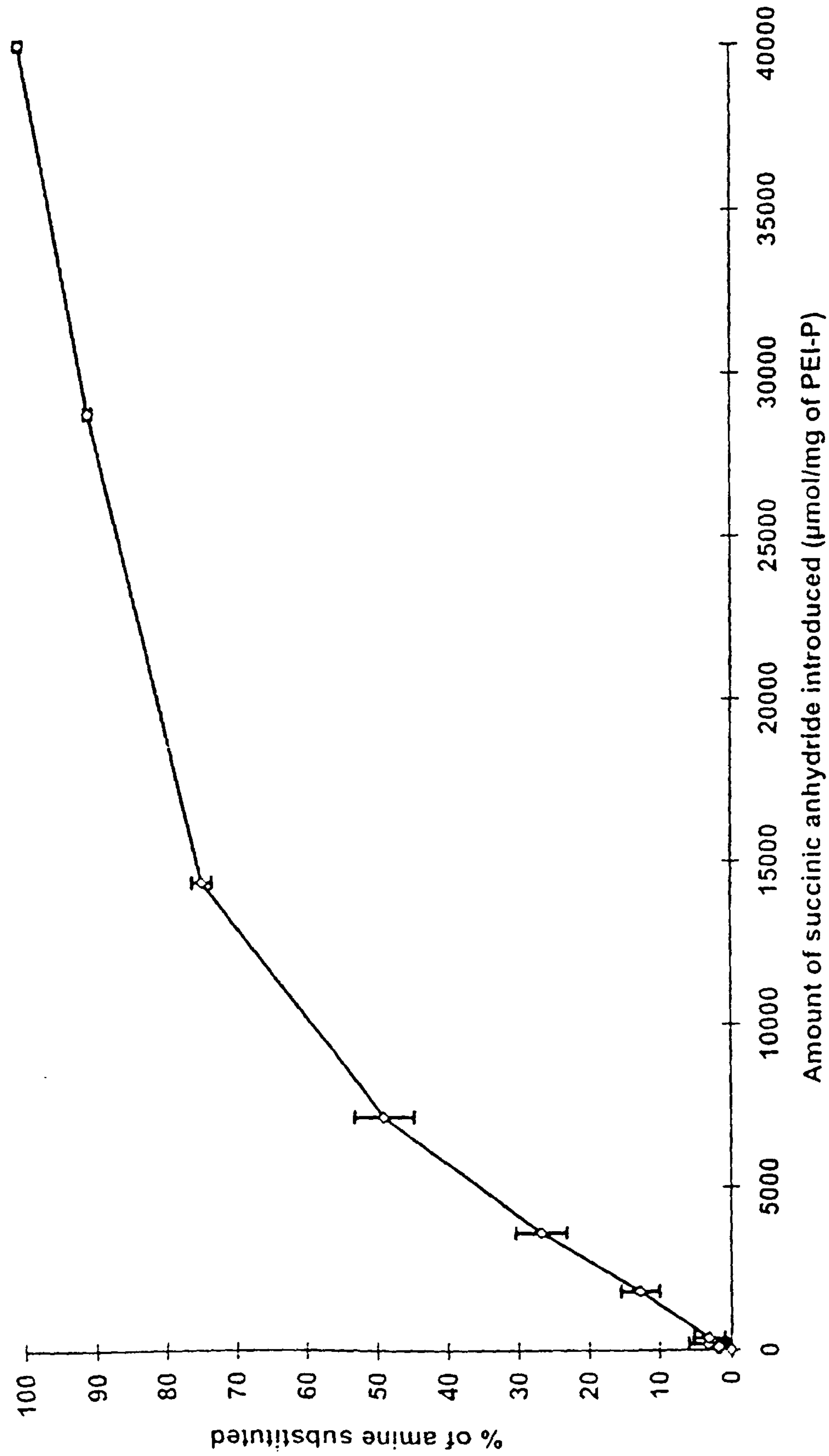
% of amine substituted
0
10
20
30
40
50
60
70
80
90
100
Amount of succinic anhydride introduced (µmol/mg of PEI-P)
0
5000
10000
15000
20000
25000
30000
35000
40000

Fig. 17

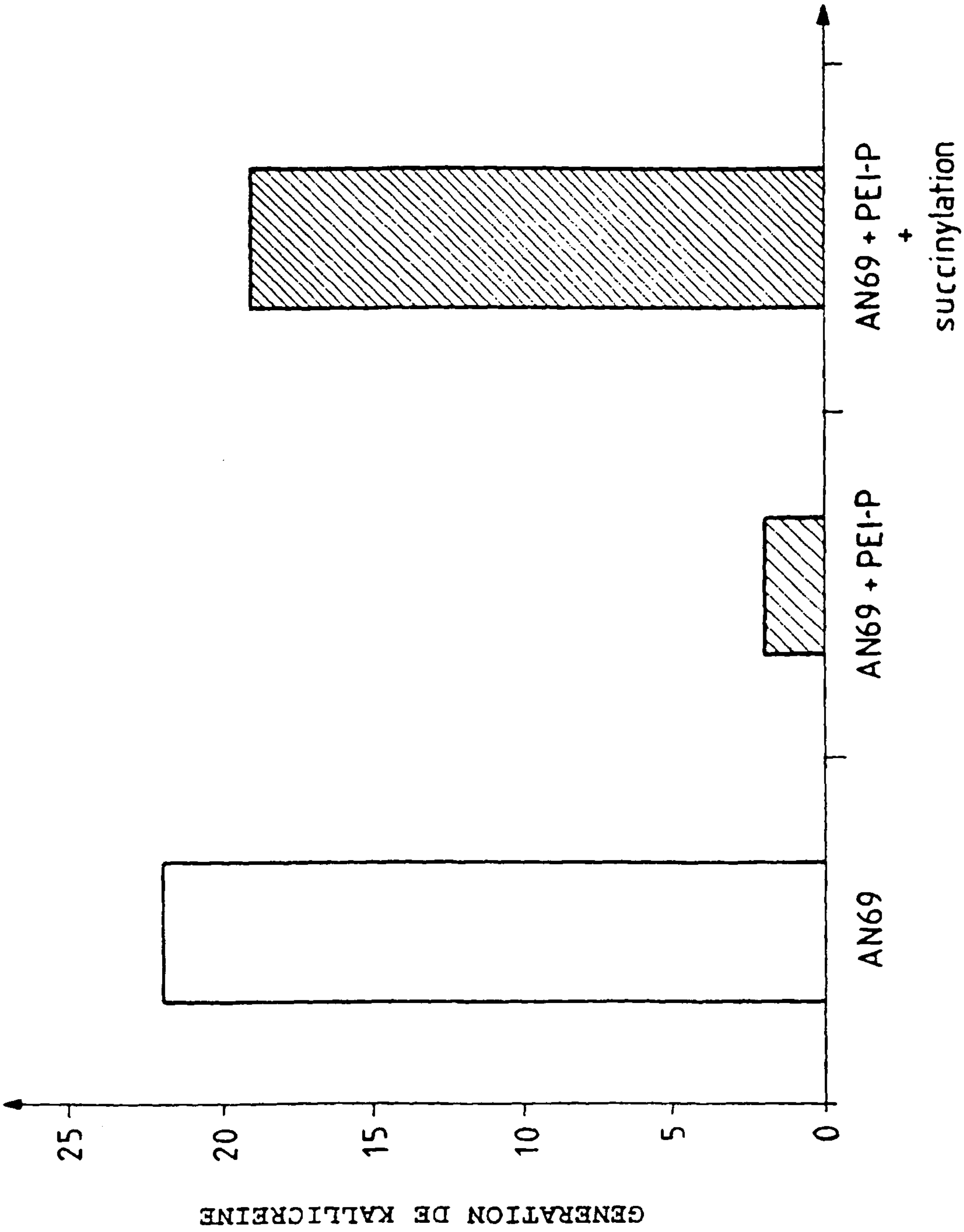
GENERATION DE KALLICREINE
0
5
10
15
20
25
AN69
AN69 + PEI-P
AN69 + PEI-P + succinylation

FIG_18

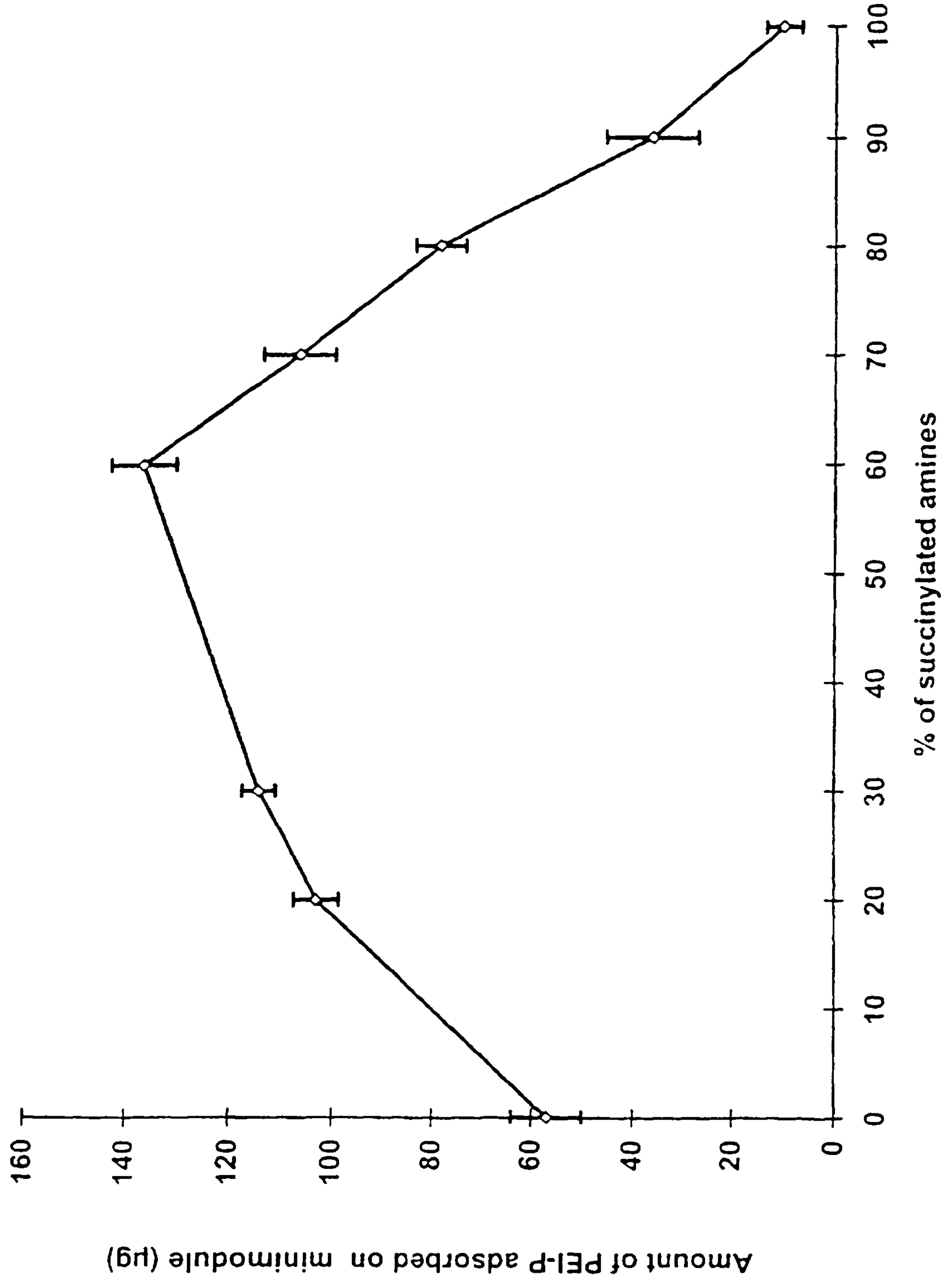
% of succinylated amines
Amount of PEI-P adsorbed on minimodule (µg)
0
10
20
30
40
50
60
70
80
90
100
0
20
40
60
80
100
120
140
160

Fig. 19

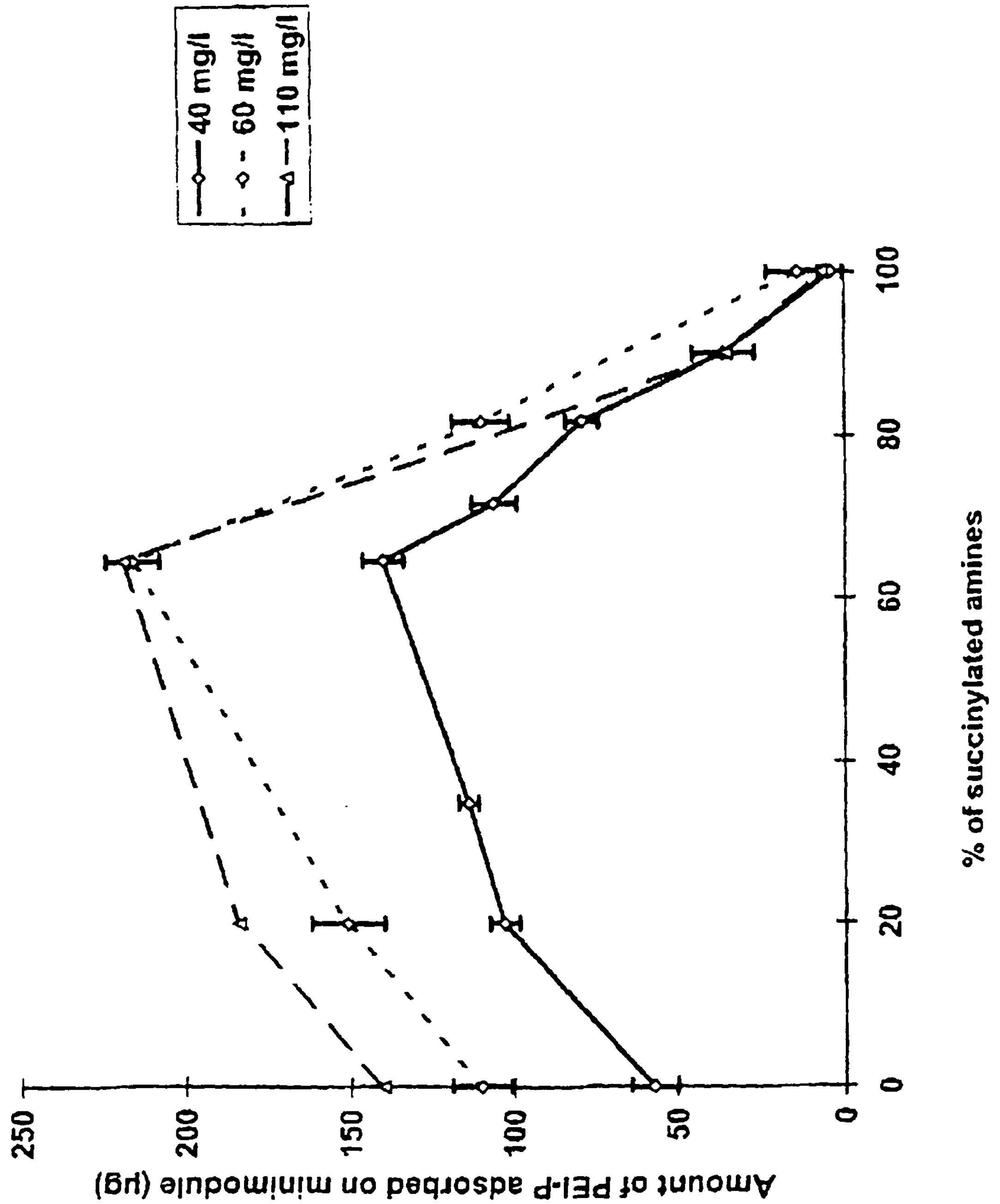
40 mg/l
60 mg/l
110 mg/l
Amount of PEI-P adsorbed on minimodule (µg)
250
200
150
100
50
0
0
20
40
60
80
100
% of succinylated amines

Fig. 20

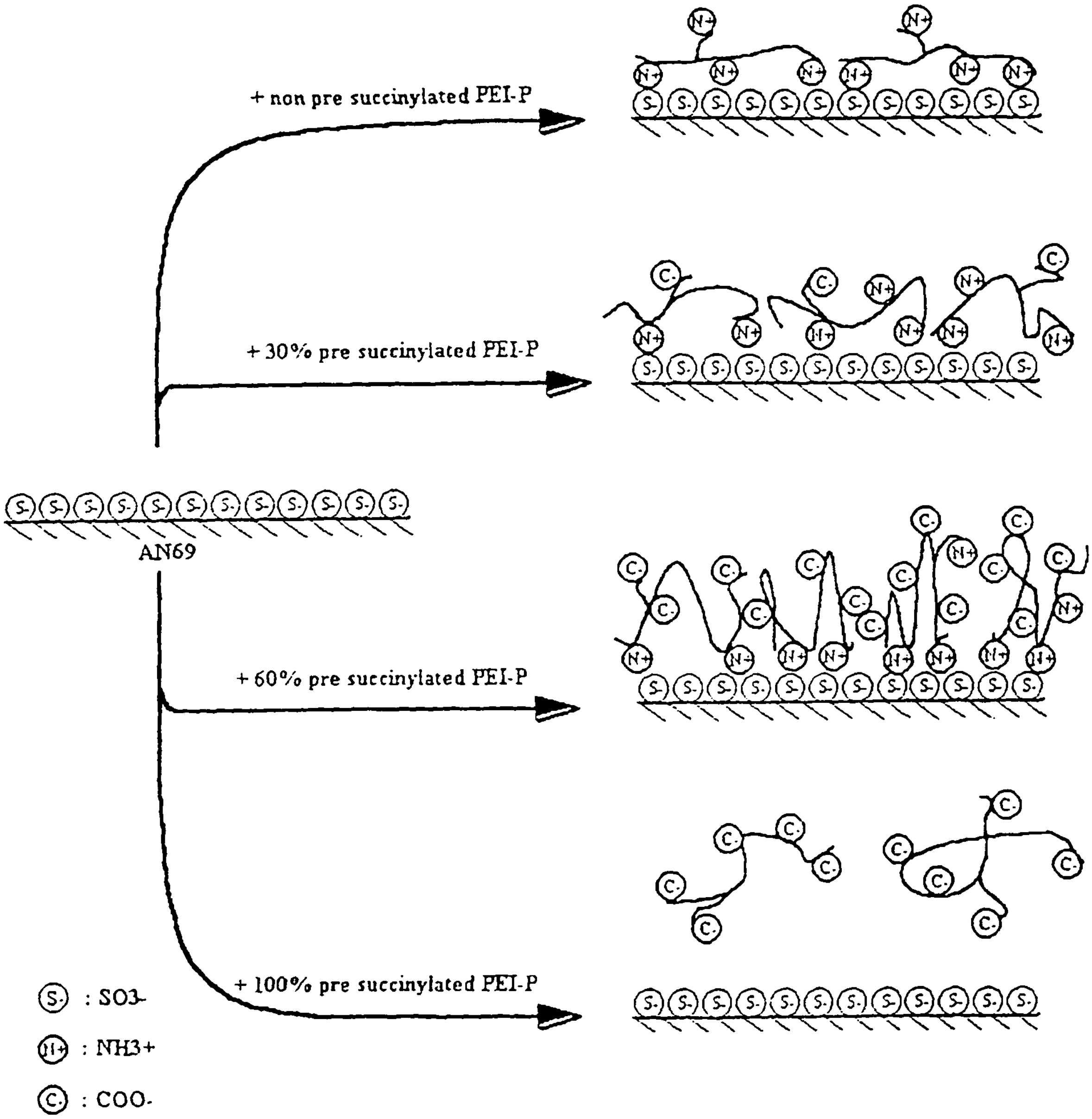
+ non pre succinylated PEI-P
+ 30% pre succinylated PEI-P
AN69
+ 60% pre succinylated PEI-P
+ 100% pre succinylated PEI-P
: SO3-
: NH3+
: COO-

Fig. 21

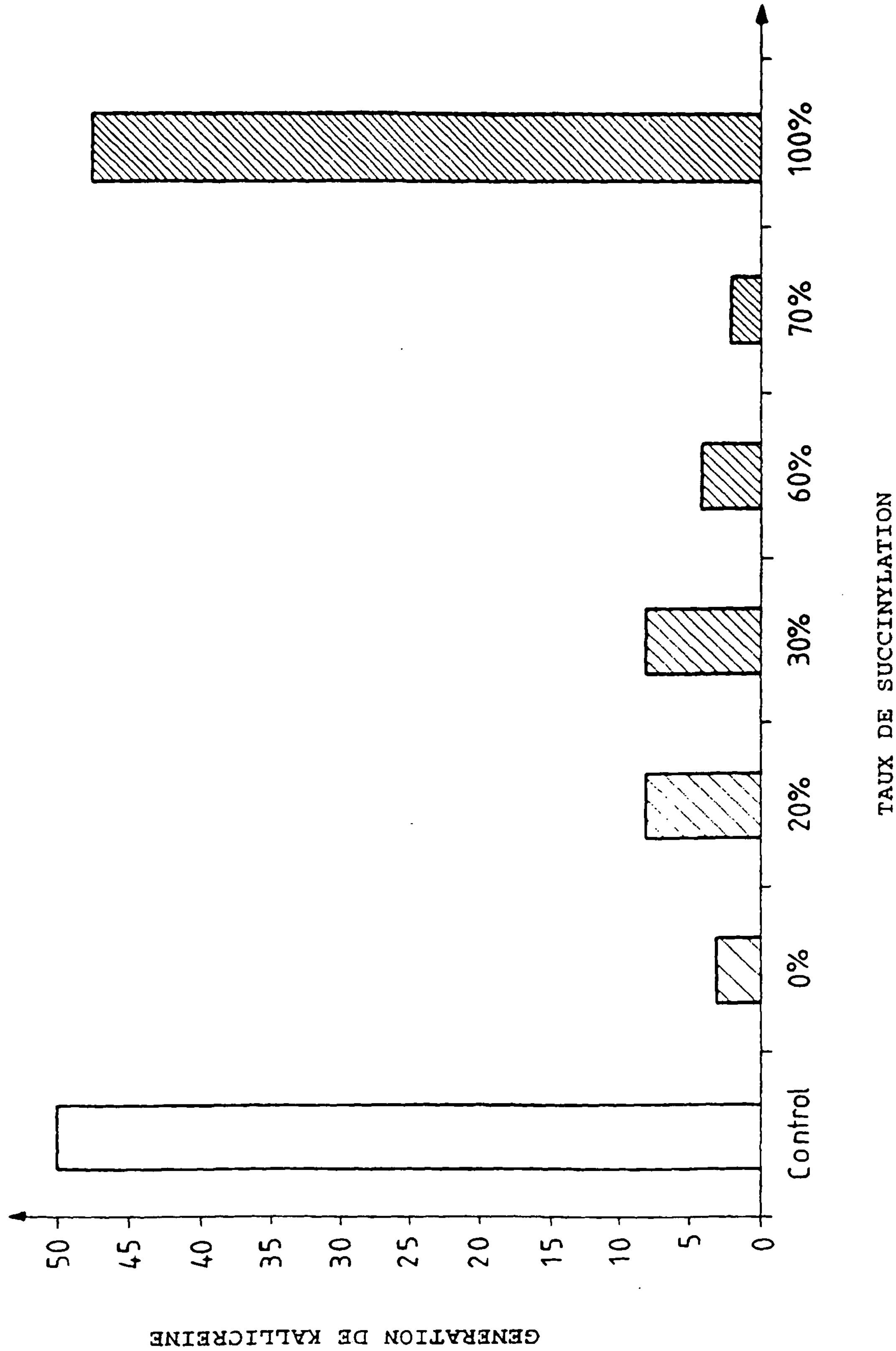
GENERATION DE KALLICREINE
0
5
10
15
20
25
30
35
40
45
50
Control
0%
20%
30%
60%
70%
100%
TAUX DE SUCCINYLATION

FIG_22

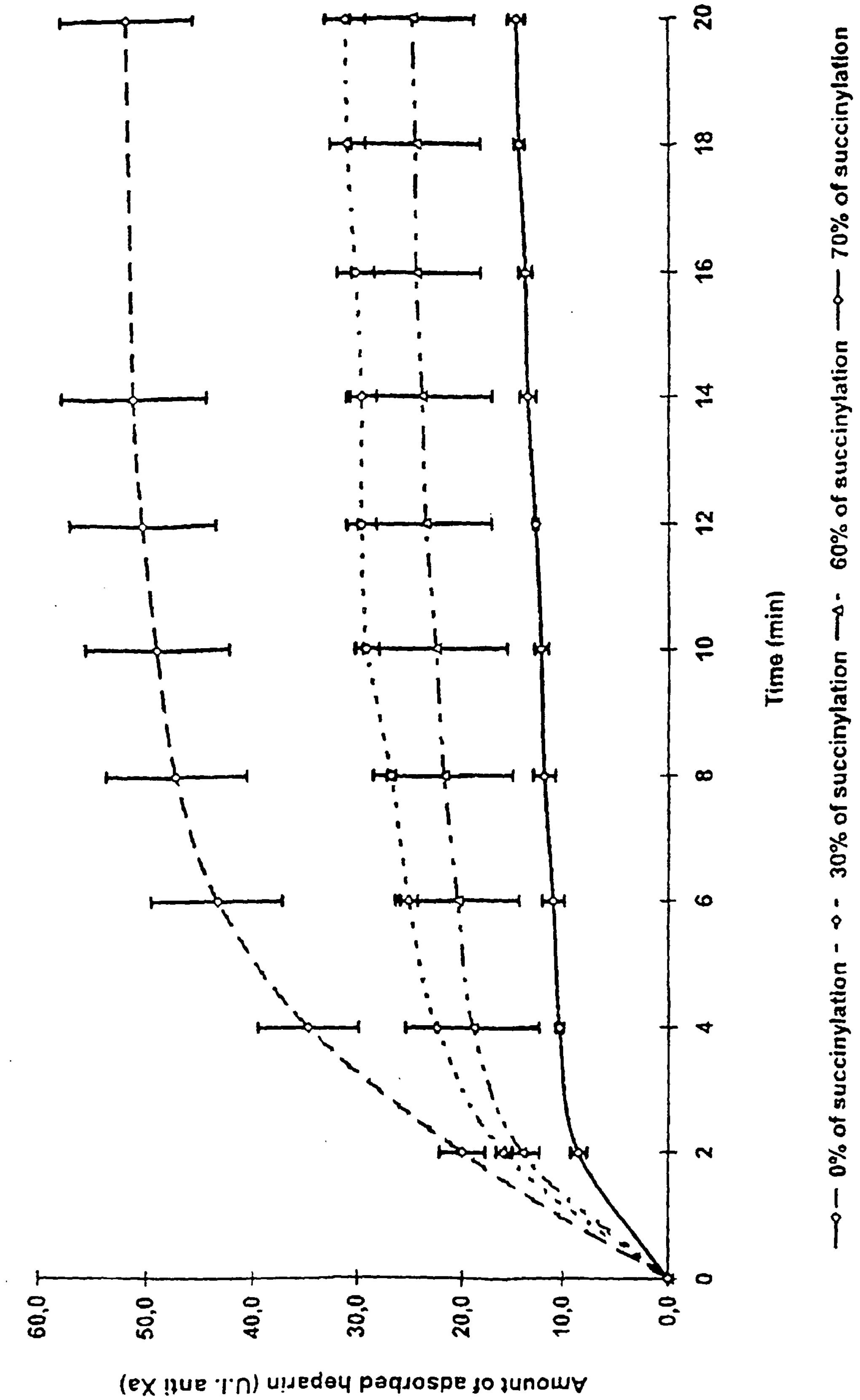
Amount of adsorbed heparin (U.I. anti Xa)
60,0
50,0
40,0
30,0
20,0
10,0
0,0
0
2
4
6
8
10
12
14
16
18
20
Time (min)
0% of succinylation
30% of succinylation
60% of succinylation
70% of succinylation

Fig. 23

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9207023 A
- WO 9207006 A
- WO 9205201 A
- DE 4209988 A
- GB 2197720 A
- US 5403917 B
- US 4217338 A


### Littérature non-brevet citée dans la description


- Anaphylactoide réactions associated with reuse of hollow-fiber hemodialyzers and ACE inhibitors. *Kidney International,* 1992, vol. 42, 1232-1237
- **S. Mitzner et al.** Extracorporal endotoxin removal by immobilized polyethyleneimine. *ARTIFICIAL ORGANS,* Septembre 1993, vol. 11 (9), 775-781
- **Kopaciewicz, W et al.** Synthesis of Cation-Exchange Stationary Phases Using an Adsorbed Polymeric Coating. *JOURNAL OF CHROMATOGRAPHY, NETHERLANDS,* 16 Mai 1986, vol. 358 (1), 107-117
- **Thomas et al.** *Colloids and surfaces A,* 1993, vol. 77, 125-139
- **Malmsten et al.** *J. Colloid and Interface Science,* 1996, vol. 177, 70-78
- Pratique de l'analyse organique calorimétrique et fluorométrique. **J. Bartos ; M. Tesez.** Pratique de l'analyse organique calorimétrique et fluorométrique. Masson, 1984, 84-85
- **Quash et al.** *J. Immunol. Methods,* 1978, vol. 22, 165-174
- **Bradford, A.P. ; Aitken, A. ; Beg, F. ; Cook, K.G. ; Yeaman, S.J.** Amino acid sequence surrounding the lipoic acid cofactor of bovine kidney 2-oxoglutarate dehydrogenase complex. *FEBS Lett.,* 1987, vol. 22 (1), 211
- **Bradford, A.P. ; Howell, S. ; Aitken, A. ; James, L.A. ; Yeaman, S.J.** Primary structure around the lipoate attachment site on the E2 component of bovine heart PDH complex. *Biochem. J.,* 1987, vol. 245 (3), 919
- **Click, R.E. ; Benck, L. ; Alter, B.J.** Enhancement of antibody response by mercaptoethanol. *Cell. Immunol.,* 1972, vol. 3, 156
- **Click, R.E. ; Benck, L. ; Alter, B.J.** Immune response in vitro. I. Culture conditions for antibody synthesis. *Cell. Immunol.,* 1972, vol. 3, 264
- **Flannery, G.R. ; Burroughs, A.K. ; Butler, P. ; Chelliah, J. ; Hamilton-Miller, J. ; Brumfitt, W. ; Baum, H.** Antimitochondrial antibodies in primary biliary cirrhosis recognize both specific peptides and shared epitopes of the M2 family of antigens. *Hepatology,* 1989, vol. 10, 370
- **Fussey, S.P.M. ; Ali, S.T. ; Guest. J.R. ; James, O.F.W. ; Bassendine, M.F. ; Yeaman, S.J.** Reactivity of primary biliary cirrhosis sera with Escherichia coli dihydrolipoamide acetyltransferase (E2p) : Characterization the main immunogenic region. *Proc. Natl. Acad. Sci.,* 1990, vol. 87, 3987
- **Fussey, S.P.M. ; Guest, J.R. ; James, O.F.W. ; Bassendine, M.F. ; Yeaman, S.J.** Identification and analysis of the major M2 autoantigens in primary biliary cirrhosis. *Proc. Natl. Acad. Sci. USA,* 1988, vol. 85, 8654-8658
- **Hallgren, H.M. ; Buclkey, C.E. ; Gillusten, V.A. ; Yunis, E.A.** Lymphocyte phytohemagglutinin responsiveness. Immunoglobulins and autoantibodies in aging man. *J. Immunol.,* 1973, vol. 111, 1101
- **Isenberg, D.A. ; Collin, C.** Detection of cross-reactive anti-DNA antibody idiotype on renal tissue-bound immunogobulins from lupus patients. *J. Clin. Invest.,* 1985, vol. 76, 287
- **Kalunian, K.C. ; Sahakian, N.P. ; Ebling, F.M.** Idiotypic characteristics of immunoglobulins associated with systemic lupus erythematosus. *Arth. Rheum.,* 1989, vol. 32, 513
- **Klinman, D.M. ; Shirai, A. ; Ishigatsubo, Y. ; Conover, J. ; Strinberg, A.D.** Quantitation of IgG and IgM secreting B cells in the peripheral blood of patients with systemic lupus erythematosus. *Arthritis Rheum.,* 1991, vol. 134, 1404
- **Lafer. E.M. ; Rauch, J. ; Andrzejewski, C.J.R. ; Mudd, D. ; Furie, B. ; Schwartz, R.S. ; Stollar, B.D.** Polyspecific monoclonal lupus autoantibodies reactive with bath polynucleotides and phospholipids. *J. Exp. Med.,* 1981, vol. 153, 897

- **Legastelois, S. ; Thomas, V. ; Quash, G. ; Métais, M.P. ; Tebib, J. ; Moreira, A. ; Monier, J.C.** Naturally occurring antibodies reacting with lipoic acid : screening method, characterization and biochemical interest. *J. Immunol. Meth.,* 1994, vol. 171, 111
- **Naor, D. ; Bonavida, B. ; Robinson, R.A. ; Shibata. I.N. ; Percy, D.E. ; Chia, D. ; Barnett, E.V.** Immune response of New Zealand mice with trinitrophenylated syngeneic mouse red cells. *Eur. J. Immunol.,* 1976, vol. 6, 783
- **Ohmori, H. ; Yamamoto. I.** Activation of murine lympocytes by 2-mercaptoethanol and related thiol compounds and its mechanism. I.. Relationship between mitogenic activities and augmenting effects on antibody synthesis in vitro. *Immunopharmacol,* 1981, vol. 3, 333
- **Ohmori, H. ; Yamamoto, I.** Mechanism of augmentation of the antibody response in vitro by 2-mercaptoethanol-induced stimulation of the uptake of cystine, an essential animo acid. *J. Exp. Med.,* 1982, vol. 155, 1277
- **Opitz, H.G. ; Opitz, U. ; Lemcke, H. ; Hewlett, G. ; Schreml, W. ; Flad, H.D.** The role of fetal calf serum in the primary immune response in vitro. *J. Exp. Med.,* 1977, vol. 145, 1029
- **Romball, C.G. ; Weigle, W.O.** The effect of aging on the induction of experimental autoimmune thyroidite. *J. Immunol.,* 1987, vol. 193, 1490
- **Sasaki, T. ; Muryoi, T. ; Hatakeyama, A. ; Suzuki, M. ; Sato, H. ; Seino, J. ; Saito, T. ; Yoshinaga, K.** Circulating anti-DNA immune complexes in active lupus nephritis. *Am. J. Med.,* 1991, vol. 91, 355
- **Shoenfeld, Y. ; Isenberg, D.A. ; Rauch, J. ; Madaio, M. ; Stollar, B.D. ; Schwartz, R.S.** Idiotypic cross-reactions of monoclonal human lupus autoantibodies. *J. Exp. Med.,* 1983, vol. 158, 718
- **Stephens, P.E. ; Darlison, M.G. ; Lewis, H.M. ; R., G.J.** The pyruvate dehydrogenase complex of E.coli K12. Nucleotide sequence encoding the dihydrolipoamide acetyltransferase component. *Eur. J. Biochem.,* 1983, vol. 133 (3), 481
- **Suzuki, K. ; Hara, M. ; Harigai, M. ; Ishizuka, T. ; Hirose, T. ; Matsuki, Y. ; Kawaguchi, Y. ; Kitani, A. ; Kawagoe, M. ; Nakamura, H.** Continuous removal of anti-DNA antibody, using a new extracorporeal immunoadsorption system, in patients with systemic lupus erythematosus. *Arth. Rheum.,* 1991, vol. 34, 1546
- **Terman, D.S. ; Buffaloe, G. ; Mattioli, C. ; Cook, G. ; Tillquist, R. ; Sullivan, M. ; Ayus, J.C.** Extracorporeal immunoadsorption : initial experience in human systemic lupus erythematosus. *Lancet II,* 1979, 824